(19) Europäisches Patentamt / European Patent Office / Office européen des brevets

(11) **EP 4 044 923 B1**

(12) **EUROPEAN PATENT SPECIFICATION**

(45) Date of publication and mention
of the grant of the patent:
**15.02.2023 Bulletin 2023/07**

(21) Application number: **20789142.5**

(22) Date of filing: **13.10.2020**

(51) International Patent Classification (IPC):
**A61B 5/145** (2000.01)        **A61B 5/00** (1968.09)
**A61B 5/1455** (2006.01)        **A61B 5/1477** (2006.01)

(52) Cooperative Patent Classification (CPC):
**A61B 5/14517; A61B 5/4266;** A61B 5/1455;
A61B 5/1477

(86) International application number:
**PCT/EP2020/078685**

(87) International publication number:
**WO 2021/074099 (22.04.2021 Gazette 2021/16)**

(54) **SYSTEM AND METHOD FOR SWEAT RATE DETERMINATION**

SYSTEM UND VERFAHREN ZUR SCHWEISSRATENBESTIMMUNG

SYSTÈME ET PROCÉDÉ DE DÉTERMINATION DU DÉBIT DE TRANSPIRATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.10.2019 EP 19202956**

(43) Date of publication of application:
**24.08.2022 Bulletin 2022/34**

(73) Proprietor: **Koninklijke Philips N.V.
5656 AG Eindhoven (NL)**

(72) Inventors:
• **PELSSERS, Eduard, Gerard, Marie**
  **5656 AE Eindhoven (NL)**
• **DELLIMORE, Kiran, Hamilton, J.**
  **5656 AE Eindhoven (NL)**
• **VAN LIESHOUT, Ron, Martinus, Laurentius**
  **5656 AE Eindhoven (NL)**
• **GERHARDT, Lutz, Christian**
  **5656 AE Eindhoven (NL)**
• **BOUMA, Peter, Hermanus**
  **5656 AE Eindhoven (NL)**
• **JOHNSON, Mark, Thomas**
  **5656 AE Eindhoven (NL)**
• **HUIJBREGTS, Laurentia, Johanna**
  **5656 AE Eindhoven (NL)**

(74) Representative: **Philips Intellectual Property &
Standards
High Tech Campus 52
5656 AG Eindhoven (NL)**

(56) References cited:
**WO-A1-2019/060689        US-A1- 2010 179 403
US-A1- 2018 042 585**

• **NISHIYAMA TETSUNARI ET AL: "Irregular
activation of individual sweat glands in human
sole observed by a videomicroscopy",
AUTONOMIC NEUROSCIENCE: BASIC AND
CLINICAL, vol. 88, no. 1-2, 1 April 2001
(2001-04-01), pages 117-126, XP055952405, NL
ISSN: 1566-0702, DOI:
10.1016/S1566-0702(01)00229-6**

**Description**

FIELD OF THE INVENTION

This invention relates to a system and method for determining a sweat rate, in particular a sweat rate per gland and measuring biomarker concentration.

BACKGROUND OF THE INVENTION

Non-invasive, semi-continuous and prolonged monitoring of biomarkers that indicate disease/health status and well-being is in demand for monitoring, for example, dehydration, stress, sleep, children's health and in perioperative monitoring.

Sweat, tear fluid and saliva may all be obtained non-invasively. Sweat is a particularly accessible biofluid, and is a rich source of information relating to the physiology and metabolism of a subject.

Some examples of clinical relevant components of sweat are $Na^+$, $Cl^-$ and/or $K^+$ to monitor dehydration, lactate as an early warning for inflammation (which is relevant to sepsis), glucose for diabetics and neonates, and cortisol in relation to sleep apnea and stress monitoring.

Continuous monitoring of high-risk patients, such as those with serious chronic conditions, pre- or post-operative patients, and the elderly, using sweat biomarker monitoring devices can provide higher quality diagnostic information than regular biomarker spot checks as normally done by repeatedly drawing multiple blood samples. Such continuous monitoring may be in a hospital setting or elsewhere. Human sweat alone or as mixture with sebum lipids may be an easily accessible source for biomarker measurements in wearable on-skin devices. For instance, cholesterol is an important biomarker associated with elevated risk in development of cardiovascular diseases. Inflammatory markers or cytokines, such as interleukins (e.g. TNF-a, IL-6) play an important role in the immune response and detection or disease monitoring of joint damage in rheumatoid and psoriatic arthritis, and bowel disease.

Examples of biomarkers that can be detected in eccrine/apocrine sweat using suitable capture species (antibodies, aptamers, molecular imprint polymers, etc.) are: small molecules such as urea, creatinine, cholesterol, triglycerides, steroid hormones (cortisol), glucose, melatonin; peptides and proteins, including cytokines such as IL-1alpha, IL-1beta, IL-6, TNF alpha, IL-8 and TGF-beta IL-6, Cysteine proteinases, DNAse I, lysozyme, Zn-$\alpha$2-glycoprotein, cysteine-rich secretory protein-3 and Dermcidin; and large biomarkers such as the Hepatitis C virus.

As summarized by Mena-Bravo and de Castro in "Sweat: A sample with limited present applications and promising future in metabolomics", J. Pharm. Biomed. Anal. 90, 139-147 (2014), it has been found that the results from sweat sensing can be highly variable, and a correlation between values determined from blood and sweat samples appears to be lacking for various biomarkers. In this respect, historical studies in this area have involved relatively crude sampling techniques, such as collecting large sweat volumes in bags or textiles. Deficiencies in such techniques may have been a contributing factor to this apparent lack of correlation. The review of Mena-Bravo and de Castro thus highlights further key frustrations with conventional sweat sensing techniques in terms of the difficulty of producing enough sweat for analysis, the issue of sample evaporation, the lack of appropriate sampling devices, the need for trained staff, and issues relating to the normalization of the sampled volume.

Efforts have been made to address these issues by bringing wearable sensors into nearly immediate contact with sweat as it emerges from the skin. A recent example is the wearable patch presented by Gao et al. in "Fully integrated wearable sensor arrays for multiplexed in situ perspiration analysis", Nature 529, 509-514 (2016). The patch includes a sensor array for measuring $Na^+$, $K^+$, glucose, lactate, and skin temperature. However, the focus of this study is on the development and the integration of the sensors themselves which, whilst evidently crucial, does not address issues relating to sweat sample collection. The latter is mostly done by placing a several $cm^2$ sized absorbent pad between the skin and the sensor. The assumption is that, providing ample sweat is produced (hence tests are carried out on individuals that are exercising), the pad will absorb the sweat for analysis, and newly generated sweat will refill the pad and "rinse away" the old sweat. It is, however, likely that the time-dependent response of the sensor does not directly reflect the actual level of biomarkers over time because of accumulation effects. The sample collection and presentation to the published sensors may not be well-controlled so that continuous reliable sensing over a long period of time is difficult. Such patches may also not be designed to handle the tiny amounts of sweat that are produced under normal conditions, i.e. in the order of subnaniliters to nanoliters per minute per sweat gland.

Adult humans produce heat in the order of 100 Joules per second (100 Watt) when at rest. For a person wearing clothes at a temperature of around 22 °C this heat is removed by passive means such as losing heat by conduction and convection. In this case, the core temperature remains constant. However, when i) a person engages in physical labor or exercise and/or ii) the ambient temperature is increased, such conduction/convection processes are insufficient to maintain the core temperature. To maintain homeostasis, the body induces dilation of blood vessels in the skin to cool the blood, and starts to produce sweat which by evaporation cools the skin.

The amount of sweat produced by persons at ambient temperature with only light exercise or light labor is relatively small as discussed by Taylor in "Regional variations in transepidermal water loss, eccrine sweat gland density, sweat secretion rates and electrolyte composition in resting and exercising humans", Extrem Physiol Med 2013; 2:4, and Simmers in "Prolonged and localised sweat stimulation by iontophoretic delivery of the slowly-metabolised cholinergic agent carbachol", Journal of Dermatological Science 89 (2018) 40-51". In the so-called thermal neutral zone, which is about in the range of 25 °C to 30 °C, the core temperature remains very stable and inducing sweat production is not required for cooling down the body. This zone is defined for a naked man at rest. For a person in a resting state wearing clothes, the thermal neutral zone is lower: in the range of about 13 °C to 22 °C. Hence, when the temperature is in this zone and the person is in a resting state, the sweat production is very low.

According to Taylor, in resting and thermal neutral conditions, the sympathetic discharge (secretion by the coil of the sweat gland) may not elicit measurable sweating since sweat reabsorption may match its formation rate. Simmers measured the sweat production rates of persons that were wearing clothes, being exposed to an air-conditioned environment, doing primarily non-manual labor and found sweat rates with a typical value of about 0.3 nl/min/gland (values measured between zero and 0.7 nl/min/gland). When persons are at rest but at an elevated temperature of 36°C, a sweat production rate was measured by Taylor to be, on average, 0.36 mg·cm$^2$·min$^{-1}$. When assuming 2.03 million sweat glands per 1.8 m$^2$ (skin area of an average person) and sweat density of 1 g/ml, the average sweat production is about 3.2 nl/min/gland. Due to the elevated temperature above the thermal neutral zone the body requires cooling and indeed the sweat production rate is increased.

Accordingly, persons in a sedentary state, such as hospital patients, have a minimal sweat rate and there is therefore a significant delay between sweat excretion and biomarker detection, which can prevent timely monitoring and early warning of any impending complication. The concentration of particular relevant biomarkers is sweat rate dependent and therefore sweat rate per gland has to be assessed for a clinically relevant interpretation. Conventional sweat sensing solutions have limited application since they require the monitored person to be engaged in exercise, and tend to use rather complex microfluidics and sensors to determine the sweat rate.

WO 2018/125695 A1 discloses wearable sweat biosensing devices with active sweat sampling. An active method is described for transporting sweat which utilises the electromechanical effect of electrowetting. Electrowetting plates comprise a hydrophobic dielectric layer (e.g., Teflon) covering electrodes. A sweat coupling "wicking" component made of a hydrophilic material permits sweat from the skin surface to slowly diffuse over time to the electrowetting plates, whereupon the sweat is transported via the electromechanical effect. This approach is very time consuming, and may be ineffective for small sweat volumes due to evaporation. Moreover, the technique entails mixing of sweat received from the skin at different times, which is undesirable for reliable semi-continuous biomarker measurements.

US 2015/0112165 A1 discloses a method to determine the sweat rate per gland. The method involves using numerous sweat rate sensors to monitor a cumulative change in dielectric value of a porous material in respective sweat collecting chambers. Sodium sensors each monitor the sodium concentration of the sweat in the respective chambers. By use of a correlation curve derived from a volunteer test, the sodium ion concentration is correlated with total sweat flow rate. This approach has two major drawbacks: (i) it assumes a number of sweat glands per surface area during the volunteer test, and (ii) it assumes that the correlation of sodium concentration and sweat rate as determined by the volunteer test is applicable to any particular person/patient. The rather large differences observed between individuals can make the latter assumption unwise, and illness can make such differences even greater.

Heikenfeld et al., in "Digital nanoliter to milliliter flow rate sensor with in vivo demonstration for continuous sweat rate measurement", Lab Chip, 2019,19, 178, and Yang et al. in "Wearable microfluidics: fabric-based digital droplet flowmetry for perspiration analysis", Lab on a Chip. Accepted 4th January 2017. DOI: 10.1039/c61c01522k, disclose sweat rate sensors which collect sweat within a chamber positioned adjacent the skin. A sweat droplet grows from an outlet of the chamber until it is released from the outlet by contacting and being transferred to a wick opposing the outlet. Immediately prior to this release, the sweat droplet contacts one of a pair of electrodes, which electrode is mounted on the wick. The other electrode is mounted in the chamber. The electrodes are thus shorted by the connection provided by the sweat in the chamber and the sweat droplet which is still attached thereto. This shorting of the electrodes immediately prior to release of the sweat droplet into the wick enables the device to count the sweat droplets. The design nevertheless necessitates provision of a sweat rate sensor per chamber. This makes the arrangement disadvantageously complex. Moreover, the design may be incompatible with the provision of alternative sweat droplet sensing principles.

WO 2019/060689 A1 discloses a discrete volume sensing system flow rate and analyte sensor.

US 2018/042585 A1 discloses sweat sensing devices with prioritized sweat data from a subset of sensors.

US 2010/179403 A1 discloses a method and kit for sweat activity measurement. Nishiyama et al., "Irregular activation of individual sweat glands in human sole observed by a videomicroscopy", Autonomic Neuroscience: Basic and Clinical, vol. 88, no. 1-2, 1 April 2001, pages 117-126, DOI: 10.1016/ S1566-0702(01)00229-6, discloses the detection of the activity of individual sweat glands using videomicroscopy in combination with the detection of sudomotor bursts.

## SUMMARY OF THE INVENTION

The invention is defined by the independent claims. The dependent claims define advantageous embodiments.

According to an aspect there is provided a system comprising: a sensor for sensing sweat droplets; an apparatus for receiving sweat from one or more sweat glands and transporting the sweat as discrete sweat droplets to the sensor; and a processor configured to: record sweat droplets sensed by the sensor during a time period; determine time intervals between consecutive sensed sweat droplets during the time period; and identify, using the time intervals, at least one active period of each of the one or more sweat glands during which the respective sweat gland is excreting sweat, and at least one rest period of each of the one or more sweat glands during which the respective sweat gland is not excreting sweat, the active and rest periods being assigned to the one or more sweat glands.

The apparatus may sample sweat from the skin, and transport the sweat dropwise to the sensor. Depending on the design of the apparatus, the respective sweat samples may be constituted by sweat from a single sweat gland or more than one sweat gland. This may complicate determination of the sweat rate per gland, since the number of sweat glands is ambiguous.

Sweat glands are known to excrete sweat in a cyclic manner. Active, or "sweat burst", periods during which the sweat glands are excreting sweat are separated by rest periods during which the sweat glands are not excreting sweat. The sweat burst periods tend to last typically for about 30 seconds, while the rest periods may be about 150 seconds.

The present invention is based on the realization that this cyclic behavior of sweat glands may be used to determine the number of sweat glands supplying the sweat sensed by the sensor. During the sweat bursts, one or more active sweat glands excrete sweat which is carried in the form of a train of discrete sweat droplets to the sensor. This results in a series of distinct sensor signals, i.e. pulses, being generated corresponding to the discrete sweat droplets being detected by the sensor. By taking account of the time intervals between consecutive sweat droplets (pulses), the active and rest periods of the sweat gland or glands may be determined. The process of identifying the active and rest periods necessarily involves assigning such active and rest periods to the one or more sweat glands. The number of sweat glands can then be determined.

The method thus relies on determining the sweat burst period(s) of individual sweat glands. The apparatus may, for example, receive sweat from the skin via one or more chambers. Each of the chambers may be configured to receive sweat from, for instance, a maximum of five sweat glands. In other words, the inlets of each of the chambers may be dimensioned to span a skin area occupied by the outlets of a maximum of five individual sweat glands. To this end, the area of each inlet may be, for example, in the range of 0.05 mm$^2$ to 2 mm$^2$, such as 0.75 mm$^2$ to 1.5 mm$^2$.

Any suitable sensor may be employed for the purpose of sensing the sweat droplets, such that each of the sensed sweat droplets may be recorded/registered. For example, a capacitance, conductivity, impedance, optical and/or biomarker sensor may be used.

The processor may be further configured to determine the number of sweat glands to which the active and rest periods are assigned.

The processor may be further configured to: receive a measure of the volume of each of the recorded sweat droplets, e.g. corresponding to the width of the pulses, and determine the sweat rate per gland from the number of sweat droplets, the measure of the volume of each of the recorded sweat droplets, and the determined number of sweat glands.

The number of sweat droplets sensed by the sensor during a time period, and the measure of the volume of each of the recorded sweat droplets, together with the determined number of sweat glands enables calculation of the sweat rate per gland.

The processor may be configured to identify the at least one active period and the at least one rest period based on the measure of the volume of each of the recorded sweat droplets and the time intervals. Consideration of both the time intervals and the measure of the volume of each of the recorded sweat droplets may permit interpretation of sensor signal patterns in which the sensor signals corresponding to an active period of one sweat gland overlap with or overlay those of another sweat gland.

The sensor may be configured to sense an indicator of the volume of the sweat droplets. The processor may thus be configured to receive the sensed indicator. The indicator may, for example, be the contact time of each sweat droplet with the sensor, e.g. the time taken for the sweat droplet to pass through the sensor, i.e. the pulse width of each sensor signal. The pulse width may be used to determine the volume of the sweat droplet providing the speed of migration of the sweat droplet through the sensor is known or may be estimated.

The processor may be configured to fit data received from the sensor to a first template model, thereby to identify the active and rest periods of each of the one or more sweat glands, the data comprising at least the time intervals, and the measure of the volume of each of the recorded sweat droplets.

An algorithm involving fitting the sensor signal patterns to a template model may provide a convenient means of identifying the active and rest periods, and assigning these to the one or more sweat glands.

The fitting to the first template model may additionally use: a number of sweat droplets in the at least one active period, a duration of the at least one active period, and/or a duration of the at least one rest period. Taking one or more of such factors into account may enhance the capability of the template model fitting algorithm to identify the active and rest periods, and assign these to the one or more sweat glands.

The processor may be configured to assess a goodness of fit of the data to the first template model. Based on the goodness of fit, the processor may fit at least a portion of the data to a further first template model. The portion of data satisfying a goodness of fit criterion may, for example, be subtracted from the original data and the remainder of the data may be fitted to the further first template model, thereby enabling an iteration to be performed.

The processor may be configured to, following fitting the data to the first template model, fit at least a portion of the data to a second template model, wherein the first template model is based on at least some of the sweat droplets deriving from a sweat sample constituted by sweat excreted from a single sweat gland, and the second template model is based on at least some of the sweat droplets deriving from a further sweat sample constituted by sweat excreted from two or more sweat glands. Such a two-step fitting approach may be particularly useful when, due to sweat being received from a relatively large skin area, there is a significant probability of a (further) sweat sample being constituted by sweat excreted from two or more sweat glands.

The apparatus may be arranged to transport sweat droplets having a predetermined volume to the sensor. By the sweat droplets having a predetermined volume, the ease with which the signal sensor patterns may be assigned to the one or more sweat glands may be enhanced. In other words, the fitting space may be advantageously restricted in size.

The sensor may comprise a sensing device for detecting a parameter relating to the concentration of an analyte whose concentration varies as a function of sweat rate, wherein the processor is configured to use the parameter in assigning the active and rest periods to the one or more sweat glands. The sweat rate dependence of the parameter may thus assist to resolve any ambiguity encountered in interpreting the sensor signal patterns.

The sensing device may be a conductivity sensor and the parameter is conductivity. The conductivity sensor, in particular, may assist in the determination of the sweat rate per gland. This is because the conductivity of the sweat may act as a proxy for the sodium ion concentration, which is sweat rate dependent. Moreover, the conductivity may be readily sensed using a relatively simple electrode arrangement.

The sensor may comprise a biomarker sensor. The biomarker sensor may be, for example, a lactate sensor. The concentration of particular relevant biomarkers, such as lactate, is sweat rate dependent and therefore the capability of the system to determine the sweat rate per gland may assist to provide a clinically relevant interpretation of the sensed biomarker concentration.

To this end, the processor may be configured to receive a plurality of biomarker concentrations from the biomarker sensor during the at least one active period of a respective sweat gland, and determine a variation of the biomarker concentration in time within the at least one active period.

Moreover, in the case of lactate, timescales associated with sweat gland-related changes, i.e. due to the active and rest periods of the sweat glands, and blood-related changes in lactate concentration in the sweat excreted onto the skin, may be used to differentiate the former source of lactate from the latter. Accordingly, measuring the lactate concentration in sweat as a function of time may lead to suitable differentiation of sweat gland-derived and blood-derived changes in lactate concentration.

Further provided is a method comprising: receiving sweat from one or more sweat glands; transporting the sweat as discrete sweat droplets to a sensor; sensing the sweat droplets using the sensor during a time period; recording the sensed sweat droplets during the time period; determining time intervals between consecutive sensed sweat droplets during the time period; using a processor to identify, using the time intervals, at least one active period of each of the one or more sweat glands during which the respective sweat gland is excreting sweat, and at least one rest period of each of the one or more sweat glands during which the respective sweat gland is not excreting sweat, the active and rest periods being assigned to the one or more sweat glands.

The method may further comprise: determining the number of sweat glands to which the active and rest periods are assigned.

The method may enable determination of the sweat rate per gland. In this respect, the method may comprise receiving a measure of the volume of each of the recorded sweat droplets; and determining the sweat rate per gland from the number of recorded sweat droplets, the measure of the volume of each of the recorded sweat droplets, and the determined number of sweat glands.

The identifying, via the processor, the at least one active period and the at least one rest period may be based on the measure of the volume of each of the recorded sweat droplets and the time intervals.

The method may comprise using the sensor to sense the measure of the volume, wherein receiving the measure of the volume comprises receiving the sensed measure of the volume from the sensor.

The identifying may comprise fitting data received from the sensor to a first template model, the data comprising at least the time intervals, and the measure of the volume of each of the recorded sweat droplets. The fitting may, for example, additionally use: a number of sweat droplets in the at least one active period, a duration of the at least one active period, and/or a duration of the at least one rest period.

The method may additionally comprise assessing a goodness of fit of the data to the first template model, and optionally, based on the goodness of fit, fitting at least a portion of the data to a further first template model.

The identifying may further comprise, following fitting the data to the first template model, fitting at least a portion of the data to a second template model, wherein the first template model is based on at least some of the sweat droplets deriving from a sweat sample constituted by sweat excreted from a single sweat gland, and the second template model is based on at least some of the sweat droplets deriving from a further sweat sample constituted by sweat excreted from two or more sweat glands.

BRIEF DESCRIPTION OF THE DRAWINGS

Embodiments of the invention are described in more detail and by way of non-limiting examples with reference to the accompanying drawings, wherein:

Fig. 1 shows a first example of an apparatus for transporting sweat droplets to a sensor;
Fig. 2 shows a second example of an apparatus for transporting sweat droplets to a sensor;
Fig. 3 shows a third example of an apparatus for transporting sweat droplets to a sensor;
Fig. 4 shows a fourth example of an apparatus for transporting sweat droplets to a sensor;
Fig. 5 shows a fifth example of an apparatus for transporting sweat droplets to a sensor;
Fig. 6 shows a sixth example of an apparatus for transporting sweat droplets to a sensor;
Fig. 7 shows a seventh example of an apparatus for transporting sweat droplets to a sensor;
Fig. 8 shows an eighth example of an apparatus for transporting sweat droplets to a sensor;
Fig. 9 shows a ninth example of an apparatus for transporting sweat droplets to a sensor;
Fig. 10 shows a tenth example of an apparatus for transporting sweat droplets to a sensor;
Fig. 11a shows part of an electrowetting arrangement according to an example;
Fig. 11b shows part of an electrowetting arrangement according to another example;
Fig. 12 shows an example of an apparatus having a plurality of chambers which are each connected to a common interconnection in parallel;
Fig. 13 shows a sensor for sensing sweat droplets according to an example;
Fig. 14 shows six exemplary sensors for sensing sweat droplets;
Fig. 15 shows plots of the time taken for a sweat droplet to pass through a sensor as a function of droplet volume for a beam-shaped sweat sample (dotted line) and a hemispherical sweat droplet (solid line);
Fig. 16 shows part of a system for sensing sweat droplets according to an example;
Fig. 17 shows a further example of an apparatus for transporting sweat droplets to a sensor;
Fig. 18 shows a graph depicting two sweat bursts and two rest periods (upper pane), an enlarged view of the first sweat burst and the first rest period (middle pane), and a graph of the sweat rate sensor signal as a function of time (lower pane);
Fig. 19 shows a graph depicting two sweat bursts of a first sweat gland and two sweat bursts of a second sweat gland (upper pane), and a graph of the sweat sensor signal as a function of time (lower pane);
Fig. 20 shows a graph of the sweat rate sensor signal as a function of time when sweat droplets derived from two sweat glands excrete sweat into respective chambers, but all of the sweat droplets exactly coincide with each other;
Fig. 21 shows a graph of the sweat rate sensor signal as a function of time when sweat droplets derived from two sweat glands excrete sweat into respective chambers, and some of the respective sweat droplets coincide with each other;
Fig. 22 shows a graph of the sweat rate sensor signal as a function of time when sweat droplets derived from two sweat glands excrete sweat into respective chambers, and there is some overlap between the respective sets of sensor signals but the respective sweat droplets do not coalesce with each other;
Fig. 23 shows a flowchart of a method for determining a sweat rate per gland according to an example;
Fig. 24 shows an example of an algorithm for attributing sweat droplets to the gland(s) from which they derived;
Fig. 25 shows a graph of the sweat rate sensor signal as a function of time when the sweat rate is relatively high;
Fig. 26 shows graphs of the sweat rate sensor signal as a function of time when the sweat droplet derives from one gland per chamber (upper pane), and when the sweat droplet derives from two glands per chamber (lower pane);
Fig. 27 shows a graph of the sweat rate as a function of time with a schematic depiction of the frequency of the electrowetting wave when the latter is not synchronized with sweat droplet formation (upper pane), and a graph of the associated sweat rate sensor signal as a function of time (lower pane);
Fig. 28 shows graphs analogous to those shown in Fig. 27, but with more pronounced ramp up and down at the start and end of the sweat burst respectively;
Fig. 29 shows part of a further exemplary electrowetting arrangement;
Fig. 30 shows a graph of a sweat rate sensor signal as a function of time for a sweat gland excreting at a defined average rate (upper pane), and graphs showing two models of lactate concentration as a function of time (lower panes);

Fig. 31 shows part of another system for sensing sweat droplets according to an example in which a calibration fluid is also supplied dropwise to the sensor;
Fig. 32 shows a further system for sensing sweat droplets according to an example;
Fig. 33 shows a first view of part of a further exemplary apparatus for transporting sweat droplets to a sensor;
Fig. 34 shows a second view of the part of the apparatus shown in Fig. 33;
Fig. 35 shows a third view of the part of the apparatus shown in Fig. 33;
Fig. 36 shows a fourth view of the part of the apparatus shown in Fig. 33;
Fig. 37A shows a plan view of an alternative outlet of a chamber to the outlet design shown in Figs. 34-36;
Fig. 37B shows a plan view of another alternative outlet of a chamber to the outlet design shown in Figs. 34-36;
Fig. 38 shows sweat droplets being transported using the apparatus shown in Figs. 33-36;
Fig. 39 shows electrical connections of the electrodes shown in Figs. 35, 36, and 38;
Fig. 40 shows a first view of part of a still further exemplary apparatus for transporting sweat droplets to a sensor;
Fig. 41 shows a second view of the part of the apparatus shown in Fig. 40;
Fig. 42 shows sweat droplets being transported using the apparatus shown in Figs. 40 and 41;
Fig. 43 shows electrical connections of the electrodes shown in Figs. 41 and 42;
Fig. 44 provides a view of a yet further exemplary apparatus for transporting sweat droplets to a sensor; and
Fig. 45 provides a view of an apparatus having an alternative electrical connections design.

DETAILED DESCRIPTION OF THE EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

As noted above, conventional sweat analysis techniques tend to be restricted to persons engaged in exercise for the purpose of inducing sufficient sweating to enable measurement. This is inappropriate in healthcare settings where patients are mostly sedentary and the sweat rate is correspondingly relatively low, e.g. in the order of 0.2 nl/min/gland.

A related problem of conventional sweat sensing devices is the significant time delay between sweat excretion and biomarker measurement at such low sweat rates. The filling of the sweat collection chambers employed in such devices may take up to several hours.

So-called sweat rate dependent biomarkers require measurement of sweat rate per gland in order for the biomarker data to be meaningful. However, known systems having capability to measure the sweat rate per gland have the disadvantage of highly complex designs. Ten or more complex flow sensors may, for example, be required to monitor the sweat flow in multiple sweat collection chambers. WO 2018/125695, for example, discloses a complex system utilizing numerous sweat rate sensors and sodium biomarker sensors in concert to determine the average sweat rate per gland.

At low sweat rates, evaporation becomes a disturbing factor leading to artificially elevated biomarker concentrations. Evaporation can also inhibit or prevent sweat from reaching the sensor, especially at low sweat rates (in the order of 0.2 nl/min/gland) and volumes.

A further disadvantage of conventional sweat sensing devices is that the electrochemical sensors, often used for semi-continuous measurement, may require frequent recalibration and offline calibration. This may have a negative workflow impact when such devices are used for monitoring a subject.

Provided is a system which may be used in the determination of a sweat rate per gland. The system comprises an apparatus and a sensor. The apparatus receives sweat from the skin and transports the sweat as discrete sweat droplets to the sensor. The sensor senses each of the sweat droplets. The system further comprises a processor which registers the number of sensed sweat droplets during a time period. The processor also determines time intervals between consecutive sensed sweat droplets, and may optionally receive a measure of the volume of each of the sensed sweat droplets. The time intervals and optionally the measure of the volume are then used by the processor to identify sweat burst and rest periods of the sweat gland or glands producing the sweat. This identification process necessarily involves assigning the sweat burst and rest periods to the sweat gland or glands, such that the processor is permitted to determine the number of sweat glands involved in producing the sweat.

The sweat rate per gland may then be determined from the number of sensed sweat droplets, the measure of the volume of each of the sweat droplets, and the determined number of sweat glands.

The apparatus samples sweat from the skin, and transports the sweat dropwise to a sensor. Depending on the design of the apparatus, the respective sweat samples may be constituted by sweat from a single sweat gland or more than one sweat gland. This may complicate determination of the sweat rate per gland, since the number of sweat glands is ambiguous.

Sweat glands are known to excrete sweat in a cyclic manner. Active, or "sweat burst", periods during which the sweat glands are excreting sweat are separated by rest periods during which the sweat glands are not excreting sweat. The sweat burst periods tend to last for typically about 30 seconds, while the rest periods may be about 150 seconds.

The present invention is based on the realization that this cyclic behavior of sweat glands may be used to determine the number of sweat glands supplying the sweat sensed by the sensor. During the sweat bursts, one or more active sweat glands excrete sweat which is carried in the form of a train of discrete sweat droplets to the sensor. This results in a series of distinct sensor signals, i.e. pulses, being generated corresponding to the discrete sweat droplets being detected by the sensor. By taking account of the time intervals between consecutive sweat droplets (pulses), and optionally the measure of the volume of each of the sensed sweat droplets, e.g. corresponding to the width of the pulses, the active and rest periods of the sweat gland or glands may be determined.

Consideration of both the intervals and the measure of the volume of each of the counted sweat droplets may facilitate interpretation of sensor signal patterns in which the sensor signals corresponding to an active period of one sweat gland overlap with or overlay those of another sweat gland. The process of identifying the active and rest periods necessarily involves assigning such active and rest periods to the one or more sweat glands. The number of sweat glands can then be determined.

The number of sweat droplets sensed by the sensor during a time period, and the measure of the volume of each of the counted sweat droplets, together with the determined number of sweat glands enables calculation of the sweat rate per gland.

The apparatus provides the sensor with a discretized flow of sweat instead of the continuous flow of sweat used in conventional sweat sensing devices. The fluid transport assembly causes the sweat droplet to be released from the outlet of the chamber and transported to the sensor. The migration of droplets towards, and in some examples through, the sensor may, for instance, be via an interfacial tension method and/or by application of pressure, as will be further described herein below.

The dropwise or discretized flow of sweat offers several unique benefits with respect to continuous flow. The delay between excretion of sweat and the actual determination of the biomarker concentration may be reduced, e.g. from typically 1-2 hours to about 10-15 minutes for subjects in a sedentary state. The capability of handling minute amounts of sweat and being able to transport this relatively rapidly to the sensor may enable, in the case of the sensor comprising a biomarker sensor, biomarker concentrations to be determined, even when subjects are in a sedentary state. Moreover, the sweat rate may be more straightforwardly determined, e.g. using simpler sensors, when the sweat is provided as discrete sweat droplets rather than as a continuous flow.

Since the apparatus releases the sweat droplets from the outlet before transporting the sweat droplets to a sensor, there is no requirement for a sensor to be provided for each chamber, e.g. in order to sense the sweat droplet while it is still attached to the bulk of the sweat collected in the chamber, as in some of the prior art devices. The present apparatus may correspondingly provide greater design flexibility. For example, the apparatus may transport sweat to a sensor which is spatially removed from the outlet.

As will be described in greater detail below with reference to the Figures, the fluid transport assembly may comprise a surface extending between the outlet and the sensor. The surface may have, for example, a topological and/or chemical gradient down which the sweat droplets migrate to the sensor during use of the apparatus. An electrowetting technique may alternatively or additionally be used. Such an electrowetting technique uses an electric field to effect transient modification of the wetting properties of a surface in order to cause migration of the sweat droplet along the surface towards the sensor.

The fluid transport assembly may alternatively or additionally apply pressure to the sweat droplet in order to release the sweat droplet from the outlet and/or transport the released sweat droplet to the sensor. The pressure may be applied via, for example, a flow of carrier fluid in which the sweat droplet is immiscible flowing in the direction of the sensor.

The resulting train of sweat droplets can be detected and counted by using, for instance, a simple detector having a pair of electrodes between which each sweat droplet passes. A facile means of measuring the sweat rate is correspondingly provided.

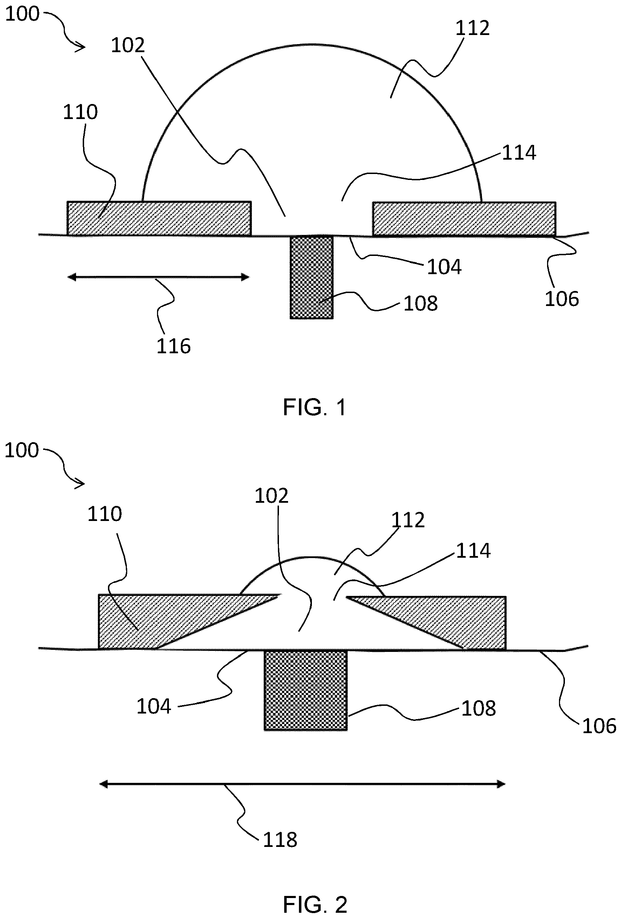

Fig. 1 schematically depicts, in cross-sectional view, part of an apparatus 100 according to an example. The apparatus 100 comprises a chamber 102 having an inlet 104. The inlet 104 receives sweat from the skin 106. As shown in Fig. 1, the inlet 104 may be disposed adjacent to a surface of the skin 106. Whilst a single chamber 102 is depicted in Fig. 1, this is not intended to be limiting, and in other examples a plurality of chambers 102 may be included in the apparatus 100, as will be further described herein below.

The inlet 104 is shown proximal to a sweat gland 108. In this case, the sweat excreted by the sweat gland 108 enters and fills the chamber 102 via the inlet 104. As shown in Fig. 1, the apparatus 100 may comprise a plate 110 which is attached to the surface of the skin 106. In the depicted example, a lower surface of the plate 110 is in direct contact with the surface of the skin 106. In this case, the chamber 102 takes the form of an aperture delimited by the plate 110. The plate 110 may be formed of any suitable material, e.g. a polymer, capable of being disposed on the skin. For example, the plate 110 may have at least a degree of flexibility so as to enable conformal application to the surface of the skin 106. More rigid plates 110 may also be contemplated, providing the inlet 104 can receive sweat from the skin 106.

In order to collect sweat from a subject, the plate 110 may, for instance, be adhered to the surface of the skin 106 using a suitable biocompatible adhesive. Alternatively, the plate 110 may be held against the surface of the skin 106 by fastenings, e.g. straps, for attaching the plate 110 to the body of the subject.

In an embodiment, each inlet 104 of the plurality of chambers 102 is dimensioned to receive sweat from, on average, 0.1 to 1 active sweat glands. This may assist the apparatus 100 to be used for determination of the sweat rate per sweat gland, as will be explained in more detail herein below.

Each inlet 104 may, for example, have an area between 0.005 mm$^2$ to 20 mm$^2$. The inlet area may be selected according to the other dimensions of the chambers 102, and the number of chambers 102 included in the apparatus 100. The rationale behind the inlet area and dimensions will be discussed in greater detail herein below.

It is preferable that the diameter of the inlet 104 for receiving sweat from the skin 106 is selected to be relatively small, for example 200-2000 $\mu$m, such as 300-1200 $\mu$m, e.g. about 360 $\mu$m or about 1130 $\mu$m. The diameter of sweat gland outlets on the surface of the skin 106 are typically in the range of about 60 $\mu$m to 120 $\mu$m. A relatively small inlet 104 may assist to reduce the chances of two or more sweat glands 108 excreting into the same inlet 104, which can complicate interpretation of sensor signals, as will be explored in further detail below. To compensate for the limited amounts of sweat being received into an individual chamber 102, the apparatus 100 may, for instance, include a plurality of such chambers 102, for example 2 to 50 chambers 102, such as 10 to 40 chambers 102, e.g. about 25 chambers 102.

Once the chamber 102 has been filled with sweat, a sweat droplet 112 protrudes from an outlet 114 of the chamber 102. In the example shown in Fig. 1, the outlet 114 is delimited by an upper surface of the plate 110, and a hemispherical sweat droplet 112 forms on top of the outlet 114 once the chamber 102 has been filled with sweat.

More generally, the apparatus 100 may be configured such that the speed of formation of the sweat droplet 112 is determined by the sweat rate, while the volume of the sweat droplet 112 is determined by the fluid transport assembly. This will be explained in further detail.

The respective areas of the inlet 104 and the outlet 114 may be selected to ensure efficient filling of the chamber 102 and sweat droplet 112 formation over a range of sweat rates. In some examples, the inlet 104 and the outlet 114 have selected fixed dimensions for this purpose. Alternatively, the apparatus 100 may be configurable such that at least some of the dimensions and geometry relevant to sweat droplet 112 formation can be varied.

In a preferred example (not shown in Fig. 1), the chamber 102 is dimensioned to fill up with sweat within 10-15 minutes. The formation of the hemispherical sweat droplet 112 following filling of the chamber 102 preferably occurs typically within 10 seconds at relatively low sweat rate, e.g. 0.2 nl/min/gland.

The diameter of the outlet 114 may, for example, be in the range of 10 $\mu$m to 100 $\mu$m, e.g. 15 $\mu$m to 60 $\mu$m, such as about 33 $\mu$m, in order to assist in controlling the sweat droplet 112 size so that its volume is uniform and reproducible. By the outlet 114 having such a diameter, e.g. about 33 $\mu$m, several sweat droplets 112 may be formed during a single sweat burst (typically lasting 30 seconds) of a sweat gland 108, even with sweat rates as low as 0.2 nl/min/gland. Consequently, sufficient sweat droplets 112 may be generated and transported by the apparatus 100 to the sensor in order for the sweat rate to be reliably estimated.

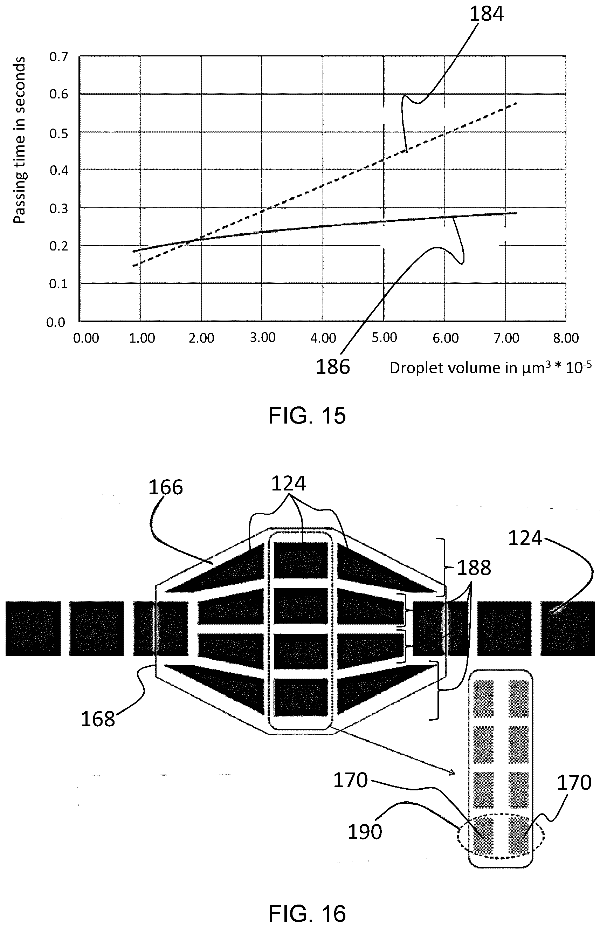

The apparatus 100 may enable the formation of relatively uniformly sized sweat droplets 112, and in addition may handle variable sweat droplet 112 volumes as well. Regarding the latter, the sensor to which the apparatus 100 transports the sweat droplets 112 may be configured to both count the sweat droplets 112 and determine the time it takes for each sweat droplet 112 to pass through the sensor. This time is linearly related via the *a priori* known migration speed to the volume of the sweat droplet 112, as will be explained in more detail below with reference to Fig. 15.

As an indication of the scale of the part of the exemplary apparatus 100 shown in Fig. 1, the length 116 (denoted by the double-headed arrow) is about 500 $\mu$m. More generally, the dimensions of the chamber 102, inlet 104, and outlet 114 may be selected according to, for instance, the sweat rate of the subject. The volume of the chamber 102 may be minimized in order to decrease the filling time. This may assist to ensure a minimal delay between actual sweat excretion and sensing/monitoring of the sweat droplets 112. For example, the volume of the chamber 102 may be in the range of 0.1-100 nl, such as 0.5-50 nl, e.g. 1-20 nl.

The volume of the chamber 102 may be minimized in various ways in order to minimize the time required to fill the chamber 102 with sweat. Such modifications may be, for instance, to the plate 110 delimiting the chamber 102.

Fig. 2 shows an example in which the chamber 102 tapers from the inlet 104 towards the outlet 114. The volume of such a tapering chamber 102 will be less than, for example, a cylindrical chamber 102, such as that of the apparatus 100 shown in Fig. 1, having the same height and base diameter dimensions.

By illustration, the length dimension 118 of the plate 110 shown in Fig. 2 is about 500 $\mu$m. The tapering chamber 102 in this example has a conical geometry, i.e. having a truncated cone shape with a volume of $1/3\pi h[R^2 + Rr + r^2]$ (h = 50 $\mu$m; R = 360 $\mu$m; r = 33 $\mu$m). For a relatively low sweat rate of 0.2 nl/min/gland, the filling time of this tapering chamber 102 may be about 10 minutes and the sweat droplet 112 formation may take around 12 seconds. By contrast, filling of a cylindrical chamber 102 shown in Fig. 1 having the same height (50 $\mu$m) and base (360 $\mu$m) dimensions may take around 50 minutes, and the formation time of the hemispherical sweat droplet 112 may be more than 3 hours.

At this point it is noted that sweat glands 108 tend to excrete in sweat bursts, each sweat burst being followed by a rest period in which the glands 108 are not excreting. During the sweat burst period the sweat rate may be about six times larger than the average sweat rate. The reason is that in a time window of 180 seconds there is typically a sweat burst of 30 seconds and a rest period of typically 150 seconds, hence there is a factor of six between the average sweat rate and the sweat rate during a sweat burst. In the above illustrative example of a chamber 102 having a truncated conical shape, the time to form the depicted sweat droplet 112 is about 12 seconds during the sweat burst of the sweat gland 108.

In the example shown in Fig. 2, 56% of the surface area of the sweat droplet 112 is in contact with the upper surface of the plate 110. The upper surface of the plate 110 may be provided with a gradient, such as a topological and/or chemical gradient, for the purpose of releasing the sweat droplet 112 from the outlet 114 and transporting the sweat droplet 112 to the sensor, as will be discussed further herein below in relation to the fluid transport assembly. Suffice to say at this point that, in the case of such a topological and/or chemical gradient, the surface area of the sweat droplet 112, in contact with the upper surface of the plate 110, required for releasing the sweat droplet 112 from the outlet 114 may depend on the steepness of the chemical and/or topological gradient, and the volume of the sweat droplet 112.

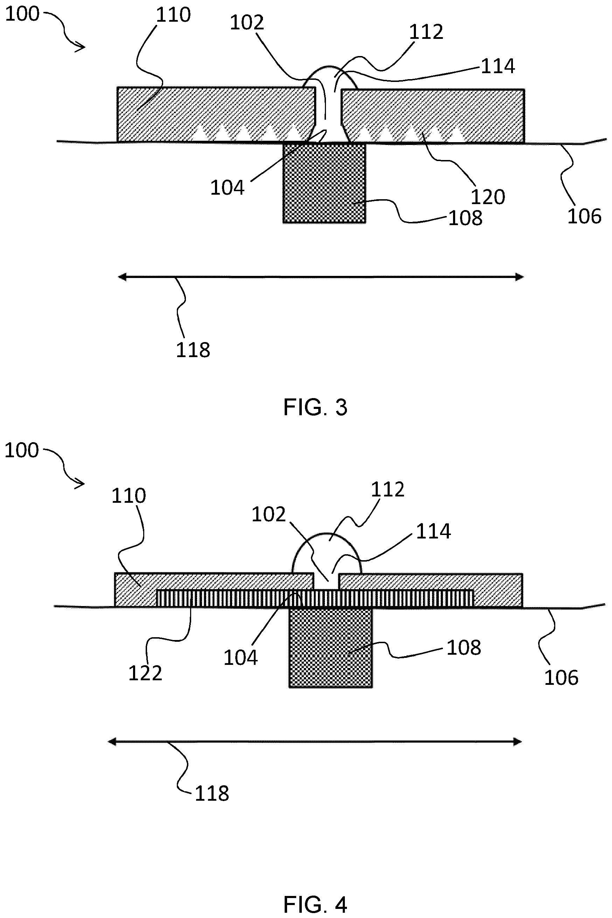

Fig. 3 shows another example of how the volume of the chamber 102 may be minimized. In this example, the chamber 102 is partitioned into compartments, at least some of the compartments being fluidly connected to each other in order to permit the chamber 102 to be filled with sweat. As shown in Fig. 3, the compartments may be formed by pillars 120. Such pillars 120 may form part of the plate 110, and in such an example may be formed by patterning, e.g. etching, the lower surface of the plate 110. Other suitable ways of forming such pillars 120 will be readily apparent to the skilled person.

Fig. 4 schematically depicts another example in which a porous material 122, e.g. a frit-like material, such as a sintered glass material, partitions the chamber 102 into compartments. The volume of the chamber 102 may be decreased due to the space occupied by the partitions between the pores of the porous material 122. Depending on the shape of the chamber 102, and the degree to which the material partitioning the pores occupies the chamber 102, the filling time of the chamber 102 may be, for instance, reduced by to 1-4 minutes.

The porous material 122 may further serve as a filter for species, such as aggregated proteins, which may otherwise block downstream components of the apparatus 100, such as the outlet 114 or the fluid transport assembly. In addition, the porous material 122 may assist to prevent fouling of the apparatus 100 by certain sweat components and impurities. The porous material may, for instance, be selected to have specific adsorption properties for proteins and other species which it may be desirable to remove from the sweat entering or being contained within the chamber 102. Removing such impurities may be advantageous due to lessening the risk of the impurities altering the surface properties in the fluid transport assembly, e.g. due to adsorption onto a surface of the fluid transport assembly, such as on the electrowetting tiles of an electrowetting arrangement (when such an electrowetting arrangement is included in the fluid transport assembly). Thus, the porous material may assist to mitigate the risk that such impurities impair the hydrophilic/hydrophobic balance required for release of the sweat droplets 112 from the outlet 114, and migration of the sweat droplets 112 to the sensor.

In examples where the porous material 122 comprises, or is, an incompressible frit-like material positioned adjacent the surface of the skin 106, the porous material 122 may prevent, partly due to its incompressibility, blockage by bulging of skin 106 into the chamber 102.

The diameter of the pores of the porous material 122 may be, for instance, in the range of 100 nm to 10 $\mu$m. The diameter of the partitions between the pores may also, for instance, be in the range of 100 nm to 10 $\mu$m, in order to minimize the risk that such partitions themselves block the exit of a sweat gland 108. In this respect, the exit diameter of sweat glands 108 is typically in the range of about 60 $\mu$m to 120 $\mu$m.

It should be noted for the avoidance of doubt that the volume minimizing measures described with reference to Figs. 1-3 may be used in any combination in order to minimize the volume of the chamber 102, so as to assist sweat droplet 112 formation even at relatively low sweat rates. For example, the apparatus 100 may comprise a tapering chamber 102 which is also partitioned into compartments, e.g. by inclusion of a pillared structure 120 and/or a porous material 122.

As noted above, the apparatus 100 comprises a fluid transport assembly which is arranged to enable release of the sweat droplet 112 protruding from the outlet 114. The fluid transport assembly may thus, for example, comprise a structure which detaches the sweat droplet 112, e.g. the hemispherical sweat droplet 112, from the outlet 114.

A formed sweat droplet 112 may be anchored to the bulk of sweat which has filled the chamber 102 due to the attractive intermolecular forces between the water molecules in the sweat.

In practice, the sweat droplet 112 does not have a single contact angle value, but rather a range from a maximum to a minimum contact angle, which are called the advancing contact angle and the receding contact angle, respectively. The difference between the advancing and receding contact angles is known as contact angle hysteresis.

These forces resist movement of the sweat droplet 112 from the outlet 114. Such forces lead to retention of the sweat droplet 112 above the filled chamber 102. The fluid transport assembly enables these forces to be overcome, such as to detach the sweat droplet 112 (and transport the sweat droplet 112 downstream towards the sensor). The fluid transport assembly may be configured to enable a well-defined dislodgement of the sweat droplet 112 from the chamber 102. In other words, detachment of the sweat droplet 112 ensures unambiguous discrete sweat droplet 112 definition.

The fluid transport assembly may, for instance, be provided with a passive and/or an active gradient for dislodging, i.e. releasing, the sweat droplet 112. The passive gradient may include a chemical and/or a topological gradient. The active gradient may be provided by an applied pressure and/or by an electric field of an electrowetting arrangement.

The detachment or release of the sweat droplet 112 may in some examples occur at the moment that the sweat droplet 112 reaches a certain diameter. At that diameter, an active and/or a passive gradient, e.g. which may be experienced by at least part of, and preferably the entirety of, the sweat droplet 112, may be sufficiently large to overcome the contact angle hysteresis of the sweat droplet 112, such that the sweat droplet 112 is released from the outlet 114.

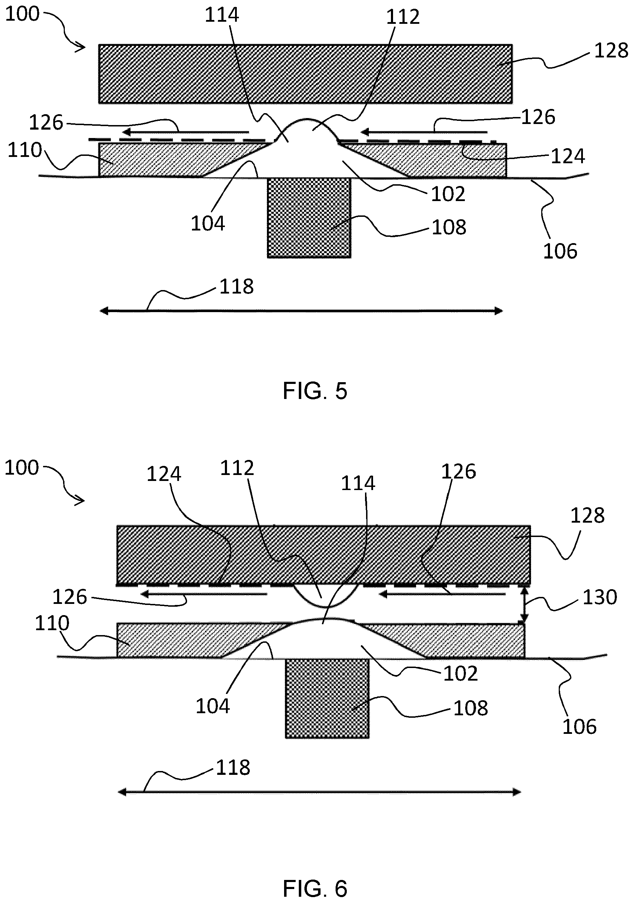

Fig. 5 schematically depicts an example in which sweat droplet 112 detachment is effected by an electrowetting technique. This may be regarded as an example of an "active" interfacial tension method, in which a force is actively applied in order to overcome the contact angle hysteresis of the sweat droplet 112.

As shown in Fig. 5, the upper surface of the plate 110 is provided with a series of discrete electrowetting tiles 124. For the purpose of detaching from the outlet 114 and/or transporting an aqueous sweat droplet, the electrowetting tiles 124 may comprise an electrode which is coated with a hydrophobic material, such as a fluoropolymer. The transport assembly may comprise an electric field generator (not shown) for charging and discharging each of the electrowetting tiles 124 of the series in sequence. Charging of an electrowetting tile 124 may cause the surface properties of the electrowetting tile 124 to switch from hydrophobic to hydrophilic, thereby to instantaneously overcome the contact angle hysteresis of the sweat droplet 112. The sweat droplet 112 may correspondingly migrate onto the charged electrowetting tile 124. Subsequent discharge of the charged electrowetting tile 124 and charging of the subsequent electrowetting tile 124 in the series may cause the sweat droplet 112 to migrate to the subsequent electrowetting tile 124, and so on. This sequence may be regarded as an "electrowetting wave".

In the example shown in Fig. 5, detachment from the outlet 114 may occur when the sweat droplet 112 has grown to acquire a sufficiently large diameter that the sweat droplet 112 at least partially overlaps a pair of electrowetting tiles 124. In this case, once an electrowetting wave passes along the electrowetting tiles 124, the sweat droplet 112 spanning the pair of electrowetting tiles 124 will be dislodged from the outlet 114 accordingly. In this example, the sweat droplets 112 may not be all of a uniform size or volume, because the sweat droplet 112 may continue to grow to varying degrees in the period between the sweat droplet 112 reaching the requisite diameter and the arrival of the electrowetting wave. In this respect, the sweat droplet 112 size may be determined by the frequency of the electrowetting wave.

In an alternative example, the fluid transport assembly may employ a "passive" gradient to release the sweat droplet 112 from the outlet 114. The term "passive" in this context means, in general terms, that the fluid transport assembly does not actively apply a force in order to overcome the contact angle hysteresis of the sweat droplet 112.

For instance, the upper surface of the plate 110 may be provided with a chemical and/or topological gradient which enables detachment of the sweat droplet 112 from the outlet 114. The topological gradient may be provided by the upper surface of the plate 110 being inclined, such that, when the apparatus 100 is orientated for use, the gradient of the incline spanning the sweat droplet 112 diameter is sufficiently large to overcome the contact angle hysteresis.

The chemical gradient may be provided by the surface having hydrophilic and hydrophobic moieties thereon, which moieties are arranged to provide a wettability gradient along the surface. For example, microfluidic channels functionalized with hydrophobic $CH_3$- moieties (towards the skin 106) and hydrophilic OH-moieties (towards the sensor) may be used to create a chemical gradient (Morgenthaler et al, Langmuir; 2003; 19(25) pp 10459-10462).

The chemical gradient may be, for example, provided with hydrophilic/hydrophobic domains at the molecular level, such that the wettability gradient varies substantially continuously along the surface. Such a chemical gradient may, for instance, be provided by grafted polymer chains functionalizing the surface of the plate 110. Alternatively or additionally, hydrophilic/hydrophobic domains of $\mu$m dimensions may be provided on the surface such as to provide a stepwise wettability gradient. Preferably, the domains are arranged to have a gradual change in distribution over the length of the surface in the direction of the sensor.

When such a passive, e.g. chemical and/or topological, gradient is employed for detachment of the sweat droplet 112, detachment may occur when the sweat droplet 112, e.g. the hemispherical sweat droplet 112, reaches a certain size. Once the diameter of the sweat droplet 112 is such that the gradient spanning the diameter is sufficiently large to overcome the contact angle hysteresis, the sweat droplet 112 will become detached from the outlet 114. In this sense, such a gradient may result in each of the sweat droplets 112 being transported to the sensor having a similar size/volume relative to each other. After the sweat droplet 112 is detached, viscous drag may also play a role in retarding sweat droplet 112 motion due to the driving force created by the surface energy gradient.

In the examples shown in Figs. 5 and 6, the fluid transport assembly comprises a further plate 128 which is separated from and opposes the plate 110 delimiting the chamber 102. The further plate 128 may enable control to be exerted over the volume of the sweat droplet 112. This may be achieved, for instance, by the further plate 128 being separated from the plate 110 by a defined distance 130. The sweat droplet 112 may increase in size until it makes contact with the further plate 128. In practice, when the sweat droplet 112 contacts the further plate 128, the sweat droplet 112 may become detached by "jumping" over to the further plate 128. This may be regarded as a further example of an interfacial tension method for detaching the sweat droplet 112 from the outlet 114.

Referring to Fig. 6, the separation distance 130 between the plate 110 and the further plate 128 may be selected such that it is large enough to ensure that the diameter of the forming sweat droplet 112, e.g. hemispherical sweat droplet 112, is sufficiently large before contacting the further plate 128, i.e. the lower surface of the further plate 128 which opposes the upper surface of the plate 110.

In the example shown in Fig. 6, the lower surface of the further plate 128 may be provided with a passive (e.g. chemical and/or topological) and/or active (e.g. via an applied pressure and/or an electric field) gradient for transporting the sweat droplet 112 towards the sensor, in the direction of the arrows 126. Following detachment by "jumping" of the sweat droplet 112 to the further plate 128, the sweat droplet 112 may start to migrate via the gradient. As briefly described above, the separation distance 130 between the plate 110 and the further plate 128 may be selected to ensure that the diameter of the forming sweat droplet 112 is sufficiently large before contacting the further plate 128. This may assist to ensure immediate migration/transport of uniformly sized sweat droplets 112.

As shown in Fig. 6, the lower surface of the further plate 128 is provided with electrowetting tiles 124. The electrowetting tiles 124 and the electric field generator (not shown) may be similar to the arrangement described above in relation to Fig. 5. However, in the case of Fig. 6, migration of the sweat droplet 112 on the further plate 128 via the electrowetting tiles 124 may mean that sweat droplet 112 migration will occur when the next electrowetting wave reaches the sweat droplet 112 which has been released onto the further plate 128. Accordingly, a sufficiently high electrowetting wave frequency may ensure transport/migration of sweat droplets 112 of relatively uniform size/volume to the sensor. On the other hand, if the frequency of the electrowetting waves is relatively slow, the size/volume of the sweat droplet 112 may be determined by both the electrowetting wave frequency and the separation 130 of the plate 110 and the further plate 128, i.e. since the sweat droplet 112 may grow in the period between electrowetting waves.

In a non-limiting example, the fluid transport assembly may be configured to control the separation 130 between the plate 110 and the further plate 128. This may, for instance, be achieved by the fluid transport assembly comprising a mechanism which engages at least one of the plates 110, 128, which mechanism is configured to move at least one of the plates 110, 128 such as to adjust the separation of the plates 110, 128. The control exerted over the mechanism may be manual and/or automatic. Regarding the automatic control, the fluid transport assembly may, for example, control the separation 130 according to the sweat rate of the sweat gland 108. In such an example, the fluid transport assembly may include a controller configured to control the mechanism to move at least one of the plates 110, 128 according to a determined sweat rate, e.g. as detected by the sensor. Thus, the fluid transport assembly may be configured to control the separation 130 in a dynamic manner.

At relatively high sweat rates the sweat droplet 112 formation may risk being too rapid, and uncontrollable sweat droplet coalescence may occur. This may be mitigated by increasing the separation 130, since it may take a longer time to detach a larger sweat droplet 112 onto the further plate 128.

At relatively low sweat rates, the number of sweat droplets 112 transported to the sensor may be relatively low. This issue may be alleviated by decreasing the separation 130 in order to increase the number of (smaller) sweat droplets 112 formed on the further plate 128.

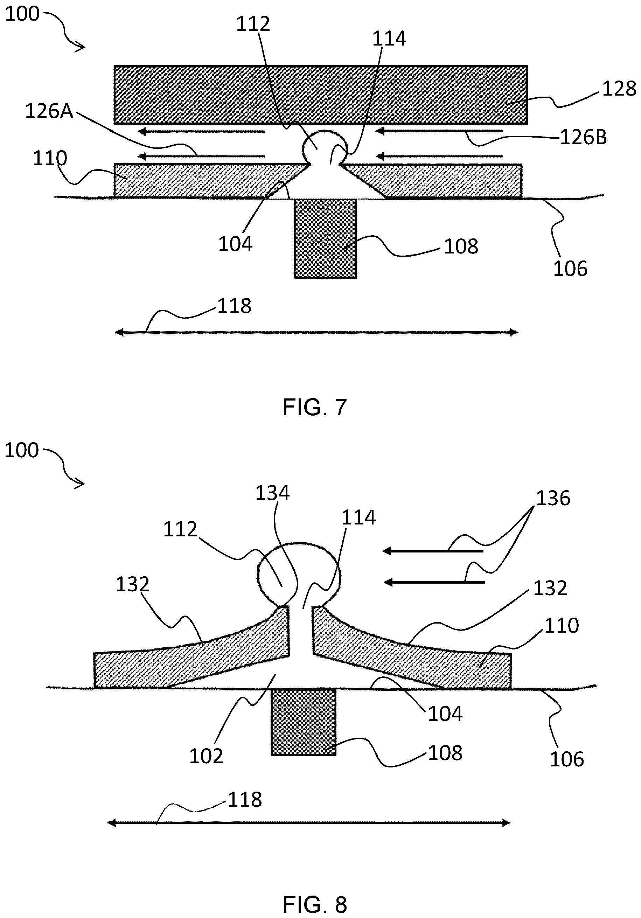

Fig. 7 shows an example in which sweat droplet 112 detachment is achieved with a fluid transport assembly in which the upper surface of the plate 110 and the lower surface of the further plate 128 are both provided with a passive gradient, e.g. a chemical and/or topological gradient. In this respect, the arrows 126A and 126B respectively denote the direction of the gradient provided on the upper surface of the plate 110 and the lower surface of the further plate 128 for transporting the sweat droplet 112 towards the sensor.

Defined sweat droplet 112 detachment may alternatively or additionally be achieved by the fluid transport assembly applying a pressure gradient to the sweat droplet 112 protruding from the outlet 114. This may be considered as an example of providing an active gradient in order to overcome the contact angle hysteresis of the sweat droplet 112, since the fluid transport assembly actively applies a pressure/force to the sweat droplet 112 in order to overcome the contact angle hysteresis of the sweat droplet 112.

The pressure gradient may, for example, be applied by contacting the protruding sweat droplet 112 with a flow of carrier fluid. The carrier fluid is preferably a fluid with which the sweat droplet 112 is immiscible. By virtue of the sweat droplet 112 being thus substantially prevented from mixing with the carrier fluid, the sensor may be able to detect each discrete sweat droplet 112 being carried thereto by the carrier fluid. Suitable examples of such a carrier fluid include oils that do not absorb moisture, i.e. have relatively low or negligible hygroscopicity, such as oxycyte. Oxycyte is a perfluorocarbon compound which is commonly used as a blood replacement.

In such an example in which a carrier fluid flow detaches the sweat droplet 112, a further plate 128 may be provided opposing the plate 110 delimiting the chamber 102, as previously described. The sweat droplet 112 may form and grow until the sweat droplet 112 makes contact with the further plate 128, whereupon the sweat droplet 112 may block the passage defined by the space between the respective plates 110, 128. The sweat droplet 112 may then be displaced by the flow of carrier fluid. In this manner, relatively uniformly sized sweat droplets 112 may be afforded; their size being determined by the distance 130 between the plates 110, 128, as previously described in relation to Fig. 6. The flow of carrier fluid may further assist in transporting the sweat droplets 112 to the sensor.

In cases where, for example, this flow of carrier fluid is insufficient to detach the sweat droplet 112, the fluid transport assembly may be configured to induce pulses or peaks in the flow rate, which pulses may provide sufficient pressure to release the sweat droplet 112 from the outlet 114. A piezoelectric pump may, for instance, be used to induce such peaks in the flow rate of the carrier fluid. This may be straightforwardly achieved by varying the pulse frequency of the pump.

Fig. 8 schematically depicts an example apparatus 100 in which the fluid transport assembly is configured to provide an active gradient, e.g. via an applied electric field in the context of an electrowetting arrangement. Moreover, the plate 110 delimiting the outlet 114 has a contoured (upper) surface 132, with the outlet 110 of the chamber 102 being arranged at a summit 134 of the contoured surface 132.

When the fluid transport assembly applies pressure to the sweat droplet 112 via a flow of carrier fluid, as denoted by the arrows 136, the flow may be directed to the protruding sweat droplet 112 at the summit 134 of the contoured surface 132. As shown in Fig. 8, the chamber 102 may include a narrower neck region which extends to the summit 134 of the contoured surface 132. This structure may facilitate detachment of the sweat droplet 112 since less energy is required to overcome droplet inertia caused by contact angle hysteresis. The latter is particularly so if the contoured surface is hydrophobic.

This structure may be considered as a passive supporting structure, and can be termed a "stalk" structure, with the outlet 114 being positioned atop the stalk, which stalk delimits the neck of the chamber 102. As described above, this structure may assist with sweat droplet 112 detachment, particularly when utilizing an active pressure gradient. Accordingly, this supporting structure may be advantageously used in the context of the active pressure gradient between the plate 110 and the further plate 128, as described above in relation to Fig. 7.

This contoured "stalk" structure may be fabricated in any suitable manner. For example, micromachining techniques, such as deep reactive ion etching (DRIE), lithography, electroplating and molding (LIGA), wet etching, fused deposition modelling (FDM), projection micro-stereo-lithography, and direct-write additive manufacturing, may be employed (KS Teh. Additive direct-write microfabrication for MEMS: A review. Front. Mech. Eng. 2017; 12(4):490-509).

Following detachment of the sweat droplet 112, the sweat droplet 112 is transported via the fluid transport assembly to the sensor, e.g. a sweat rate sensor and/or a biomarker sensor. The sensor may, for example, comprise a cell through which the sweat droplet 112 may be transported. In such an example, the fluid transport assembly may effect transportation or migration of the sweat droplet 112 to and through the sensor.

The released sweat droplet 112 is transported at least as fast as the subsequent sweat droplet 112 protrudes from the outlet 114. Preferably, migration of a sweat droplet 112 is faster than formation, i.e. the protruding, of the subsequent sweat droplet 112. This is in order to ensure unambiguous sweat droplet 112 definition, i.e. to ensure transport of a train of discrete sweat droplets 112 to the sensor.

In other words, the fluid transport assembly may maintain the discrete droplet characteristics of the sweat droplets 112 by ensuring rapid transport/migration relative to sweat droplet 112 formation. This may have advantages over a continuous flow of sweat, especially at low sweat rates, in terms of lessening or avoiding diffusion of components, such as biomarkers, between sweat samples collected at different points in time.

The biomarker concentration in each sweat droplet 112 may be primarily or solely determined by the size/volume of the sweat droplet 112, which leads to relatively straightforward measurement, as will be described further herein in relation to Fig. 15. Accordingly, when the sensor to which the train of discrete sweat droplets 112 is transported is configured to enable detection of the concentration of a biomarker in the respective sweat droplets 112, the biomarker concentration as a function of time may be determined, with less possibility of error associated with diffusion of biomarkers between sweat sampled at different points in time. Such accumulation effects may otherwise lead to a lower biomarker concentration being measured than the actual concentration in sweat sampled during a particular time period. Thus, the apparatus 100 may offer a means of overcoming a key disadvantage of conventional continuous flow sweat monitoring.

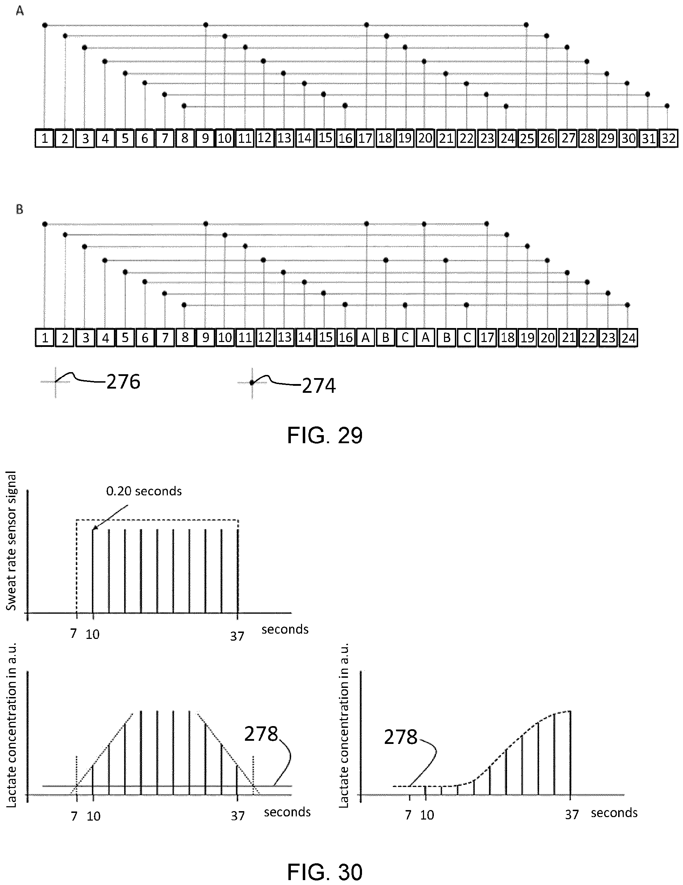

Sweat glands 108 operate in a cyclic manner. The sweat glands 108 typically excrete for typically 30 seconds during a sweat burst period, followed by a rest period of about 150 seconds. The capability of the apparatus 100 to enable determination of detailed information concerning biomarker concentrations as a function of time, by virtue of the discretized flow of sweat droplets 112 supplied to the sensor by the fluid transport assembly, may apply even during the sweat burst period of a sweat gland 108. The sweat gland bursts may correspondingly be detected, which may have various advantages, including the capability to determine lactate concentrations as will be described in more detail herein below with reference to Figs. 29 and 30.

The relatively rapid transport of the sweat droplets 112 to the sensor via the fluid transport assembly may also assist to alleviate/minimize the problem of evaporation of the sweat as it migrates to the sensor. Such evaporation can, in the most severe case, prevent the sweat droplet 112 from reaching the sensor. Ensuring minimal evaporation of the sweat droplets 112 is especially important at low sweat rates/volumes.

Similarly to the above description of detachment of the sweat droplet 112, the fluid transport assembly may be provided with a passive and/or active gradient for transporting the detached sweat droplet 112 to the sensor. Thus, transport of the sweat droplet 112 may be via an interfacial tension technique and/or by application of pressure, as will be further described herein below.

A notable advantage associated with the passive gradient is that no power is required to be supplied to the fluid transport assembly for transporting the discrete sweat droplets 112 to the sensor.

When a chemical gradient is employed, the length of the chemical gradient trajectory may be restricted on one hand by the limited range in hydrophilicity/hydrophobicity balance reaching a lower contact angle of about 20° and an upper contact angle of 170°, and on the other hand by the size of the sweat droplet 112 being transported/migrated. Smaller sweat droplets 112 may require a steeper gradient than larger sweat droplets 112. In particular, relatively small sweat droplets 112 necessitate steeper gradients in order to initiate movement of the sweat droplet 112, due to the relatively small length of the sweat droplet 112 effectively restricting the exposure of the sweat droplet 112 to the gradient.

Transport distances of, for example, 5-10 mm are achievable and, in principle, are sufficient in practical terms for the apparatus 100 to function, depending on the size of the sweat droplets 112.

With a view to minimizing the contact angle hysteresis of the sweat droplet 112, the chemical gradient may be formed in various ways. As described above in relation to detachment of the sweat droplet 112 via a chemical gradient, a stepwise chemical gradient may, for instance, be employed. Preferably, the domains are arranged to have a gradual change in distribution over the length of the surface in the direction of the sensor. Such a stepwise gradient may, for example, be fabricated from hydrophobic domains formed of nanopillars and hydrophilic domains formed of siloxy species. In practice the chemical gradient may result in contact angles between about 15° to about 166°, which are typical for superhydrophilicity and superhydrophobicity.

In an alternative example, the chemical gradient may be provided with hydrophilic/hydrophobic domains at the molecular level, such that the wettability gradient varies substantially continuously along the surface. Such a chemical gradient may, for instance, be provided by grafted polymer chains functionalizing the surface of the plate 110, as discussed above in relation to detachment of the sweat droplet 112.

These examples are underpinned by the theory of chemical wettability gradients which governs the movement of droplets along a solid surface with an alternating contact angle. Such an alternating contact angle may be formed by varying the chemical composition of the solid surface, as previously described, thereby to attain a chemical anisotropy on the surface. The resulting wettability gradient changes the surface tension forces at the liquid-solid interface. Since the sweat droplet 112 tends towards minimizing its surface energy, it moves from less wettable (larger water contact angle) towards more wettable (smaller water contact angle) regions.

A sweat droplet 112 placed on a horizontal chemical wettability gradient surface is subjected to two main, counteracting forces: driving force and viscous drag. The driving force is created by the surface energy gradient which promotes the motion of the sweat droplet 112 whereas viscous drag opposes the motion of the sweat droplet 112. The contact angle hysteresis acts as a resistant force to the movement, trying to retain the sweat droplet in its static position. The sweat droplet 112 accelerates under the resultant force of these opposing forces.

Estimates of the sweat droplet 112 velocities that can be achieved via a chemical wettability gradient have been obtained from a theoretical model. This model estimates that for a water droplet of 100 $\mu$m in diameter (size $\approx$0.26 nl) and 4° hysteresis with a hydrophobic contact angle of 150°, if the chemical gradient is assumed to be linear, the wettability gradient minimum may be $d\cos\theta/dx = 0.83$ mm$^{-1}$, in order to overcome the hysteresis and thereby start droplet motion. Given that the air-water surface tension is 0.072 N/m and the dynamic viscosity of water is $8.8871 \times 10^{-4}$ Pa.s, in this specific example the theoretical model also predicts a droplet velocity ranging between 0.6 - 10 cm/s.

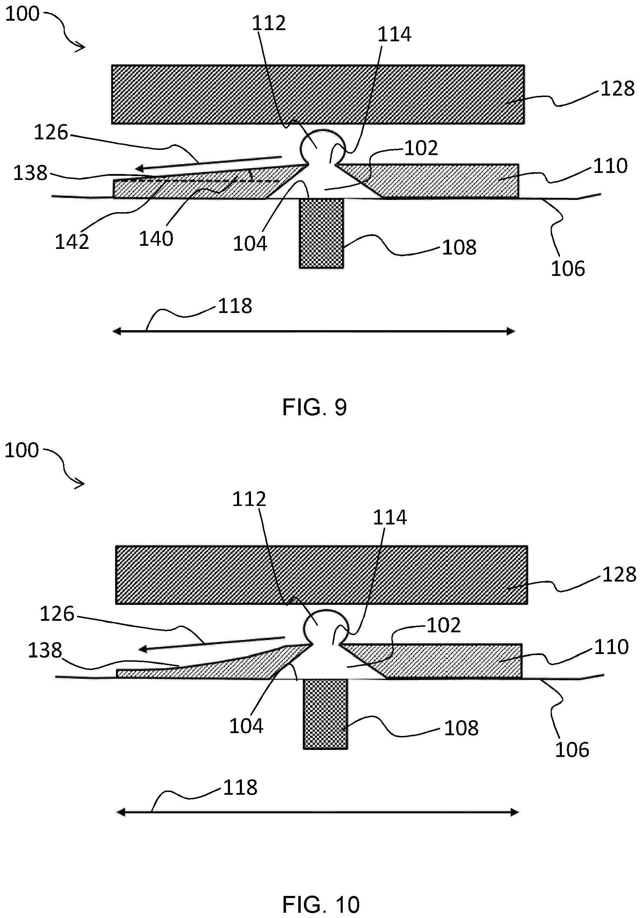

Fig. 9 schematically depicts an exemplary apparatus 100 in which the fluid transport assembly is provided with a passive topological gradient. In this example, the topological gradient is provided by an inclined surface 138. The inclined surface 138 inclines, i.e. climbs, towards the outlet 114, such that, when the apparatus 100 is orientated for use, e.g. horizontally as shown, the sweat droplet 112 is transported down the inclined surface 138 in the direction of the sensor.

In the example shown in Fig. 9, the inclined surface 138 corresponds to an upper surface of the plate 110. The angle of incline 140 relative to a horizontal 142 is selected to be sufficiently large to overcome the contact angle hysteresis of the sweat droplet 112.

Whilst the inclined surface 138 shown in Fig. 9 has a linear (topological) gradient, alternative types of inclined surface may be contemplated. In this respect, Fig. 10 schematically depicts an apparatus 100 having an inclined surface 138 in the form of a slope having portions with different angles of incline.

Whilst in Figs. 9 and 10 the inclined surface 138 is shown as an outer or external surface of the plate 110, this is not intended to be limiting. It is preferred that a sloping channel or internal passage provided within the plate 110 comprises the inclined surface 138.

More generally, and whilst not visible in the cross-sectional representations provided in Figs. 1-10, the passages included in the fluid transport assembly for transporting the sweat droplets 112 toward the sensor may be at least partially, and preferably fully, enclosed in order to minimize evaporation of the sweat droplets 112.

The inclined surface 138 may be formed in any suitable manner, such as by application of various micromachining techniques, e.g. deep reactive ion etching (DRIE), lithography, electroplating and molding (LIGA), bead blasting with or without wet etching, fused deposition modelling (FDM), projection microstereolithography, and direct-write additive manufacturing, and so on.

Passive migration of the sweat droplets 112 may, in practice, be achieved by a combination of the above-described chemical and topological gradients.

Alternatively, the fluid transport assembly may be provided with an active gradient, for instance, a pressure gradient in order to transport the sweat droplets 112. The pressure gradient may, for example, be applied by contacting the sweat droplet 112 with a flow of carrier fluid, as previously described in relation to detachment of the sweat droplet 112.

In examples where the carrier fluid and the sweat droplet 112 are immiscible with each other, the sensor may be able to detect each discrete sweat droplet 112 being carried thereto by the carrier fluid. Suitable examples of such a carrier fluid include oils that do not absorb moisture, i.e. have relatively low or negligible hygroscopicity, such as oxycyte.

The fluid transport assembly may comprise a plate 110 opposing a further plate 128. In such an example, the sweat droplet 112 may form and grow until the sweat droplet 112 makes contact with the further plate 128, whereupon the sweat droplet 112 may block the passage defined by the space between the respective plates 110, 128. The sweat droplet 112 may then be displaced by the flow of carrier fluid, e.g. a constant flow of the carrier fluid, which is propelled by the pressure gradient. In this manner, relatively uniformly sized sweat droplets 112 may be transported to the sensor, their size being determined by the distance 130 between the plates, as previously described in relation to Fig. 5.

In cases where, for example, this flow of carrier fluid is insufficient to detach the sweat droplet 112, the fluid transport assembly may be configured to induce pulses or peaks in the flow rate, which pulses may provide sufficient pressure to release the sweat droplet 112 from the outlet 114. A piezoelectric pump may, for instance, be used to induce such peaks in the flow rate of the carrier fluid. This may be straightforwardly achieved by varying the pulse frequency of the pump.

The fluid transport assembly may, for example, include or be connectable to a reservoir of the carrier fluid (not shown), thereby to enable a continuous supply of carrier fluid during operation of the apparatus 100. Preferably, the fluid transport assembly is connectable to an external reservoir of carrier fluid which is not itself included in the apparatus 100, since for 24 hour continuous operation over a number of days, e.g. 7 days, a relatively large volume of carrier fluid, e.g. a liter or more, may be required.

In a non-limiting example, the carrier fluid, e.g. oil, may be circulated by a pump included in the fluid transport assembly, as previously described. The sweat may be separated from the carrier fluid following sensing, and passed to a waste receptacle (not shown) which collects the sweat. Separation of the sweat from the carrier fluid may be assisted when the sweat is immiscible in the carrier fluid. In this way the carrier fluid can be recycled which also reduces the volume of carrier fluid needed. The waste receptacle may, for instance, have a capacity to hold milliliter volumes of sweat.

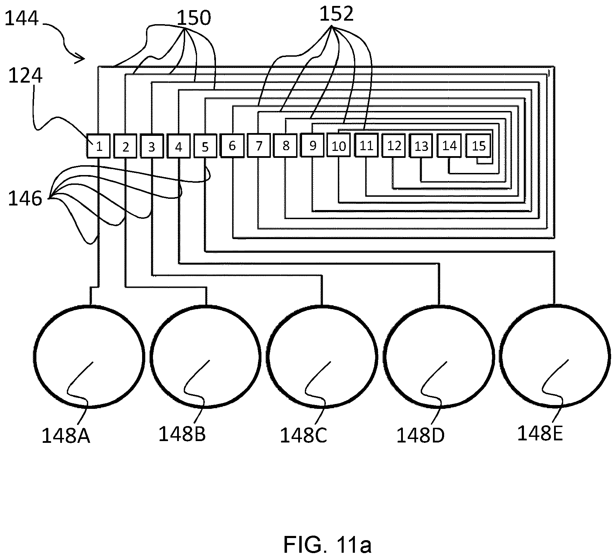

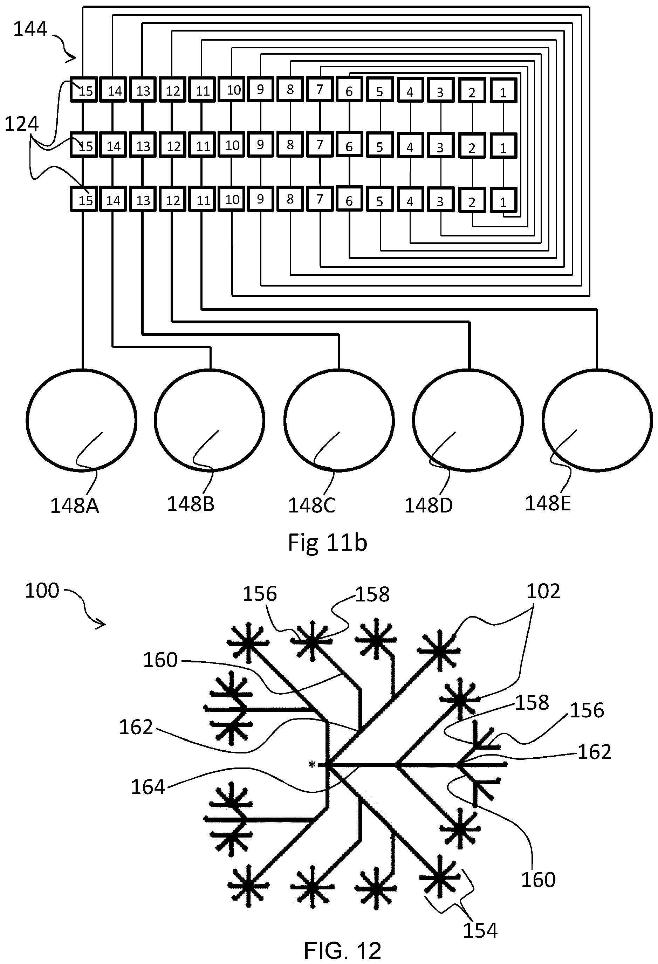

As described previously in relation to Figs. 5 and 6, the fluid transport assembly may comprise electrowetting tiles 124 (the term "electrowetting tiles" is an alternative for the term "electrowetting electrodes") and an electric field generator for detaching and transporting the sweat droplet 112. The electrowetting tiles 124 and the electric field generator may at least partly constitute an electrowetting arrangement 144, examples of which are schematically represented in Figs. 11a and 11b.

For transporting an aqueous sweat droplet 112, the tiles 124 each comprise an electrode which is coated with a hydrophobic material, such as a chloropolymer, for example parylene C, or a fluoropolymer, for example CYTOP®, and an electric field generator for charging/discharging the electrowetting tiles 124. Also parylene can be used as a hydrophobic material and also a layered coating of various substances can be used, such as sputtering tantalum pentoxide on the electrode, coated with parylene and finally with CYTOP®. The electrowetting arrangement 144 may operate in practice by the applied electric field causing electrowetting tiles 124 to become charged, and thereby switch from hydrophobic to hydrophilic, as previously described.

The electrowetting arrangement 144 may require control electronics and an energy source in order to actively transport the sweat droplet 112. Fig. 11a shows an example in which the electrowetting arrangement 144 may nevertheless be realized in a relatively simple manner.

In this example, only 3 to 8 controlled local voltages may be employed to generate multiple electrowetting waves. Such electrowetting waves may effect transport/migration of the sweat droplet 112 over the required, e.g. full, length of the fluid transport assembly, i.e. such that the sweat droplet 112 is transported to, and optionally through, the sensor.

Fig. 11a shows an electrowetting arrangement 144 comprising a planar two-dimensional electrode design. Fig. 11a illustrates for simplicity the connections for fifteen electrowetting tiles 124. However, the skilled person will nevertheless appreciate that this electrowetting arrangement 144 can be scaled-up to include, for example, one hundred or more electrowetting tiles 124.

Fig. 11a shows three sets of configurations: each first line 146 connects one of the electrowetting tiles numbered 1 to 5 to a respective external connection pad 148A-E; each second line 150 connects one of the electrowetting tiles numbered 1 to 5 to a respective electrowetting tile numbered 6 to 10; and each third line 152 connects one of the electrowetting tiles numbered 6 to 10 to a respective electrowetting tile numbered 11 to 15. The variation in the thickness of lines 146, 150 and 152 evident in Fig. 11a is only for guiding the eye of the reader.

The electrowetting arrangement 144 may be operated by applying a sequence of charge-discharge actions, e.g. each action being spaced by for example a tenth of a second, from the first pad 148A to the second pad 148B to the third pad 148C to the fourth pad 148D and finally to the fifth pad 148E. In this manner, an electrowetting wave may propagate from electrowetting tile 1 to electrowetting tile 15. The sequence may be repeated, such that the electrowetting wave repeatedly sweeps the array of electrowetting tiles 124. The sequence may also be reversed, having an electrowetting wave propagating from electrowetting tile 15 towards tile 1. The frequency at which the electrowetting wave passes along the array may at least partly determine the size/volume of the sweat droplet 112, as previously described in relation to Fig. 5.

Whilst Fig. 11a illustrates the electrowetting arrangement 144 with a two-dimensional electrode design, the same principle is applicable to a three-dimensional design, for example using vertical interconnect access (VIA) connections.

However, the electrical wiring in the same plane shown in Fig. 11a allows for a single structure in one plane, avoiding VIAs to other layers. This design may thus be relatively inexpensive to manufacture. The drawbacks of such a wiring design are that a substantial surface area is used, and the structure of the electrowetting pathway may be limited. For instance, the design shown in Fig. 11a may not support pathways with bifurcations, such as the branched structure shown in Fig. 12.

In the alternative example shown in Fig. 11b, parallel electrowetting paths are wired. It is noted that the direction of numbering of the electrowetting tiles is reversed in Fig. 11b with respect to Fig. 11a. An electrowetting wave proceeds from 1 to 15 and at tile 1 there is an outlet of a chamber 102. The reason for reversing the numbers is that on the side of tile 15 a sweat rate sensor may be provided.

Owing to the wiring pattern shown in Fig. 11b, respective electrowetting paths may, for example, converge in order to supply a single sweat rate sensor. Fig. 11b also shows that with an in-plane design multiple chambers 102 may be addressed.

More generally, the use of an electrowetting arrangement 144 in order to transport/migrate sweat droplets 112 may offer relatively rapid migration and precise control over the transport, e.g. velocities, of the sweat droplets 112. The propagation of the electrowetting wave may, in principle, be applied to transport sweat droplets 112 over relatively long distances. The latter advantage is also applicable to the examples in which the fluid transport assembly applies a pressure to the sweat droplet 112, although an energy source is required in both cases.

When electrowetting is employed for sweat droplet 112 migration, the gradient length is between two electrowetting tiles 124, and therefore a much stronger force may be achieved than with a chemical gradient. The choice of migration principle may nevertheless depend on the application intended for the apparatus 100. As noted above, migration of sweat droplets 112 via a chemical gradient does not require an energy source.

Whilst Figs. 1-10 show a single chamber 102 from which the sweat droplet 112 is transported to a sensor, the apparatus 100 may include a plurality of chambers 102, and the fluid transport assembly releases sweat droplets 112 protruding from the respective outlets 114 of the chambers 102, and transports the respective sweat droplets 112 to the sensor.

In such an example, the fluid transport assembly may transport the respective sweat droplets 112 by means of an interfacial tension method and/or via an applied pressure, as previously described.

The fluid transport assembly may fluidly connect the respective outlets of each of the plurality of chambers 102 to the sensor in parallel. By connecting each chamber 102 to the sensor in parallel, rather than in series, a fully formed migrating sweat droplet 112 from one chamber 102 does not pass the outlet 114 of another chamber 102 on its path towards the sensor. In this way, the parallel arrangement effectively prevents such a fully formed sweat droplet 112 from colliding with a partially formed sweat droplet 112 growing from the outlet 114 of a downstream chamber 102. Moreover, the parallel arrangement avoids that a fully formed migrating sweat droplet 112 is hindered, e.g. pinned, by the outlet 114 of a downstream chamber 102.

Fig. 12 schematically depicts an apparatus 100 in which respective sweat droplets 112 from a plurality of chambers 102 are transported via an arrangement of pathways defining a branched structure. The plurality of chambers 102 are arranged in groups 154. A subset of the plurality of chambers belong to each group. The groups 154 may be spatially separated from each other, such that each group 154 is supplied with sweat from an area of skin 106 which is spatially removed from the respective areas of skin supplying the chambers 102 of the other groups 154.

As shown in Fig. 12, first branches 156 fluidly connect each chamber 102 of the respective group 154 to a first interconnection 158. In this respect, such a first interconnection 158 may be provided for each group 154. Second branches 160 fluidly connect the first interconnections 158 to a respective second interconnection 162. As shown in Fig. 12, such a second interconnection may be provided per two or more groups. The second interconnections may be fluidly connectable to the sensor (not shown in Fig. 12).

Optionally, third branches 164 fluidly connect two or more of the second interconnections to a respective third interconnection, which is denoted by the asterisk in Fig. 12. The sensor may receive the sweat droplets 112 from each of the plurality of chambers 102 via this third interconnection.

The above-described interfacial tension methods may, for example, be employed to transport sweat droplets 112 along the respective branches of the apparatus 100 shown in Fig. 12. This may enable transportation of the sweat droplets 112 at relatively high speeds from the respective chambers 102 to the sensor. The speed of sweat droplet 112 migration being at least as fast as, and in this example preferably significantly faster than, sweat droplet 112 formation may lead to the requisite train of discretized sweat droplets 112 being transported to the sensor, as previously described.

Since none of the chambers 102 are downstream of any of the other chambers 102 in this branched structure, the risk of migrating sweat droplets 112 colliding with partially formed sweat droplets 112 protruding from respective outlets 114 is effectively removed. A fully formed sweat droplet 112 coalescing with a partially formed sweat droplet 112 may present difficulties due the difficulty in determining the respective contributions of the fully formed and partially formed sweat droplets 112 to the volume of the coalesced sweat droplet 112. Accordingly, by removing the risk of such collisions the branched structure may assist in reducing sweat sampling related ambiguities in determining the sweat rate per gland.

This branched structure also avoids that a fully formed migrating sweat droplet 112 is hindered, e.g. pinned, by the outlet 114 of a downstream chamber 102. Moreover, the branched structure may permit transporting of a relatively large number of sweat droplets 112 using a relatively compact apparatus 100.

The branched structure may mean that the number of sensors, e.g. sweat rate sensors, can be kept to a minimum, since the sweat droplets 112 originating from numerous chambers 102 are directed to the same destination. This may represent a key advantage over known solutions which employ a sensor for each sweat collection chamber.

Fully formed migrating sweat droplets 112 originating from different chambers 102 may collide with one another prior to reaching the sensor. For example, for an apparatus 100 having one hundred chambers 102, and 0.1 sweat glands 108 per chamber 102 on average, on average nine chambers 102 will produce sweat droplets 112 deriving from a single gland 108, and on average about zero to one chamber 102 will produce sweat droplets 112 deriving from two sweat glands 108.

As briefly mentioned above, the collision of fully developed sweat droplets 112 originating from single glands 108 can be relatively straightforwardly detected due to the increased size/volume of the coalesced sweat droplet passing through the sensor. This is particularly so given that the majority of sweat droplets 112 transported to the sensor will not have combined with others, thereby providing a baseline. The sensor to which the apparatus 100 transports the sweat droplets 112 may be configured to both count the sweat droplets 112 and determine the time it takes for each sweat droplet 112 to pass through the sensor. This time is linearly related via the *a priori* known migration speed to the volume of the sweat droplet 112, as will be explained in more detail below with reference to Fig. 15. The issue of sweat droplets 112 deriving from two sweat glands 108 will be further discussed herein below.

Preferably, each sweat droplet 112 travels the same distance to the end of the fluid transport assembly from the outlet 114 of the chamber 102 at which they were formed. In the design shown in Fig. 12, one hundred sweat droplets 112 can be efficiently transported. Smaller and larger branched structures may also be contemplated. The branched structure may also be optimized according to the particular application of the apparatus 100, and particularly according to the density of active sweat glands 108 on the skin 106.

The above-described electrowetting tiles 124 and domains used to provide a stepwise chemical gradient may, for instance, be particularly well suited for forming the branched structure. The branched structure may also be compatible with the use of a pressure gradient. In this case the pressure may be applied upstream of the chambers 102 depicted in Fig. 12. The applied pressure may, however, be limited in order to avoid that the pressure counteracts sweat excretion.

The sensor may, in some examples, count the discrete sweat droplets 112 supplied to it via the fluid transport assembly of the apparatus 100. This may enable the sweat rate to be determined, as will be further described herein below.

Any suitable sensing principle may be employed for this purpose. It is an advantage associated with the capability of the apparatus 100 to produce a train of discrete sweat droplets 112 that a relatively simple sensor may be employed to detect each droplet 112, and thereby enable the sweat rate to be estimated.

For example, a capacitance sensing principle may be particularly useful for counting the sweat droplets 112.

Such a sensing principle may also enable estimation of the time during which the sweat droplet 112 passes through the detector, i.e. between the plates of the capacitor, since the dielectric change between air and a sweat droplet 112 (about 99% water) is relatively large (about a factor of 80). The time taken for the sweat droplet 112 to pass through the sensor may be indicative of the volume of the sweat droplet 112, as will be discussed further in relation to Fig. 15.

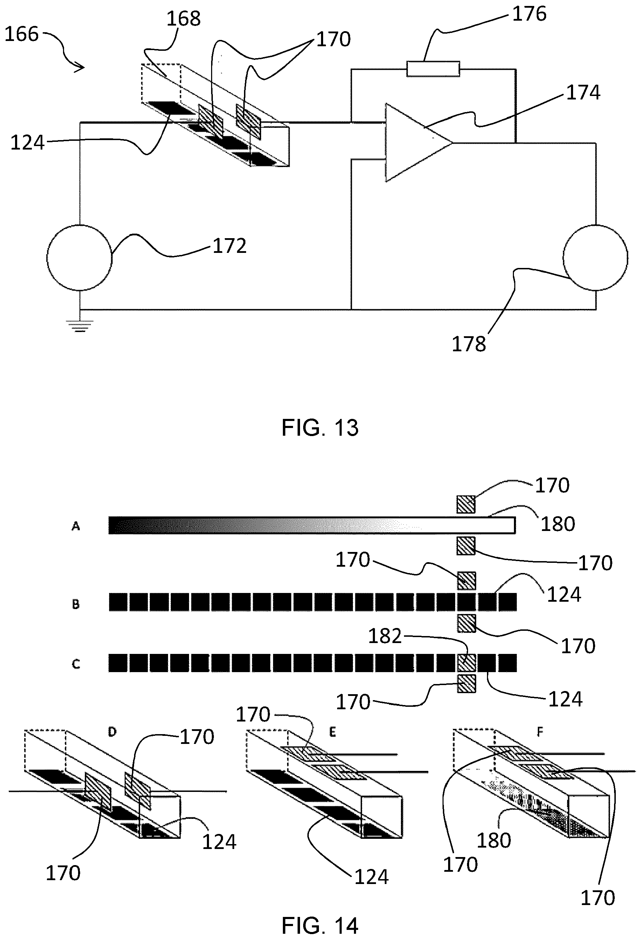

Fig. 13 shows a sensor 166 which counts the sweat droplets 112 supplied to it via the apparatus 100. As shown in Fig. 13, the sensor 166 comprises a cell 168 through which the sweat droplets 112 pass. The cell 168 may, for instance, employ one or more of the sweat droplet 112 transport principles described above in relation to the fluid transport assembly of the apparatus 100. In this respect, the cell 168 in the example shown in Fig. 13 includes electrowetting tiles 124 (being coated with a hydrophobic coating not drawn) in order to transport the sweat droplets 112 through the cell 168. In a non-limiting example, the cell 168 may be integral to the fluid transport assembly of the apparatus 100. Alternatively, the cell 168 may be a part which is connectable to a port defining a terminus of the fluid transport assembly of the apparatus 100.

The exemplary sensor shown in Fig. 13 comprises a pair of electrodes 170. One or both of the electrodes 170 may be in direct contact with the sweat droplets 112 passing through the cell 168. One or both of the electrodes 170 may alternatively be prevented, e.g. via a suitable coating, layer, etc., from being directly contacted by the passing sweat droplets 112. The electrodes 170 shown in Fig. 13 oppose each other, although alternative relative positioning of the electrodes 170 may be contemplated, as will be described with reference to Fig. 14.

When air occupies the gap between the electrodes 170, i.e. no sweat droplet 112 is present between the electrodes 170, the relative dielectric value between the electrodes 170 is about 1. When a sweat droplet 112 is passing between the electrodes 170, the relative dielectric value increases to about 80, owing to the sweat droplet 112 being around 99% water. This large difference means that the sweat droplet 112 can be easily detected, and thus the sweat droplets 112 may be straightforwardly counted using such a sensor 166.

Since the capacitance of the capacitor shown in Fig. 13 may be relatively small due to the geometry of the electrodes 170 and cell 168, it may be connected to a pre-amplifier circuit that utilizes an electrical AC signal 172 and feeds the response signal into the remaining part of the electronics for recording the passing of a sweat droplet 112 and measuring the time taken for the sweat droplet 112 to pass between the electrodes 170. By use of an operational amplifier 174 and a resistor 176, this small current may be transformed into a measurable voltage, with minimal draining of the capacitor by the subsequent reading electronics, as denoted in Fig. 13 by the voltmeter 178. To further reduce noise, the sensor plus pre-amplifier can, for example, be shielded. Numerous alternative sensor circuit designs will be immediately apparent to the skilled person.

Alternatively or additionally to the above-described capacitance sensor, the sensor 166 may comprise a conductivity sensor for counting the sweat droplets 112. In this respect, a sensor 166 comprising a conductance cell may be particularly suitable if it is also desired to measure the ion concentration in the sweat droplets 112. A conductance cell may therefore count the sweat droplets 112, measure the time taken for each of the sweat droplets 112 to pass through the cell, and enable measurement of the ion concentration.

The conductivity sensor may comprise two electrodes 170 with which the sweat droplets 112 may make direct contact. The conductivity may be measured during passing of the sweat droplet 112 between the electrodes 170. Various sensor arrangements may be contemplated for implementing such a conductivity sensor, as will be explained in more detail with reference to Fig. 14.

Any suitable electrical scheme may be employed for measuring electrical conductance. Typically, an AC signal is used to probe the conductance. An electrical voltage may be applied with a frequency, for example, in the order of 100 to 10000 Hz. This may assist to prevent electrolysis effects which may otherwise disturb the measurement. Such an electrical scheme may further comprise electronics for recording the passing of a sweat droplet 112 through the sensor 166, and measuring the time taken for the sweat droplet 112 to pass therethrough. Numerous alternative electrical schemes will be immediately apparent to the skilled person.

Changes in electrical conductivity of the sweat droplet 112 may derive from variations in concentration of dissolved salts, and in particular sodium chloride. Sodium chloride is the dominant compound that mainly determines variations in electrical conductance in sweat. The sodium chloride concentration in sweat varies between 0.06 g/100 mL and 0.76 g/100 mL, i.e. the concentration can vary by a factor of about 12. This means that such variation in sodium chloride concentration is straightforwardly measurable.

As briefly mentioned above, the sensor 166 may permit measurement of the ion concentration in the sweat droplets 112. In order for such a measurement to be useful for making a clinical interpretation, the sweat rate per gland may need to be reliably estimated. A determination of the number of sweat glands 108 supplying sweat to a respective chamber 102 may therefore need to be made. Measuring the ionic concentration may enable determination of this number of sweat glands 108, as will be further described herein below.

Fig. 14 schematically depicts several examples of the sensor 166. Examples A-C of FIG. 14 show how the sensor 166, and in particular the electrodes 170, may be arranged with respect to the fluid transport assembly of the apparatus 100.

Example A of Fig. 14 shows a fluid transport assembly in which a chemical gradient 180 transports the sweat droplets 112 to and through the electrodes 170 of the sensor 166. In this example, a sweat droplet 112, e.g. having an approximately hemispherical shape, is transported via the chemical gradient and is detected by the sensor 166 upon passing between the pair of electrodes 170. The sweat droplet 112 may make contact with the electrodes 170 or may be prevented from making contact due to, for example, an isolating coating being applied to the electrodes 170, as previously described.

Example B of Fig. 14 is the same as Example A except that the chemical gradient 180 is replaced with electrowetting tiles 124. In this example, the sweat droplets 112 are transported to and through the sensor 166 by the electrowetting wave employed by the fluid transport assembly to effect migration of the sweat droplets 112 from the chamber(s) 102 from which they derive.

Example C of Fig. 14 is the same as Example B except that an electrowetting tile 182 functions both to transport the sweat droplet 112 through the sensor 166 and, together with the electrode 170, detect the sweat droplet 112. In this case, the dual-function electrode 182 is coated with an isolating coating, e.g. a hydrophobic coating, in order to fulfil its sweat droplet 112 transportation function.

Example D of Fig. 14 shows a sensor 166 having a cell 168 in the form of a rectangular channel. Electrowetting tiles 124 are mounted in the cell 168, and the electrodes 170 of the sensor 166 are arranged perpendicularly to the electrowetting tiles 124. In this example, the dimensions of the channel may be selected such that the sweat droplet 112 touches the walls of the cell 168, thereby to form a meniscus at the head and tail of the sweat droplet 112 over the cross-section, e.g. the square, rectangular, triangular, circular, etc., cross-section, of the cell 168. In this example, the electrodes 170 may be prevented from making direct contact with the sweat droplet 112, e.g. via a suitable isolating coating, or otherwise. The resulting "beam-shaped" sweat droplet 112 passing through the cell 168 may facilitate estimation of the volume of the sweat droplet 112, as will be described in more detail with reference to Fig. 15.

Example E of Fig. 14 is the same as Example D except that the electrodes 170 of the sweat sensor 166 are mounted next to each other; the electrodes 170 being both opposite the electrowetting tiles 124.

Example F of Fig. 14 is the same as Example E except that a gradient, e.g. a chemical gradient 180 and/or a pressure gradient, is provided in the channel within the cell 168. If a pressure gradient is provided along the length of the channel, no chemical gradient or electrowetting tiles are required in order to transport sweat droplets 112 through the cell 168.

When the capacitance sensing is employed, all of the Examples A-F may be contemplated, with or without an isolator for preventing direct contact between the sweat droplets 112 and the electrodes 170 (although one of the electrodes 182 is isolated in the case of Example C, as previously described).

When conductance sensing is employed, Examples A, B, D, E and F may be contemplated, and in this case no isolator is applied to, e.g. coated on, the electrodes 170.

Numerous alternative sensor arrangements may be contemplated. For example, a sensor 166 may be provided by dividing an electrowetting tile 124 into two separate parts. One part may, for example, take the form of an outer rim coated with an isolator, and a central portion which is not connected to the outer rim. This concentric electrode structure may alternatively comprise respective uncoated electrodes for conductance measurement.

Sweat droplets 112 transported by the apparatus 100 to the sensor 166 may have different sizes/volumes. This may be due to variation in the size of the sweat droplets 112 being formed at the outlet(s) 114 of the chamber(s) 102. Such size/volume variation may also arise due to merging or coalescing of sweat droplets 112 during transport to the sensor.

By measuring the time taken for a sweat droplet 112 to pass through the sensor 166, the volume of the sweat droplet 112 may be determined. The sweat rate may then be determined from the number of sweat droplets 112 sensed, i.e. counted, by the sensor during a given time period, and the volume of the sweat droplets 112.

A sweat droplet 112 may be transported through the sensor 166 in substantially the same form or shape, e.g. hemispherical shape, as it took in the fluid transport assembly upstream of the sensor 166. Alternatively, the sweat droplet 112 may be fed into a channel 168, e.g. a cylindrical, cuboidal, or prismatic channel 168, in order to reshape the sweat droplet 112, as briefly described above in relation to Fig. 13. The hemispherical sweat droplet 112 may thus be formed into, for instance, a cylindrical shape or a beam-shape, depending on the shape of the cross-section of the channel 168. The sensor 166 may be arranged to sense the reshaped sweat droplet 112 as it passes through the channel 168.

In the case of an interfacial tension method, the gradient or electrowetting tiles 124 may be extended to more than one wall of the channel 168, and in the case of a cylindrical channel 168, may cover a substantial part of the circumference. This may assist to avoid that transport through the channel 168 is hampered by the surface area of the gradient or electrowetting tiles 124 being relatively small with respect to the surface area of the channel not covered by the gradient or electrowetting tiles 124.

The time taken for the sweat droplet 112 to pass through the sensor 166 as a function of the volume of the sweat droplet 112 may depend on the shape of the cross-section of the channel 168 which the sweat droplet 112 adopts when passing through the sensor 166. This is illustrated in Fig. 15 which provides plots of the time taken for a sweat droplet 112 to pass through a sensor 166 as a function of sweat droplet 112 volume for a beam-shaped sweat sample (dotted line 184) and a hemispherical sweat sample (solid line 186).

In the non-limiting example of Fig. 15, the velocity of the migrating sweat droplet 112 is 700 $\mu$m/s, and the sensor 166 has a length in the downstream direction through the sensor 166 of 60 $\mu$m. The time taken for the sweat droplet 112 to pass through the sensor 166 is defined as the time from when the sweat droplet 112 starts to overlap with the sensor 166, e.g. the electrodes 170 of the sensor 166, until this overlap ends.

For a hemispherical sweat droplet 112, the time taken for the sweat droplet 112 to pass through the sensor 166 is approximately equal to: (the diameter of the sweat droplet 112 plus the sensor length)/(the migration velocity of the sweat droplet 112).

For a beam-shaped sweat droplet 112 which has taken the shape of a rectangular prismatic channel 168, the time taken for the sweat droplet 112 to pass through the sensor 166 is approximately equal to: (the length of the sweat droplet 112 plus the sensor length)/(the migration velocity of the sweat droplet 112).

As shown in Fig. 15, the time taken for a hemispherical sweat droplet 112 to pass through the sensor 166 is less sensitive to the volume of the sweat droplet 112 than in the case of a cylindrical or beam-shaped sweat droplet 112. For example, a hemispherical sweat droplet 112 may take about 1.14 times longer to pass through the sensor 166 than a hemispherical sweat droplet 112 of half the volume. However, a cylindrical or beam-shaped sweat droplet 112 will take about 2 times longer to pass through the sensor 166 than a similarly shaped sweat droplet 112 of half the volume.

Accordingly, a sensor 166 comprising a channel 168 which is dimensioned such that the sweat droplet 112 forms a meniscus at the head and tail of the sweat droplet 112 spanning the cross-section of the cell 168, may improve the capability of the sensor 166 to determine variation in volume of the sweat droplets 112.

For reference, the range of volume spanned by the plots in Fig. 15 corresponds to a sweat droplet 112 diameter, in the case of hemispherical sweat droplet, of 70 to 140 $\mu$m, and corresponds to a sweat droplet 112 length, in the case of a beam-shaped droplet, of 40 to 340 $\mu$m.

When a beam-shaped sweat droplet 112 is passed through the sensor 166, and a gradient, e.g. a chemical gradient, is used for transporting the sweat droplet 112 therethrough, the migration velocity will increase with increasing sweat droplet 112 length. This increased migration velocity may counteract the above-described improved sensitivity of the time taken for the sweat droplet 112 to pass through the sensor 166 to the volume of the sweat droplet 112.

In the case of electrowetting tiles 124 being used to transport the beam-shaped sweat droplet 112 through the sensor 166, sweat droplets 112 having a length shorter than, for example, about 70 $\mu$m may be prevented from passing through the sensor 166 because the sweat droplets 112 are too short to overlap pairs of adjacent electrowetting tiles 124. On the other hand, transport of sweat droplets 112 which are longer than, for example, about 140-200 $\mu$m may be blocked due to the fact that the force as generated by one charged electrowetting tile 124 may be insufficient to move such a large sweat droplet 112.

Fig. 16 shows a sensor 166 comprising a channel 168 for shaping the sweat droplets 112, as described above. A plurality of electrowetting paths 188 are provided in the channel 168, which electrowetting paths 188 are spatially separated from each other in directions perpendicular to the direction of flow through the sensor 166, such that a sweat droplet 112 is transported through the sensor 166 by one or more of the electrowetting paths 188 depending on the volume of the sweat droplet 112. The electrowetting paths 188 each comprise a plurality of electrowetting tiles 124 for transporting sweat droplets 112 from the fluid transport assembly to and through the sensor 166.

The respective electrowetting waves provided to the electrowetting paths 188 may be substantially simultaneous with each other, such that sweat droplets 112 spanning more than one of the electrowetting paths 188 are transported by these electrowetting paths 188 synchronously.

When a relatively large sweat droplet 112 is transported via the fluid transport assembly to the sensor 166, the sweat droplet 112 may first enter the channel 168. Upon initially entering the channel 168, the sweat droplet 112 may change shape from a hemispherical shape to a beam-shape, depending on the shape of the cross-section of the entry portion of the channel 168, as previously described. In the non-limiting example shown in Fig. 16, the height of the passage of the fluid transport assembly upstream of the channel 168 may, for example, be about 150 $\mu$m, and the channel 168 of the sensor 166 may, for example, have a height of about 30 $\mu$m.

The sweat droplet 112 may then be distributed over the plurality of electrowetting paths 188, depending on the volume of the sweat droplet 112. In the non-limiting example shown in Fig. 16, four electrowetting paths 188 are provided in the channel 168. A relatively small sweat droplet 112 may remain mainly on the electrowetting tiles 124 of one of the four electrowetting paths 188. Larger sweat droplets 112 may be distributed across respective electrowetting tiles 124 of two, three or all four of the electrowetting paths 188.

Substantially simultaneous electrowetting waves may subsequently transport the sweat droplet 112 or sweat droplets 112 through the sensor 166. In the specific example shown in Fig. 16, this electrowetting wave is generated by first simultaneously charging each of the four electrowetting tiles 124 which are first encountered by the sweat droplet 112 after entering the channel 168 (the electrowetting tiles 124 on the left of Fig. 16 with an increasing width in the transport direction). Subsequently, these four electrowetting tiles 124 will be discharged, e.g. after 0.1 seconds, and immediately the four central electrowetting tiles 124 which are immediately adjacent to the initially charged and discharged electrowetting tiles 124 will be simultaneously charged, e.g. for 0.1 seconds. Immediately upon discharge of the four central electrowetting tiles 124, the next set of electrowetting tiles 124 will then be simultaneously charged, and so on.

In this manner, electrowetting waves applied simultaneously to each of the electrowetting paths 188 may cause the sweat droplet(s) 112 to be transported through the sensor 166, i.e. from left to right in Fig 16.

The sensor 166 may comprise a sensor module 190 per electrowetting path 188, in order to sense the sweat droplet 112 or sweat droplets 112 being transported, and the time taken for the sweat droplet(s) 112 to pass therethrough. Accordingly, this sensor 166 may accommodate migration of relatively small and relatively large sweat droplets 112. Due to respective sensor modules 190 being provided for each of the electrowetting paths 188, the sweat droplets 112 being transported to the sensor 166 via the apparatus 100 may be better discriminated by their size/volume, whilst enabling electrowetting to be used for transporting the sweat droplet 112 through the channel 168.

The plurality of sensor modules 190 in parallel may, for example, be regarded as providing a linear differentiation with the diameter of the sweat droplet 112. For example, if a sweat droplet 112 is split over two sensor modules 190, a sweat droplet 112 having twice the diameter will be split over four sensor modules 190, and consequently will be detected by twice the number of sensor modules 190.

In the non-limiting example shown in Fig. 16, each of the sensing modules 190 comprises a pair of electrodes 170, which electrodes 170 permit capacitance and/or conductivity-based sensing of the sweat droplets 112, as previously described. Alternative sensing principles may instead or additionally be contemplated for the sensing modules 190, such as optical and/or biomarker detection techniques.

The electrowetting arrangement 144 may be designed to assist migration of relatively small sweat droplets 112 between pairs of the electrowetting tiles 124 employed in the fluid transport assembly and/or in the sensor 166. For example, adjacent electrowetting tiles 124 may be shaped such as to interlock with each other, e.g. via the pair of electrowetting tiles 124 having respective adjacent surfaces with complementary zigzagging profiles. Such an interlocking pair of electrowetting tiles 124 may each have increased overlap with the sweat droplet 112, e.g. relative to a pair of electrowetting tiles 124 having respective adjacent flat profiles. Accordingly, the interlocking electrowetting tiles 124 may assist transportation of relatively small sweat droplets 112 to and/or through the sensor 166.

In the case of relatively large sweat droplets 112, the electrowetting tiles 124 may be broadened in directions perpendicular to the transport direction, thereby to increase the contact force between such a relatively large sweat droplet 112 and the electrowetting tiles 124. A relatively small sweat droplet 112 may still, for example, take the form of a hemispherical sweat droplet 112 when on a non-charged electrowetting tile 124, and may still overlap with an adjacent electrowetting tile 124, particularly when, for instance, interlocking electrowetting tiles 124 are employed, as previously described.

Moreover, several smaller sweat droplets 112 may be "pinched off" from a single relatively large sweat droplet 112. This may, for example, be achieved via the branched structure described above with reference to Fig. 12. In such an example, before a sweat droplet 112 reaches an interconnection a stepwise protocol may be used to divide a relatively large sweat droplet 112 into such smaller sweat droplets 112. The branched structure, which fluidly connects the chambers 102 to the sensor 166 in parallel, may assist to reduce or remove the possibility of such smaller sweat droplets 112 being interfered with by other sweat droplets 112 being transported via the branched structure.

In some examples, the sensor 166 may comprise an optical sensor for sensing the sweat droplets 112. Such an optical sensor may be an alternative, or may be included in addition, to the previously described capacitance and conductivity sensors.

The optical sensor may sense the sweat droplets 112 in any suitable manner. For example, the optical sensor may include a light source for transmitting a light beam across the path taken by the sweat droplets 112, and an opposing optical detector for sensing the light beam. The light beam may be diverted when a meniscus of a sweat droplet 112 passes thereacross. The sweat droplet 112 may be detected by the concomitant change in the transmitted light sensed by the optical detector.

Alternatively or additionally, the optical sensor may be configured to detect an absorption of light by a component in sweat. Sweat may have a particular spectroscopic fingerprint, which derives from the spectroscopic properties of each of the components of the sweat. The optical sensor may therefore, for instance, sense sweat droplets 112 with reference to such a spectroscopic fingerprint.

In other examples, the sensor 166 may comprise a biomarker sensor. The biomarker sensor may enable detection of the biomarker concentration in each sweat droplet 112, as well as permitting counting of the sweat droplets 112. The biomarker sensor may further enable the time during which the sweat droplet 112 passes through the biomarker sensor to be determined, such that a measure of the volume of the sweat droplet 112 may be derived. Accordingly, the biomarker sensor may advantageously fulfil several functions, which may simplify the system incorporating the apparatus 100 and the biomarker sensor, particularly since no additional sensor types need be included in the system.

The biomarker sensor may sense a particular biomarker, i.e. chemical biomarker, with a response which is sufficiently fast that the biomarker concentration of a sweat droplet 112 passing through the biomarker sensor may be determined. In this respect, the response time of the biomarker detector may be shorter than the time during which the sweat droplet 112 passes through the biomarker sensor.

Certain components in sweat have concentrations which are dependent on the sweat rate. Detection of such components using the biomarker sensor may enable the sweat rate per gland to be unambiguously determined.

If the response time of the biomarker sensor is limited by the diffusion of the relevant biomarker from the bulk of the sweat droplet 112 to the detection surface of the biomarker sensor, the dimensions of the channel 168 in which the biomarker sensor is disposed may be selected to be as small as possible, thereby to minimize the distance required to be traversed by the diffusing biomarker. According to the Einstein equation for diffusion, the diffusion distance is proportional to the square root of the time. Consequently, when, for example, the height of the channel 168 is reduced by a factor of two the required time for diffusion may reduce by a factor of four, thereby enabling a faster response of the biomarker sensor (when diffusion of the relevant biomarker to the biomarker sensor is the rate limiting step).

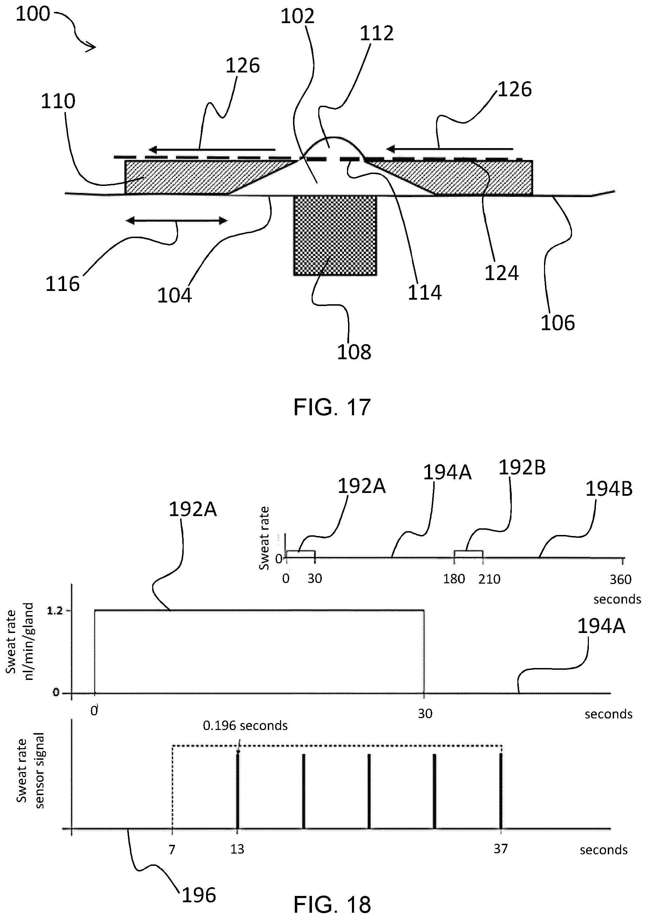

The apparatus 100 shown in Fig. 17 has a fluid transport assembly comprising electrowetting tiles 124. The size/volume of the sweat droplets 112 transported via the apparatus 100 shown in Fig. 17 may depend on the period between the electrowetting waves.

As briefly noted above, sweat glands 108 operate in a cyclic manner. The sweat glands 108 typically excrete for about 30 seconds during a burst period, followed by a rest period of about 150 seconds. The sweat burst can vary between 20 to 40 seconds or even between 10 and 50 seconds (Chen et al., "In vivo single human sweat gland activity monitoring using coherent anti-Stokes Raman scattering and two-photon excited autofluorescence microscopy", British Journal of Dermatology (2016), 174, pp803-812).

During the sweat burst phase, with a sweat rate of 1.2 nl/min/gland, a sweat droplet 112 of about 0.24 nl may be formed about every 12 seconds. Assuming that the sweat droplet 112 has a hemispherical shape, the height of the sweat droplet 112 will be about 50 $\mu$m and the diameter will be about 100 $\mu$m. When applied for the collection of sweat from sedentary subjects, the upper limit of the average sweat rate may be anticipated to be about 5 nl/min/gland. In this case, the volume of the hemispherical sweat droplet 112 will be about 6 nl and its diameter will be 285 $\mu$m.

A sweat sensing system, comprising the apparatus 100 and the sensor 166, may be configured to provide an alarm, e.g. an audio and/or visual alarm, when the sweat rate exceeds a threshold indicative of this upper limit being approached. Such a high sweat rate may in itself warrant clinical intervention. As previously noted, the sweat burst and rest periods may be about 30 seconds and about 150 seconds respectively. If the rest period at the same average sweat rate decreases, the sweat rate during the sweat burst period will decrease.

When the fluid transport assembly employs an electrowetting arrangement 144 for transporting the sweat droplets 112, several factors may be taken into account. To enable transport of the sweat droplets 112, the formed sweat droplets 112 should cover one electrowetting tile 124, and partially cover the subsequent electrowetting tile 124 in the series. Typically for hemispherical droplets of 100 $\mu$m in diameter, electrowetting tiles 124 may, for example, be used having a length (in the direction of transport) of 60 $\mu$m, with a 10 $\mu$m spacing between adjacent electrowetting tiles 124 (in the direction of transport).

The above electrowetting tile 124 dimensions are suitable for sweat droplets 112 having a diameter of 100 $\mu$m, but not for larger sweat droplets 112 which would cover more electrowetting tiles 124. In such a case, the area of the electrowetting tile 124, which is rendered transiently hydrophilic when charged, may be too small for the sweat droplet 112 to be migrated to a subsequent electrowetting tile 124 in the series.

The problems associated with relatively large sweat droplets 112 may, however, be addressed, for instance in the case of the apparatus 100 shown in Fig. 17, by adjusting the period separating consecutive electrowetting waves.

For example, instead of a 12 second time period elapsing between consecutive electrowetting waves, an electrowetting wave may be started every second. In this case, at a sweat rate of 0.2 nl/min/gland, the diameter of a hemispherical sweat droplet 112 formed in one second may be about 42 $\mu$m, which may be too small to partially overlap two adjacent electrowetting tiles 124 and, consequently, no migration of the sweat droplet 112 is initiated. However, after six subsequent electrowetting waves, the sweat droplet 112 will have grown to have a diameter of 77 $\mu$m, such that the sweat droplet 112 partially overlaps two adjacent electrowetting tiles 124, and release of the sweat droplet 112 from the outlet 114 and migration of the sweat droplet 112 may occur.

In the case of a sweat rate of 5 nl/min/gland, after one second the diameter of a hemispherical sweat droplet 112 will be 124 $\mu$m, and the sweat droplet 112 may overlap almost two adjacent electrowetting tiles 124 (two electrowetting tiles 124 and the gap therebetween may correspond to a length of 130 $\mu$m). This sweat droplet 112 diameter may thus be sufficient for migration of the sweat droplet 112. Therefore, when an electrowetting wave is initiated every second, a dynamic range from 0.2 nl/min/gland to 5 nl/min/gland may be accommodated. The number of sweat droplets 112 being counted by the sensor 166, and the time during which each sweat droplet passes through the sensor 166 may then be used to calculate the sweat rate during the sweat burst period of the sweat gland 108.

In the case of the apparatus 100 shown in Fig. 17, in which electrowetting tiles 124 are provided on the upper surface of the plate 110, different sweat rates may cause sweat droplets 112 of different sizes to be transported to the sensor 166. This may not, however, pose difficulty since the sensor 166 may both count the number of sweat droplets 112 transported thereto and determine the time taken for each sweat droplet 112 to pass therethrough. The latter is proportional to the volume of the sweat droplet 112. Hence, the sweat rate can be assessed unambiguously per sweat gland 108.

More generally, when the electrowetting arrangement 144 is employed to transport the sweat droplets 112 to and through the sensor 166, the velocity of the electrowetting wave may be determined by the frequency at which the electric field generator charges/discharges the respective electrowetting tiles 124 of the series. This switching frequency may be, for example, about 10 Hz. In the previously described case of electrowetting tiles 124 with a length of 60 $\mu$m and a gap between adjacent electrowetting tiles 124 of 10 $\mu$m, in every "step", the sweat droplet 112 may move 70 $\mu$m in 0.1 seconds. The speed at which the sweat droplet 112 is transported may therefore be 700 $\mu$m per second in this case.

Fig. 18 shows a graph depicting two sweat bursts 192A, 192B and two rest periods 194A, 194B (upper pane), an enlarged view of the first sweat burst 192A and the first rest period 194A (middle pane), and a graph of the sensor signal as a function of time (lower pane).

In the upper pane, the sweat rate is shown as a function of time. Two bursts 192A, 192B of 30 seconds and two rest periods 194A, 194B of 150 seconds are shown in this example. The middle pane shows an enlarged view which shows the first sweat burst 192A of the sweat gland 108 with a sweat rate during the burst of 1.2 nl/min/gland. In the lower pane, the sweat rate sensor signal is depicted for the first sweat burst 192A as a function of time.

A delay 196 is evident between the onset of the first sweat burst 192A and the first sweat droplet 112 being recorded by the sensor 166. This delay 196 may be ascribed to the time required for the first sweat droplet 112 of the burst to form, i.e. protrude from the outlet 114, and the time required for the fluid transport assembly to transport this sweat droplet 112 to the sensor 166.

In the example shown in Fig. 18, the time taken to form the first sweat droplet 112 of the first sweat burst 192A is 6 seconds. In other words, it takes 6 seconds for the sweat droplet 112 to grow to have a diameter which is sufficiently large to overlap the electrowetting tiles 124 delimiting the outlet 114 of the chamber 102, such that the electrowetting wave detaches the sweat droplet 112 from the outlet 114, as previously described. In this respect, Fig. 18 depicts a scenario in which the sweat rate is relatively low.

Moreover, in this non-limiting example, the distance between the chamber 102 and the sensor 166 is 5 mm. With a speed of migration of 700 $\mu$m per second, it takes about 7 seconds to transport the fully formed sweat droplet 112 to the sensor 166.

Fig. 18 shows respective sweat droplets 112 being sensed by the sensor 166 every 6 seconds during the first sweat burst 192A. Accordingly, five sweat droplets 112 are recorded by the sensor 166 for the first sweat burst 192A, which first sweat burst 192A has a duration of 30 seconds.

The sweat droplet 112 may have a hemispherical shape with a diameter of 77 $\mu$m, and the length of the sensor 166 in the direction of transport therethrough is 60 $\mu$m. With the speed of transport of the sweat droplet 112 being 700 $\mu$m per second, the time taken for the sweat droplet 112 to pass through the sensor 166 is 0.196 seconds.

In the example corresponding to Figs. 17 and 18, the ramp up and ramp down of the sweat rate at the start and end of the sweat bursts 192A, 192B respectively are fast with respect to the (1 second) period of the electrowetting wave. However, the sweat droplets 112 may vary in size during ramp up and ramp down of the sweat bursts 192A, 192B, such that the time taken by the sweat droplets 112 to pass through the sensor 166 may correspondingly vary.

In this respect, it should be noted that the velocity of 700 $\mu$m per second represents the average velocity of a sweat droplet in the case of sweat droplet 112 transportation via an electrowetting wave. However, every 0.1 seconds the subsequent electrowetting tile 124 in the series is charged by the electric field generator, and consequently the sweat droplet 112 may be regarded as moving in a stepwise manner. When the sweat droplet 112 is thus transported through the sensor 166, two features may be measured for each step: the time during which the sensor 166 output ramps up, and the time during which sensor 166 output is constant. From these measurements the average time during which the sweat droplet 112 passes through the sensor may be derived.

In an alternative non-limiting example, the electrowetting tiles 124 may instead be arranged on a lower surface of a further plate 128 positioned opposite the outlet 114. In such an example, the size/volume of the sweat droplets 112 may depend on the distance 130 between the outlet 114 and the further plate 128, and thus the size/volume of the sweat droplets 112 may be independent of the period between the electrowetting waves, as previously described. The counted number of sweat droplets 112 in a particular time period may be sufficient in this case to assess the sweat rate unambiguously, since the volume of the sweat droplet 112 is known *a priori.*

Following the sweat droplet 112 protruding from the outlet 114 to the extent that the sweat droplet 112 contacts the lower surface of the further plate 128, the electrowetting wave may transport the sweat droplet 112 along the series of electrowetting tiles 124 towards the sensor 166. As well as only the distance 130 between the outlet 114 and the further plate 128 determining the hemispherical sweat droplet 112 size/volume, the frequency with which the electrowetting waves may be applied, e.g. 0.1 per second, may be faster than the frequency of sweat droplet 112 formation. This may ensure that successive sweat droplets 112 are kept separated from each other. This example may, for instance, be suitable when the sweat rate is relatively high, such as when the system is used to monitor the sweating of athletes engaged in intensive exercise. During ramp up and ramp down of the sweat bursts 192A, 192B, the formation of a sweat droplet 112 may take more time than, for example, in the case of the example shown in Fig. 17. Hence the time taken by the sweat droplets 112 formed during such ramp up and ramp down periods to arrive at the sensor 166 may be longer than in the case of sweat droplets 112 formed at the maxima of the sweat bursts 192A, 192B.

The dynamic sweat rate measurement range may, for example, be improved by dynamically changing the frequency of the electrowetting wave according to the determined sweat rate. In other words, the electric wave generator may be configured to adjust the frequency of the electrowetting wave based on sweat rate feedback provided by the sensor 166.

In examples where a gradient, e.g. a chemical and/or a topological gradient, serves to release the sweat droplet 112 from the outlet 114, the sweat droplets 112 may all have a similar size/volume, as previously described with reference to Fig. 5.

Capability to determine the sweat rate per gland without relying on data from volunteer tests represents a key goal, since using such data neglects differences between individuals, which may be significant. To this end, a system for determining a sweat rate per gland is provided. The system comprises a sensor 166 for sensing sweat droplets 112, and an apparatus 100 for receiving sweat from one or more sweat glands 108, and transporting the sweat as discrete sweat droplets 112 to the sensor 166. The apparatus 100 may, for example, be an apparatus 100 of the type described herein above. The sensor 166 may be a sensor 166 of the type, e.g. a capacitance, conductivity, impedance, electrochemical, optical, and/or biomarker sensor, previously described.

The system comprises a processor configured to count a number of sweat droplets 112 sensed by the sensor 166 during a time period, and determine time intervals between consecutive sensed sweat droplets 112 during the time period. The processor also receives a measure of the volume of each of the counted sweat droplets 112.

The processor is further configured to identify, using the time intervals and the measure of the volume of each of the counted sweat droplets 112, active, i.e. sweat burst, periods of the one or more sweat glands 108 during which the one or more sweat glands 108 are excreting sweat, and rest periods of the one or more sweat glands 108 during which the one or more sweat glands 108 are not excreting sweat. This process of identifying the sweat burst 192A, 192B and rest periods 194A, 194B of the one or more sweat glands 108 concomitantly involves assigning the active periods 192A, 192B, and the rest periods 194A, 194B to the one or more sweat glands 108.

The processor then determines the number sweat glands 108 to which the active and rest periods are assigned, and subsequently determines the sweat rate per gland from the number of sweat droplets 112, the measure of the volume of each of the counted sweat droplets 112, and the determined number of sweat glands 108.

The system thus determines the sweat rate per gland by assigning sweat droplets 112 to particular sweat glands 108, based on the intermittent sweat excretion behavior of sweat glands 108. The system may also be physically simpler than conventional sweat sensing systems, since the apparatus 100 may transport sweat droplets 112 from several chambers 102 to a common sensor 166, as previously described (see, for example, Fig 12).

The system may also consume less energy than, for example, a sweat sensing system which monitors a continuous sweat flow. This is because such a conventional system may employ a relatively high energy consuming thermal sweat rate sensor comprising a pair of temperature probes and a heater. By contrast, the sensor 166 of the present system may simply comprise a pair of electrodes for sensing the passage of each sweat droplet 112 therebetween.

Moreover, measuring sweat rate via discretized sweat flow may enable more precise measurement of the sweat flow rate, particularly at relatively low sweat rates. By contrast, flow rate sensors in conventional systems in which the sweat is transported as a continuous flow may have comparatively greater difficulty in precisely determining the sweat flow rate, particularly when the sweat flow rate is low. A thermal sweat rate sensor of the type mentioned above may, for example, have difficulty in measuring low flow rates accurately due to heat diffusion. Other known techniques, such as sensing the cumulative change in dielectric value may also have relatively low accuracy, and may require an additional sensor, such as a sodium sensor in order to establish the sweat rate per gland.

By the fluid transport assembly fluidly connecting the respective chambers 102 to the sensor 166 in parallel, the sweat droplets 112 may be supplied to the common sensor 166 in a manner which avoids collisions between fully formed sweat droplets 112 and partially formed sweat droplets 112. Moreover, impedance by outlets 114 of downstream chambers 102 may also be avoided. The sensor 166 may, for example, comprise a pair of electrodes 170 in order to function as a capacitance, impedance and/or conductivity sensor, as previously described.

When a sweat droplet 112 passes between the two electrodes 170, i.e. through the sensor 166, an electrical property (dielectric/conductance) between the electrodes 170 changes, and sensing electronics may record this change, thereby to enable the processor to count each sweat droplet 112 passing through the sensor 166.

Moreover, the sensing electronics may further record the time taken for the sweat droplet 112 to pass through the sensor 166. The processor may, for example, calculate the volume of the sweat droplet 112 using the time taken for the sweat droplet 112 to pass through the sensor 166, and a known velocity with which the sweat droplet 112 is transported therethrough. The migration velocity may be somewhat dependent on the size/volume of the sweat droplet 112, but this can easily be determined *a priori*. The processor may apply an appropriate correction factor to account for any sweat droplet 112 size dependency of the speed of transport through the sensor 166, e.g. via a look-up table.

If two fully developed sweat droplets 112 collide and merge with each other, the time taken for the coalesced sweat droplet 112 to pass through the sensor 166 may be, for example, about two times longer than a sweat droplet 112 which has not merged with another, depending on the shape of the sweat droplet 112 within the sensor 166, as previously described in relation to Fig. 15. This means that such a larger volume sweat droplet 112 may be straightforwardly and unambiguously attributed to coalescence of two fully formed sweat droplets 112.

The apparatus 100 shown in Fig. 17 has a chamber 102 in the shape of a truncated cone. In a first non-limiting example (Example 1), the chamber 102 has a circular inlet 104 with a diameter of 360 μm, and a circular outlet 114 with a diameter of 33 μm. In a second specific non-limiting example (Example 2), the chamber 102 has a circular inlet 104 with a diameter of 1130 μm and a circular outlet 114 with a diameter of 33 μm.

Assuming an active sweat gland density on the skin 106 of one hundred active sweat glands 108 per cm$^2$, the average number of sweat glands 108 in contact with the inlet 104 is 0.1 gland and 1 gland for Example 1 and Example 2 respectively.

The probability associated with a number of glands 108 coinciding with the inlets 104 of the apparatuses 100 of the first and second examples can be calculated using a Poisson distribution. The results are shown in Table 1.

Table 1.

|      | Example 1 | Example 2 |
|------|-----------|-----------|
| P0   | 90.5%     | 36.8%     |
| P1   | 9.0%      | 36.8%     |
| P2   |           | 18.4%     |
| P3   | 0.5%      | 6.1%      |
| P>_4 |           | 1.9%      |

PX is chance of occurrence of X sweat glands 104 being in contact with the inlet 104 of the chamber 102.

As an illustrative example, the apparatus 100 is arranged to collect sweat from four collection areas on the skin 106. To this end, the apparatus 100 comprises twenty-five chambers 102 of the type shown in Fig. 17 per collection area. In this example, each inlet 104 of the chambers 102 has a diameter of 360 mm (Example 1), leading to an area of each inlet 104 of 0.1 mm$^2$.

Of the twenty-five chambers 102 (per collection area) there will be typically twenty-two or twenty-three chambers 102 which do not receive sweat from any sweat glands 108. In some exceptional cases there will be a chamber 102 which collects sweat from two or more sweat glands 102 (with a probability of around 1 in 200).

Of the twenty-five chambers 102 (per collection area) about two to three of the chambers 102 will receive sweat from one sweat gland 108. Sweat droplets 112 collected by each of these two to three chambers 102 may be sensed by the sensor 166. There remains, however, the issue of establishing the number of sweat glands 108 that contribute to the sweat droplet 112 formation.

Furthermore, the sweat rate per gland 108 may vary from 0.2 nl/min to 1 nl/min when the subject is in a sedentary state, and when the subject is engaged in intense exercise the sweat rate per gland 108 may increase to 5 nl/min or even 10 nl/min. Furthermore, the number of active sweat glands 108 may increase as a function of nerve stimulation level, which in turn is controlled by core body temperature. From an anatomical point of view, sweat glands 108 may also have different sizes, which may lead to variability in sweat rate during the sweat burst phase 192A, 192B.

The system described above addresses these issues, based on the realisation that the above-described cyclic behaviour of the sweat glands 108 may be used to determine the number of contributing sweat glands 108. This, in turn, may enable the average sweat rate per gland to be measured. Moreover, the system may permit variations in sweat rate between sweat glands 108 to be established, as will be explained herein below.

In the scenario where a chamber 102 does not receive sweat from any sweat gland 108, no sweat droplets 112 will be correspondingly transported to the sensor 166.

In the scenario where a chamber 102 receives sweat from a single sweat gland 108, that sweat gland 108 may exhibit an average sweat rate of 0.2 nl/min, with the sweat rate during a sweat burst 192A, 192B of about 1.2 nl/min (assuming a typical burst period 192A, 192B lasts about 30 seconds burst, and a rest phase 194A, 194B lasts around 150 seconds). Following filling of the chamber 102 with sweat excreted by the respective sweat gland 108, e.g. which may take about 1 to 10 minutes, a sweat droplet 112 may then protrude from the outlet 114, as schematically depicted in Fig. 17.

Sweat glands 108 which are relatively close to each other may receive nerve pulses which activate them at the same time. However, the time taken for the metabolism required for the pumping effect of the sweat gland cells to be exhausted may vary between the sweat glands 108, so partially overlapping cycles may occur.

In at least some examples, such as the one shown in Fig. 12, the distance between each of the chambers 102 and the sensor 166 may be the same. This may cause synchronous sweat bursts to be detected due to sweat glands 108 excreting into respective chambers 102 at the same time. One way of addressing this issue would be to vary this distance between respective chambers 102 and the sensor 166. However, it is unknown if the sweat glands 108 excreting into the various chambers 102 will execute a sweat burst at the same time. Moreover, non-synchronous sweat bursts of respective sweat glands 108 may coincidentally overlap, due to the distances between the respective chambers 102 and the sensor 166 being different. The identification performed by the processor may enable determination of the sweat rate per gland unambiguously, irrespective of whether there is overlap between sensor signals corresponding to different sweat glands 108. The following scenarios, which take into account a typical cycle of 30 seconds excreting followed by a 150 second rest period, are therefore considered.

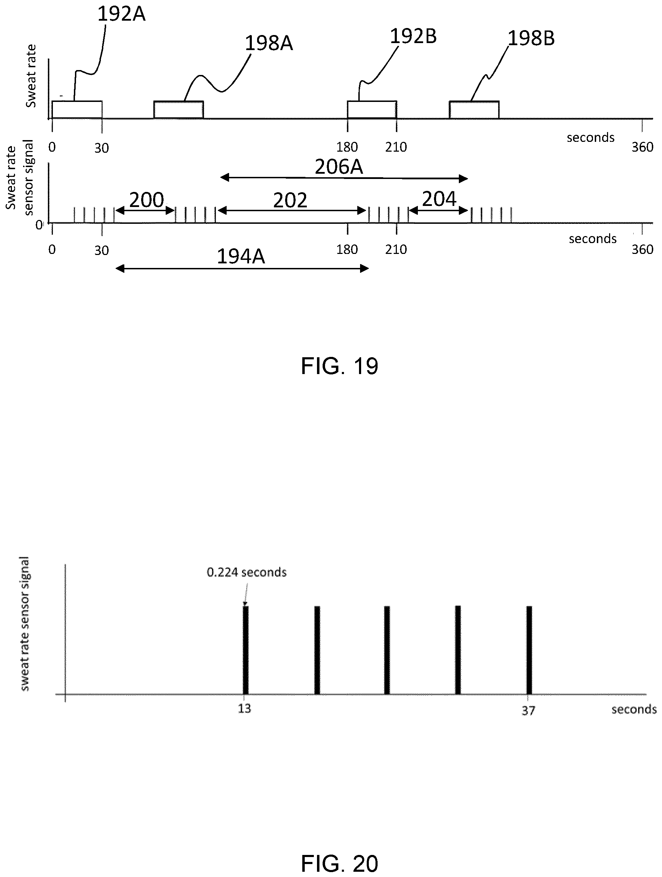

Fig. 19 shows graphs of sweat rate versus time (upper pane), and sensor signal versus time (lower pane) when respective sweat glands 108 have sweat bursts 192A, 192B, 198A, 198B at different times with respect to each other.

In the scenario shown in Fig. 19, one sweat gland 108 supplies a first chamber 102, and a different sweat gland 108 supplies a second chamber 102. The sweat droplets 112 derived from the respective glands 108 may be distinguished from each other by, for example, analysis of the sensor 166 data. In particular, the possibility of periods 200, 202, and 204 corresponding to rest periods may be excluded, since these periods 200, 202, 204 are significantly shorter than atypical rest period, which may be around 150 seconds as previously described. Since the periods denoted as 194A and 206A have durations typical of rest periods, these may be correspondingly identified as rest periods 194A, 206A of respective sweat glands 108.

Even if the first two sweat bursts 192A, 198A were to be wrongly attributed to a first sweat gland 108, and the second two sweat bursts 192B, 198B wrongly attributed to a second sweat gland 108, the average sweat rate per gland as determined by the processor would nevertheless be correct. Moreover, in the unlikely case that the sweat burst 198A of the second sweat gland 108 were to follow exactly after the sweat burst 192A of the first sweat gland 108, such that the resulting sensor 166 data were to be interpreted as a single long sweat burst of a single sweat gland 108, this erroneous assignment may be immaterial due to not altering the determined sweat rate during the sweat burst period.

The sweat rate dependence of particular biomarkers may only occur during the (active) sweat burst period of the sweat gland 108, and clearly not in the rest period. In particular, the primary sweat production and resorption leading to sweat excretion onto the skin 106 may only occur during the sweat burst period. The ratio of the primary sweat gland rate divided by the resorption rate of sweat rate dependent biomarkers may only change as a function of sweat rate. The duration of the sweat burst may not therefore influence the sweat rate. Ramping up and down will, however, influence the sweat rate, as will be discussed further herein below.

In the scenario shown in Fig. 20, sweat droplets 112 derive from two sweat glands 108 excreting sweat into respective chambers 102, but the sweat droplets 112 exactly coincide with each other. This has the effect that the time taken for each of the coalesced sweat droplets 112 to pass through the sensor 166, as shown in Fig. 20 by the width of each of the sensor signals, is increased from 0.196 seconds for a single sweat droplet 112 (see Fig. 18 above) to 0.224 seconds.

In the case of the example shown in Fig. 6 in which a series of electrowetting tiles 124 are provided on a lower surface of a further plate 128 opposing the outlet 114, all of the sweat droplets 112 form with the same size/volume, which is determined by the distance 130 between the outlet 114 and the further plate 128, as previously described. Since the sweat droplets 112 form with a size/volume which is known *a priori,* the size of the coalesced sweat droplets 112 determined via the time taken for these sweat droplets 112 to pass through the sensor clearly points to the pattern in the sensor signal being caused by two sweat glands 108.

In the alternative case of the example shown in Fig. 17, in which a series of electrowetting tiles 124 are provided on an upper surface of the plate 110, and no further plate is employed in order to detach the sweat droplet 112 from the outlet 114, the sensor signal pattern may, at first glance, point to one sweat gland 108 excreting at twice the sweat rate, rather than the actual scenario in which two sweat glands 108 are excreting at the same sweat rate. This single sweat gland 108 interpretation may, however, be ruled out, since the time between the consecutive sweat droplets 112 being detected should be correspondingly shorter, which is not the case in Fig. 20.

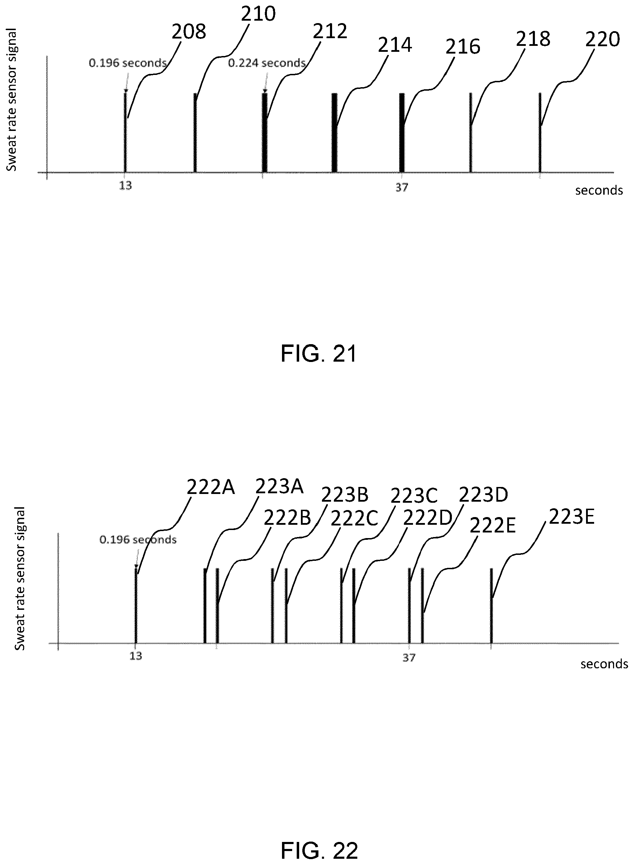

In the scenario shown in Fig. 21, sweat droplets 112 derive from two sweat glands 108 excreting sweat into respective chambers 102, but some of the sweat droplets 112 produced by the respective sweat glands 108 coincide with each other. In this case, the signals 208, 210 which correspond to the first two sweat droplets 112 sensed by the sensor 166, are assigned to a first sweat gland 108. The signals 216, 218 for the last two sweat droplets 112 are assigned to a second sweat gland 108. The remaining signals 212, 214, 215 correspond to coalescent sweat droplets 112 deriving from respective sweat droplets 112 of both sweat glands 108.

In the case of the example shown in Fig. 6, it is clear that the coalescent sweat droplets 112 must belong to respective sweat glands 108, due to the apparatus 100 transporting sweat droplets 112 having the same size/volume to the sensor 166.

However, in the case of the example shown in Fig. 17, there are possibilities to consider: (a) two sweat bursts, each resulting in five sweat droplets 112, shifted in time during sensing, and (b) one sweat burst from a first gland forming seven sweat droplets 112 and one shorter sweat burst from a second gland forming three droplets. In the latter case, one burst would have a length of 42 seconds and the second burst would have a length of 18 seconds, which is unlikely. More importantly, both possibilities (a) and (b) may lead to same average sweat rate per gland being determined, because for both cases the average sweat rate per gland during the sweat burst periods is identical.

In this case of the series of electrowetting tiles 124 being provided on the plate 110, where sweat droplets 112 can be formed of variable size, at first glance the data pattern shown in Fig. 21 could also originate from one sweat gland 108 with a relatively slow ramp up and ramp down. This may, however, be ruled out, since the time between consecutive sensor signals should also change, which is not the case in this example.

In the scenario shown in Fig. 22, sweat droplets 112 derive from two sweat glands 108 excreting sweat into respective chambers 102. Signals 222A-222E are assigned to a first sweat gland 108, while signals 223A-223E are assigned to a second sweat gland. There is some overlap between the respective sets of sensor signals but the respective sweat droplets 112 do not coalesce with each other. Such a sensor signal pattern may be ascribed to each sweat gland 108 producing a set of five sweat droplets 112, shifted in time. An alternative explanation would require a very erratic behavior of one sweat gland 108, which is physiologically unlikely, i.e. an oscillating sweat rate during a sweat burst. The latter may correspondingly be ruled out.

In the case of the example shown in Fig. 17, and an electrowetting wave being initiated every second, at a relatively low sweat rate (e.g. 0.2 nl/min/gland) a sweat droplet 112 may migrate towards the sensor 166 approximately every 6 seconds, and within one sweat burst lasting 30 seconds there will be five sweat droplets 112 formed. This may be followed by a rest period of about 150 seconds during which no sweat droplets 112 may be formed. This pattern may then be repeated. When two sweat glands 108 are active, two of these patterns will occur. Note that this discussion is confined to two chambers 102, each chamber 102 receiving sweat from a respective sweat gland 108. The case of a chamber 102 receiving sweat from more than one sweat gland 108 will also be discussed herein below.

When the respective sensor signal patterns for the two sweat glands 108 do not overlap with each other, the processor may straightforwardly identify the respective patterns, and the sweat rate per gland may be straightforwardly derived from the data pattern, e.g. as in the scenario described above with reference to Fig. 19. Sweat droplets 112 of individual sweat glands 108 may thus be distinguished by considering the cyclic behavior (sweat burst and rest periods) of the sweat glands 108. Even in the case that the sweat bursts are assigned incorrectly, there may be no effect on the determined average sweat rate per gland, as previously described.

More generally, the processor may be configured to search for the cyclic behavior of the sweat gland or sweat glands 108, and identify which sweat droplets 112 derive from which sweat gland or sweat glands 108. This may enable the processor to determine the number of sweat glands, and the sweat rate per gland.

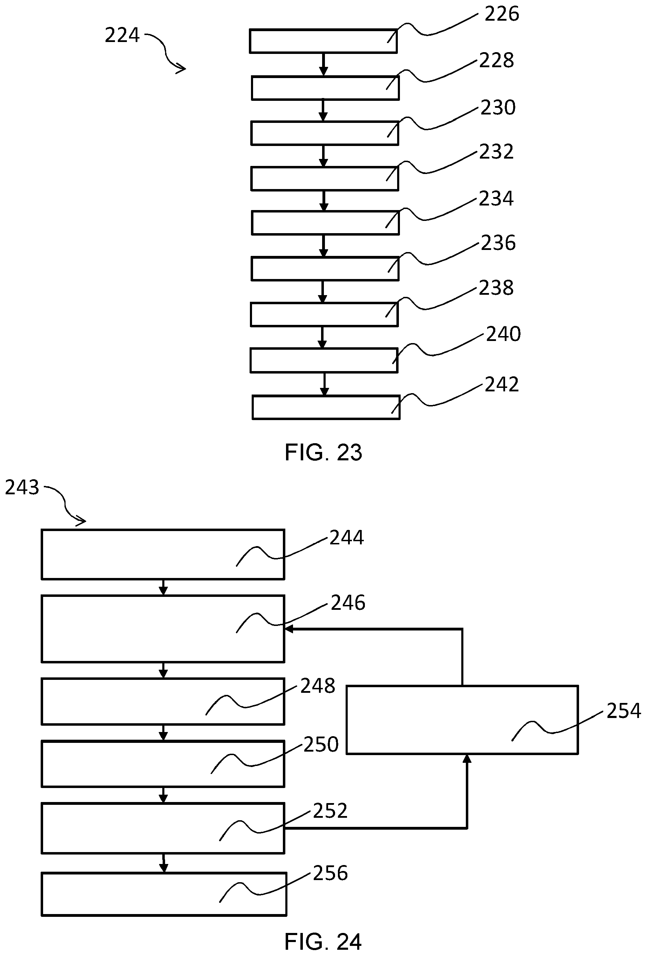

Fig. 23 shows a flowchart of a method 224 for determining a sweat rate per gland. The method 224 comprises receiving 226 sweat from one or more sweat glands, and transporting 228 the sweat as discrete sweat droplets to a sensor. Steps 226 and 228 may, for example, be implemented by the apparatus 100 described above.

In step 230, the sweat droplets are sensed using the sensor during a time period. Step 230 may, for instance, be implemented using the sensor 166 described above. The sweat droplets are counted during a time period in step 232. At 234, time intervals between consecutive sensed sweat droplets during the time period are determined. The time interval may correspond to the period between a sensor signal returning to the baseline and a subsequent increase from the baseline of a subsequent sensor signal. A measure of the volume of each of the counted sweat droplets is received at step 236, e.g. from the sensor, as previously described.

At step 238, the active, i.e. sweat burst, periods of the one or more sweat glands during which the one or more sweat glands are excreting sweat, and the rest periods of the one or more sweat glands during which the one or more sweat glands are not excreting sweat, are identified, and the active and rest periods are assigned to the one or more sweat glands. This identification and assignment uses the time intervals and the measure of the volume of each of the counted sweat droplets, as previously described with reference to Figs. 19-22.

At step 240, the number of sweat glands to which the active and rest periods are assigned is determined. The sweat rate per gland is then determined at step 242. This determination of the sweat rate per gland uses the number of sweat droplets, the measure of the volume of each of the counted sweat droplets, and the determined number of sweat glands.

Steps 232 to 242 may, for example, be implemented using the processor of the system described above.

Fig. 24 shows an example of an algorithm 243 which may be employed in order to identify the sweat burst and rest periods of the one or more sweat glands, and assign the sweat burst and rest periods to the one or more sweat glands. In other words, the algorithm 243 shown in Fig. 24 may be employed, for example by the processor of the system, to implement step 238 of the method 224.

In block 244 of the algorithm 243, the sensor signals, i.e. data pattern, over a defined time period, e.g. 10 minutes, are received from the sensor. In block 246, the received data is fitted to a template model. In particular, the fitting takes into account: the number of sensor signals, i.e. pulses, sensed in the time period, the width of each of the sensor signals, i.e. the pulse width, which may be a measure of the volume of each sensed sweat droplet, and the time intervals between consecutive sensor signals. The model fitting also takes physiologically reasonable sweat burst and rest periods into account.

In block 248, the goodness of fit of the received data to the template model is determined. In block 250, at least some of the data is identified as being suitable for basing the sweat rate determination thereon. This identification may be made on the basis of the goodness of fit of this identified data reaching or exceeding a predetermined threshold. In block 252, the fraction of the originally received data corresponding to the identified data is determined, and if this fraction is sufficiently high, the algorithm ends at 256. If, on the other hand, this fraction is below a predetermined value, e.g. 80%, then a new fitting is implemented in block 254, and blocks 246 to 252 are repeated, i.e. thereby to perform an iteration.

In a particular example, the algorithm starts with a template of (i) the number of sweat droplets in a sweat burst, (ii) the pulse time, (iii) the time during which the sweat droplet passes through the sensor, (iv) the duration time of a sweat burst period and (v) the duration time of a rest period.

In this example, each part of the data set that sufficiently resembles this template model is subtracted from the originally received data. The goodness of fit criterion may be used to control how much of the received, i.e. real, data may deviate from the model. If, by such a subtraction, a wide pulse is partially removed, the remaining pulse remains in the data set. Such a remaining pulse may, for instance, be subsequently assigned to another sweat gland.

Each part of the data set that resembles this template may again be subtracted and the process is again repeated. Further repetition of the algorithm may not be required since overlap of sweat droplets respectively originating from four glands is highly unlikely.

The size of the remaining data set is evaluated, and if, for example, this is larger than 5 to 20% of the original data set, a new iteration is started with new values for the fitting parameters. In this manner, data patterns with are not overlapping as well as overlapping data patterns may be reliably evaluated, thereby enabling the average sweat rate per gland to be determined.

In examples in which the apparatus 100, e.g. the fluid transport assembly described above, is configured to transport sweat droplets having a predetermined volume to the sensor, the fitting parameter space may be correspondingly limited. For example, when the apparatus 100 shown in Fig. 6 is employed, the volume of each of the sweat droplets 112 which are released from the outlet 114 of the chamber 102 is defined by the distance 130 between the outlet 114 and the opposing surface of the further plate 128, as previously described. By providing sweat droplets 112 having a defined, e.g. uniform, volume in this manner, the above-described identifying step 238 may be more straightforwardly implemented, i.e. than when the volume of the (non-coalesced) sweat droplets 112 is not *a priori* known.

When, for instance, the apparatus 100 shown in Fig. 17 is employed to transport sweat droplets 112 to the sensor 166, the sweat droplets 112 may have different sizes/volumes, particularly during the ramp up and ramp down phases of a sweat burst. This may necessitate a more complex implementation of step 238, for example using a more complex model where the pulse time is variable. Alternatively, a template may be used which neglects the edges, i.e. ramp up and ramp down phases, of a sweat burst in the analysis.

It is noted at this point that when more than three sweat glands 108 are supplying sweat droplets 112 to the same sensor 166, the resulting pattern analysis may become more difficult to interpret. It is for this reason that the dimensions of each inlet 104, and the number of chambers 102 per sensor 166 may be restricted (e.g. to twenty-five), such that only two to three chambers 102 are supplied by an active sweat gland, as previously described with reference to Table 1.

To increase the data volume, more than one apparatus 100 may be combined into a single wearable patch. For example, a single patch may include four apparatuses 100, with each apparatus 100 having twenty-five chambers 102. The number of apparatuses 100, and thus chambers 102, may be varied, for example, in accordance with the required precision, since sweat sampled from a greater number of sweat glands may lead to a decreasing variation in the determined average sweat rate per gland.

Fig. 20 shows the highly unlikely scenario in which the respective sweat droplets 112 originating from sweat bursts of different sweat glands 108 exactly coincide with each other, such that only one sensor signal pattern results. When sweat droplets 112 having a predetermined volume are transported by the apparatus 100 to the sensor 166, sweat droplets 112 having a larger sensed volume than the predetermined volume must be caused by coalescence of sweat droplets 112 originating from respective sweat glands 108, as previously described.

However, in the case of the apparatus shown in Fig. 17, larger sweat droplets 112 may either be caused by coalescence of sweat droplets 112 or by a higher sweat rate (recall that in this example the diameter of the hemispherical sweat droplet may vary between 77 $\mu$m and 124 $\mu$m, depending on the sweat rate per gland).

In the scenario in which respective sweat glands 108 exhibit a sweat burst simultaneously, and the resulting sweat droplets 112 are detected at the same time, the coalescent (hemispherical) sweat droplet 112 volume at the lowest average sweat rate of 0.2 nl/min/gland may have a diameter of about 97 $\mu$m. At first glance, this may be ascribed to a single sweat gland 108 excreting sweat at an average sweat rate of 2.5 nl/min/gland. However, since coalescent sweat droplets 112 at the low sweat rate (0.2 nl/min/gland) may be detected every 6 seconds, whereas a single droplet 112 at the higher sweat rate (2.5 nl/min/gland) may be detected every second, differentiation between sweat droplet 112 coalescence and relatively high sweat rates may be enabled. This information is included in the above-described algorithm.

It is known that the excretion cycles of sweat glands 108 may vary. For example, this may mean that the duration of the rest period may vary. Consequently, the algorithm may evaluate sensor signal patterns, and in particular the intervals between sensor signals, taking this variability of the rest periods into account.

It is also noted that more sweat glands 108 may become active as the sweat rate increases. The algorithm may account for such sweat gland 108 activation. In this respect, the system may, for instance, be configured on the assumption that one hundred active sweat glands are present per cm$^2$. This relatively high estimate may account for activation of further sweat glands 108 at elevated sweat rates.

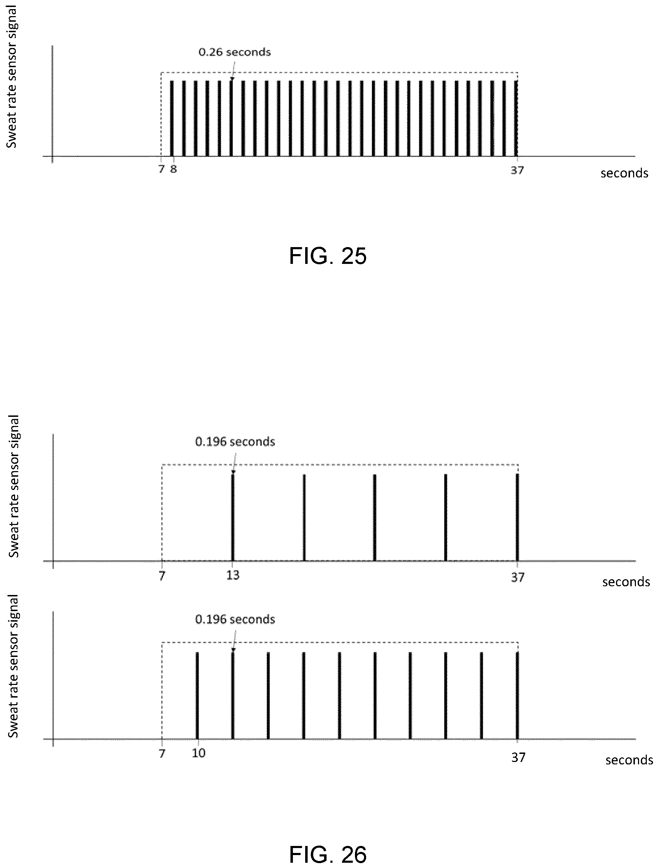

The exemplary apparatus 100 shown in Fig. 6 may provide well-defined separation of sweat droplets 112, even when the sweat rate is relatively high (e.g. 5 nl/min/gland). By an electrowetting wave being initiated every second, a sweat droplet 112 will be transported to the sensor every second, and within a sweat burst lasting 30 seconds, there will be thirty sweat droplets 112 formed. No sweat droplets 112 will be formed during the subsequent rest period, e.g. during the subsequent 150 second period. Since each incremental step of the electrowetting wave may last 0.1 seconds, the sweat droplets 112 may be clearly separated from each other, as previously described. Consequently, the same analysis method may be applied as when the sweat rate is relatively low. Fig. 25 shows a graph of the sweat rate sensor signal as a function of time when the sweat rate is relatively high.

In the example shown in Fig. 25, the average sweat rate is 5 nl/min/gland, the sweat burst period lasts 30 seconds, and the rest period lasts 150 seconds. The delay between the onset of a burst and the first sensor signal is ascribed to the time taken for a sweat droplet 112 to be formed (1 second during the sweat burst in this case), and the time taken for transport of the sweat droplet 112 to the sensor 166 (7 seconds in this case; since the migration speed is 700 $\mu$m per second, and the distance between the chamber 102 and the sensor 166 is 5 mm). As noted above, thirty sweat droplets 112 are formed during the 30 second sweat burst. In this case, the width of each sensor signal is 0.26 seconds (hemispherical sweat droplet 112 having a diameter of 124 $\mu$m; the width of the sensor 166 being 60 $\mu$m; dividing the combined length 184 $\mu$m by the migration speed of 700 $\mu$m per second).

As indicated above in relation to Example 1, the probability of two sweat glands 108 excreting sweat into the same chamber 102 may be 1 in 200. Although less likely than a chamber 102 receiving sweat from a single sweat gland 108, the less likely scenario of two sweat glands 108 excreting sweat into the same chamber 102 may still have some influence on the determination of the average sweat rate per gland. Two methods are contemplated for determining from sensor signal patterns if two sweat glands 108 (or more) are excreting into a common chamber 102.

As a first example, a system comprises four apparatuses 100, with each apparatus 100 having twenty-five chambers 102. A sweat rate sensor 166 is provided for each of the four apparatuses 100. Thus, the system has a total of one hundred chambers 102. With the area of each inlet 104 being 0.1 mm$^2$, the probability of no sweat gland 108 excreting into a chamber 102 (P0) is 90.5%, the probability of one sweat gland 108 excreting into a chamber 102 (P1) is 9%, and the probability of two or more sweat glands 108 excreting into a chamber 102 (P$\geq$2) is 0.5% (see Table 1 above). Thus, with respect to the single gland occurrence, the occurrence of two glands or more will be 1 in 18.

If a requirement is set that for the one hundred chambers 102, not more than four chambers 102 may receive sweat from two sweat glands 108 or more, using probability theory the risk of violating this requirement is about 3 in 10000. In addition, if a requirement is set that at least four chambers 102 receive sweat from a single sweat gland 108, the risk of violating this requirement is about 3 in 1000. Accordingly, such boundary requirements may assist to ensure that a sufficient number of single sweat gland 108 events are provided in order to establish a baseline sensor signal pattern, i.e. corresponding to a single sweat gland 108 excreting into a single chamber 102. With this baseline established, a sensor signal pattern resulting from two sweat glands 108 excreting into one chamber 102 can then be identified.

In principle, the requirement that four chambers 102 receive sweat from a single sweat gland 108 may be relaxed, for example to two chambers 102 receiving sweat from a single sweat gland 108. In this case, the chance of violating the requirement would be 9 in 10000.

Fig. 26 shows graphs of the sweat rate sensor signal as a function of time when the sweat droplet 112 derives from one sweat gland 108 per chamber 102 (upper pane), and when the sweat droplet 112 derives from two sweat glands 108 per chamber 102 (lower pane). It is noted that the latter scenario is distinctly different from the scenarios depicted in Figs. 19-21 in which sweat droplets 112 derive from two sweat glands 108 excreting sweat into respective chambers 102.

Regarding the scenario in the lower pane of Fig. 26, if the sweat rate of one sweat gland 108 corresponds to the sensor signal pattern in the upper pane of Fig. 26, one of the two sweat glands 108 excreting into the same chamber 102 may be located close to the sweat gland 108 excreting into the single chamber 102. This is on the assumption that sweat glands 108 which are local to one another may excrete sweat at a similar or the same rate. At first glance, the sensor signal pattern as depicted in the lower pane could be attributed to a single gland excreting sweat into a chamber 102 with a sweat rate of 0.4 nl/min/gland. However, having established the single gland baseline described above, this interpretation may be ruled out.

It should be noted that the two sweat glands 108 excreting into a common chamber 102 as depicted in the lower pane of Fig. 26 are shown as executing a sweat burst at the same time. This may be reasonable, since the respective sweat glands 108 may be relatively close to each other, and nerve pulses may reach the two sweat glands 108 at the same time and with the same intensity. When, however, the respective sweat bursts of the two sweat glands 108 are not synchronized, a sweat droplet 112 pattern may result in which, for instance, at the start and the end of the sweat burst, the sensor signals are spaced by 6 seconds, and during the sweat burst, the sensor signals are spaced by 3 seconds (see, e.g., Fig. 22). This would immediately point to two non-synchronized excreting sweat glands, and may be straightforwardly recognized.

The droplet pattern event of two sweat glands 108 excreting into a common chamber 102 may be analyzed by the algorithm shown in Fig. 24, except that in block 246 the algorithm initially fits the data pattern to a model template based on a single sweat gland 108 excreting into a chamber 102 ("lowest sweat droplet 112 count"), and then fits the data pattern to a second template model based on two sweat glands 108 excreting into a chamber 102 ("two times higher sweat droplet 112 count").

The sweat droplets 112 may be sensed, and their contact time with the sensor 166 may be determined using, for example, a capacitance and/or conductivity sensor, as previously described. The conductivity sensor, in particular, may assist in the determination of the sweat rate per gland.

This conductivity of the sweat droplets 112 may be partly determined by the concentration of ions in the sweat droplet 112. The sodium ion concentration in sweat may vary as a function of sweat rate from 0.06 to 0.76 g/100 ml. The measured conductivity of the sweat droplets 112 may be used as a proxy for the sodium ion concentration. Alternatively, a specific electrochemical sensor for sodium may be employed, providing the response speed of the sensor is sufficiently fast to sense the sodium concentration of a passing sweat droplet 112.

The following three scenarios may be considered: one sweat gland 108 excreting into a chamber 102 with a sweat rate of 5 nl/min/gland; two sweat glands 108 excreting into a chamber 102, e.g. synchronously, with each sweat gland 108 excreting at a rate of 2.5 nl/min/gland; and three sweat glands 108 excreting into a chamber 102, e.g. synchronously, with each sweat gland 108 excreting at a rate of 1.67 nl/min/gland.

A sweat rate sensor solely relying on counting the sweat droplets 112 and determining the time taken for each sweat droplet 112 to pass through the sensor 166 may not be able to distinguish between these situations because the sensor signal patterns will be the same in each of the scenarios. In order to determine the sweat rate per gland, an algorithm of the type described above may be employed or, alternatively, a sensing device for detecting a parameter relating to the concentration of an analyte whose concentration varies as a function of the sweat rate may be employed. For example, a conductivity sensor may be employed for this purpose, in which case the parameter is the conductivity, and the analyte is a sodium ion.

When a conductivity sensor is employed, the measured ionic concentration decreases stepwise from the first scenario to the second scenario to the third scenario, due to the sweat rate dependence of the ionic concentration (and sodium ion concentration). This difference in ionic concentration between the respective scenarios may be straightforwardly detected. It is not necessary to know precisely the relationship between the ionic concentration and sweat rate *a priori,* because of the above-described dominance of the scenario in which only a single sweat gland 108 excretes into a chamber 102. This dominance may be used to determine a baseline ionic concentration for a single sweat gland 108, such that the various scenarios outlined above may be distinguished from each other.

Accordingly, and more generally, the step of identifying 238 the one or more sweat glands 108 may also be based on the measured concentration of the analyte, e.g. via a conductivity measurement.

The following additional pair of scenarios may also be considered: one sweat gland 108 excreting into a chamber 102 at a sweat rate of 5 nl/min/gland; and two sweat glands 108 excreting into a chamber 102, e.g. synchronously, with each sweat gland 108 excreting at a rate of 5 nl/min/gland.

For the first of this pair of scenarios, the sweat rate sensor may sense a sweat droplet 112 every second, and in the second scenario the sweat rate sensor may sense a sweat droplet 112 every half second.

This may lead to the sensor signal pattern being interpreted as indicating that in the first scenario only one sweat gland 108 is excreting into a chamber 102, and in the second scenario there are two sweat glands 108 excreting into the same collection chamber 102. However, an alternative interpretation would be that in the first scenario only one sweat gland 108 is excreting into a chamber 102, and in the second scenario there is also one sweat gland 108 excreting into the chamber 102 but at twice the sweat rate with respect to the first scenario.

Although the second situation would seem unlikely, since local sweat glands 108 do not tend to exhibit such substantially different sweat rates from a physiological perspective, by detecting the parameter relating to the concentration of an analyte whose concentration varies as a function of the sweat rate, e.g. conductivity, an unambiguous interpretation may be attained. In this particular illustrative example, the first interpretation will lead to the ionic concentrations being measured in the respective scenarios being equal, whereas the second interpretation will lead to different ionic concentrations being measured, so only one of these interpretations may be consistent with the measured parameter.

The following further pair of scenarios may also be considered: one sweat gland 108 excreting into a chamber 102 at a sweat rate of 5 nl/min/gland; and two sweat glands 108 non-synchronously excreting into the same chamber 102, each sweat gland 108 excreting at a sweat rate of 2.5 nl/min/gland.

When the two sweat glands 108 excrete into respective chambers 102, accidental coalescence of sweat droplets 112 from the two chambers 102 would lead to an increased time taken for the coalesced sweat droplet 112 to pass through the sensor 166 (about 1.14 times longer in the case of a hemispherical sweat droplet 112; about 2 times longer in the case of a beam-shaped sweat droplet 112, as previously described), and the measured parameter, e.g. the ionic concentration, would be the same as for single sweat droplets 112. This means that this accidental coalescence of sweat droplets 112 from respective chambers 102 may be straightforwardly recognized.

The considerations are different when two sweat glands 108 are excreting into the same chamber 102 non-synchronously. In the case that the respective sensor signal patterns do not overlap with each other, the separate sweat gland 108 excretion may be straightforwardly identified, and the sweat droplets 112 may all have a similar ionic concentration.

On the other hand, when sweat bursts of the two glands 108 excreting into the same chamber 102 overlap with each other, a sensor signal pattern may result in which signals at the start and the end of the sweat burst are spaced more widely than signals during the sweat burst (see, e.g., Fig. 22). This would immediately point to two non-synchronized excreting sweat glands, and may be straightforwardly recognized. As additional evidence of the presence of two sweat glands 108 rather than one excreting into the chamber 102, the parameter measurement, e.g. the measurement of the ionic concentration, may be also considered, as previously described.

When transportation of the sweat droplets 112 is effected via electrowetting, the start of an electrowetting wave may not be synchronized with the onset of a sweat burst. This issue has significance in the case of the example shown in Fig. 17, in which the electrowetting tiles 124 are provided on the upper surface of the plate 110 delimiting the chamber 102. Sweat droplets 112 may be detected which have different sizes/volumes at the ramp up and ramp down phases of the sweat burst than during the intervening period of the sweat burst. An electrowetting wave may be initiated every second, as previously described. The sweat burst may begin at some point during this second, such that the size/volume of the first sweat droplet 112 transported to the sensor 166 may be smaller than subsequent sweat droplets 112 which have formed during the entire second between successive electrowetting waves. The latter may be regarded as "fully formed" sweat droplets 112, whereas the smaller sweat droplets 112 produced during ramp up or ramp down of the sweat burst may be regarded as being "partially formed".

It is reiterated that in the example shown in Fig. 17, at low sweat rates a sweat droplet 112 may be too small to overlap both of the electrowetting tiles 124 partially delimiting the outlet 114. As such, this sweat droplet 112 may not be transported to the sensor 166. But after six electrowetting waves, and assuming an average sweat rate of 0.2 nl/min/gland, the sweat droplet 112 may have grown sufficiently large to partially overlap these two electrowetting tiles 124.

However, if during the first electrowetting cycle only 0.5 seconds is available for sweat droplet 112 formation, the total time for sweat droplet 112 growth may be 5.5 seconds, and such a sweat droplet 112 may be correspondingly smaller than a sweat droplet 112 that formed during the full 6 seconds. In a further example in which the average sweat rate is 5 nl/min/gland, the forming sweat droplet 112 may start to overlap the electrowetting tiles 124 partly delimiting the outlet 114 after about 0.2 seconds.

Accordingly, a partially formed sweat droplet 112 which migrates to the sensor may be substantially smaller than a fully developed sweat droplet 112.

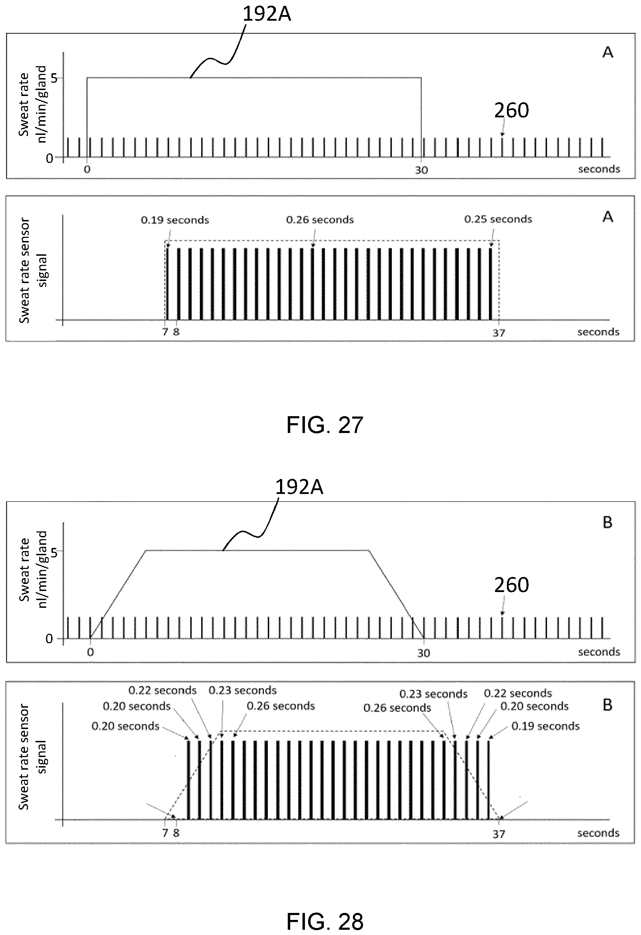

Fig. 27 shows a graph of the sweat rate as a function of time with a schematic depiction of the frequency of the electrowetting wave 260 when the latter is not synchronized with sweat droplet 112 formation (upper pane), and a graph of the associated sweat rate sensor signal as a function of time (lower pane). In the depicted example, the average sweat rate is 5 nl/min/glands, and commencement of the sweat burst 192A precedes the electrowetting wave 260 by 0.2 seconds, such that the first sweat droplet 112 had only 0.2 seconds in which to form, before being transported to the sensor 166. This is reflected in the shorter time (0.19 seconds) taken for the first sweat droplet 112 to pass through the sensor 166, as compared with the sweat droplets 112 which have formed during the entire second between consecutive electrowetting waves 260 (0.26 seconds).

The last sweat droplet 112 had 0.8 seconds to form, and this is reflected in the shorter time taken for this sweat droplet 112 to pass through the sensor 166 (0.25 seconds) than the sweat droplets 112 which formed during the entire second between electrowetting waves 260 (0.26 seconds).

Fig. 28 shows graphs analogous to those shown in Fig. 27, but with more pronounced ramps up and down at the start and end of the sweat burst respectively. Up to this point, the ramps were assumed to be fast with respect to the 1 second cycle of the electrowetting arrangement 144. However, here we consider that the ramp up and ramp down takes about 5 seconds. In this case, the sweat rate during ramp up and ramp down is less than in the middle of the sweat burst.

For similar reasons as discussed above in relation to Fig. 27, the sweat droplets 112 formed during the ramp up/down have a smaller size with respect to the sweat droplets 112 formed mid-burst. This, however, leads to a distinctive sensor signal pattern being generated, as shown in the lower pane of Fig. 28. The algorithm discussed above may recognize such a pattern, or else neglect such start up effects. The latter may involve only considering pulses having a pulse width which meets or exceeds a predetermined threshold pulse width.

It may be seen from Fig. 28 that there is a missing sensor signal at the start and end of the sweat burst, as denoted by the arrows. At these extreme start and end points of the ramp up and ramp down phases respectively, the sweat rate may be so small that the formed sweat droplet 112 does not partially overlap the two electrowetting tiles 124 partly delimiting the outlet 114. There is correspondingly no migration of the partially formed sweat droplet 112, and thus no sensor signal is recorded.

At the beginning of the sweat burst, the sweat droplet 112 may be sufficiently large to be transported only after the second pass of the electrowetting wave. In this example the first and second sensed sweat droplets 112 take the same time to pass through the sensor 166 (0.2 seconds). The subsequent sweat droplets 112 sensed by the sensor 166 during ramp up are increasingly larger, as evident from the increasing time taken for the sweat droplets 112 to pass through the sensor 166. Mid-burst, the time taken for the sweat droplets 112 to pass through the sensor 166 is constant (0.26 seconds).

At the ramp down, the sweat rate decreases, and the sweat droplet 112 size correspondingly decreases, as may be seen from the shorter times taken for the sweat droplets 112 to pass through the sensor 166.

Whilst it might be expected that the same time is taken for the first and last sweat droplets 112 to pass through the sensor 166, during the ramp up the first sweat droplet 112 is formed in 2 seconds and during ramp down the last sweat droplet 112 is formed in 1 second. Accordingly, the contact time of the first sweat droplet 112 is greater than that of the last. The partial sweat droplet 112 formed at ramp down is too small to be transported to the sensor 166, and will likely be migrated in the subsequent sweat burst. This partial sweat droplet 112 may combine with newly formed sweat received during a subsequent sweat burst, which would mean that there is no missing sensor signal at the ramp up during this subsequent sweat burst.

The above considerations may be contrasted with the case of the apparatus 100 shown in Fig. 6, in which the onset of sweat droplet 112 transportation is not determined by the frequency of application of the electrowetting waves, and consequently the sweat droplets 112 may all be of similar (predetermined) size. During the ramp up or ramp down phase, the sweat droplets 112 may emerge more slowly, and the intervals between successive sweat droplets 112 may be larger than between consecutive sweat droplets 112 produced mid-burst. If larger sweat droplets 112 are sensed, these will have resulted from coalescence of sweat droplets 112 originating from different sweat glands 108 excreting into respective chambers 102.

As a second example, a system comprises three apparatuses 100, which each have three chambers 102. A sweat rate sensor 166 is provided for each of the three apparatuses 100. Thus, the system has a total of nine chambers 102. Each of the chambers 102 has a circular inlet 104 with a diameter of 1130 $\mu$m and a circular outlet 114 with a diameter of 33 $\mu$m (see Example 2 above).

With the area of each inlet 104 being 1 mm$^2$, the probability of no sweat gland 108 excreting into a chamber 102 (P0) is 36.8%, the probability of one sweat gland 108 excreting into a chamber 102 (P1) is 36.8%, the probability of two sweat glands 108 excreting into a chamber 102 (P2) is 18.4%, the probability of three sweat glands 108 excreting into a chamber 102 (P3) is 6.1%, and the probability of four or more sweat glands 108 excreting into a chamber 102 (P≥4) is 1.9% (see Table 1 above).

In the case of a collection area served by one apparatus 100 having three chambers 102, there will be typically one chamber 102 which is not supplied with sweat by a sweat gland 108, one chamber 102 which is supplied with sweat from one sweat gland 108, and one chamber 102 which is supplied with sweat from two or more sweat glands 108.

The sensor signal patterns resulting from one or more sweat glands 108 may be suitably distinguished, such that the average sweat rate per gland may be determined, as previously described. In this case, however, there may be more incidences of two or more sweat glands 108 excreting into the same chamber 102 than in the example described above. The potential drawback is that in a very small number of cases, there may be four or five sweat glands 108 excreting sweat into the same chamber 102. This may result in a particularly complex overlapping data pattern, but with the help of a suitable criterion in the algorithm, the result can be declared invalid and the patch may be replaced accordingly.

More generally, the area of each inlet 104 may be, for example, in the range of 0.05 mm$^2$ to 2 mm$^2$, such as 0.75 mm$^2$ to 1.5 mm$^2$. This may ensure that chamber(s) 102 receive(s) sweat from sweat glands 108, but not from so many sweat glands 108 per chamber 102 that interpreting the sensor signal patterns becomes overly complex.

In the example in which there are nine chambers 102 (each having an inlet diameter of 1130 $\mu$m), the algorithm may be used as previously described, but the physical design of the system may be simpler than, for instance, the system having one hundred chambers 102.

In the example in which there are one hundred chambers 102 (each having an inlet diameter of 360 $\mu$m), the physical design of the system may be more complex, but the algorithm may be simplified by focussing on the data patterns corresponding to supply of a given chamber 102 by a single sweat gland 108. Less likely data patterns may be discarded.

From a manufacturing standpoint it may be realistic to provide each chamber 102 with its own sweat rate sensor 166, for example a capacitance or conductivity sensor due to the relatively simple design of such sensors. In this case, the algorithm may only serve the purpose of distinguishing between one or more glands 108 excreting into a particular chamber 102.

The skilled person will appreciate that more chambers 102 may be employed in order to handle variations in the sensed data. The sweat gland density of one hundred sweat glands per cm$^2$ used in the present examples should be regarded as being for the purpose of explanation only. For different average sweat gland 108 densities, the size and number of the chambers 102 can be adapted for the purpose of optimizing the results. For example, when the apparatuses 100 are to be applied to skin locations where there are relatively few active sweat glands 108, the skin surface area for sampling may be correspondingly increased in order to obtain sufficient meaningful data.

Lactate is an important biomarker because it is produced by cells if oxygen deprivation occurs. Increased levels of lactate in blood is an indication of shock. There are four shock types: hypovolemic, obstructive, cardiogenic and distributive shock. One of the causes of a distributive shock is sepsis. Shock and sepsis are serious disorders that are life threatening.

Therefore, unobtrusive measurement of lactate concentration in sweat is highly desirable. However, there are two complicating factors in correlating the concentration of lactate in sweat to the concentration of lactate in blood: (i) lactate concentration in sweat is sweat rate dependent, and (ii) lactate is secreted by the sweat gland cells themselves. Moreover, it is well known that transfer of biomarkers from blood to sweat can take up to about 10 minutes in the human body, although this is an acceptable delay from a clinical viewpoint.

The present disclosure thus far provides a solution to the first complication (i). Regarding the second complication (ii) it is further noted that the majority (90-95%) of lactate excreted in sweat onto the skin may originate from the sweat glands themselves, with the remainder (5-10%) originating from the blood. The lactate originating from sweat gland cells themselves and originating from blood should be differentiated in some manner.

Sweat gland cells are innervated with nerves, and nerve pulses activate the sweat glands. During activation, metabolism causes interstitial fluid to be pumped into the coiled tubular section of the sweat gland. The metabolism requires energy and consequently oxygen is consumed. When the nerve activity is relatively high, the produced sweat rate increases and greater quantities of oxygen are required. It is conceivable that oxygen depletion may cause the sweat glands to switch to an alternative (anaerobic) pathway, thereby producing lactate.

However, with the realization that sweat glands produce sweat in sweat bursts (lasting about 30 seconds), each sweat burst being followed by a rest period (lasting about 150 seconds), it may be reasonably assumed that sweat gland cells produce lactate in an analogous cyclic fashion with a period in the order of about 180 seconds.

Moreover, a clinically relevant increase in the lactate concentration in blood may have a significantly different time scale in the order of a few hours, e.g. 1-3 hours. The different timescales associated with sweat gland-related and blood-related changes in lactate concentration in the sweat excreted onto the skin, may be used to differentiate the former source of lactate from the latter. Accordingly, measuring the lactate concentration in sweat as a function of time may lead to suitable differentiation of sweat gland-derived and blood-derived changes in lactate concentration.

To this end, the apparatus, systems and methods described above may be usefully applied to measure lactate concentrations in sweat as a function of time. By way of a brief summary of the embodiments described above, sweat as produced by sweat glands 108 is transformed to individual sweat droplets 112 by the chambers 102 (delimited by the plate 110). Next, these sweat droplets 112 are migrated by the fluid transport assembly, e.g. using an interfacial tension method (employing a topological and/or chemical gradient or an electrowetting technique) or a pressure method, towards a sensor 166.

In this particular case, the sensor 166 may include a lactate sensor (although if the concentration of another biomarker as a function of time is of interest, a biomarker sensor specific for that particular biomarker may be included in the sensor 166). When each sweat droplet 112 contacts, e.g. traverses a detection surface of, the lactate sensor, the concentration of lactate in the sweat droplet 112 may be detected.

Providing the response of the lactate sensor is sufficiently fast, the lactate sensor may further sense the time taken for the sweat droplet 112 to traverse its detection surface. If the response time of the lactate sensor is insufficiently fast, a further sensor, e.g. a capacitance, impedance, conductivity and/or optical detector may be employed, as previously described.

In various examples detailed above, the fluid transport assembly is arranged to transport the sweat droplet at a speed of 700 $\mu$m per second. With a lactate sensor of, for example, about 60 $\mu$m in length in the transport direction of the sweat droplet 112, the time taken for each sweat droplet 112 to traverse the lactate sensor may be about 0.19 to 0.29 seconds when the sweat droplets 112 are hemispherical, and 0.15 to 0.57 seconds when the sweat droplets 112 are shaped into beam-shaped sweat droplets 112 by the channel 168 of the sensor 166, as previously described with reference to Fig. 15.

Since the response times of conventional electrochemical lactate sensors may be, at their fastest, 1 to 2 seconds, and in general may vary between 1 and 90 seconds, the following measures to decrease the speed at which the sweat droplets 112 migrate over the detection surface of the sensor 166 may be taken. It should nevertheless be noted that the speed at which the sweat droplets 112 are transported may not be reduced to the extent that coalescence of sweat droplets 112 derived from the same chamber 102 takes place.

In a first example, the sweat droplets 112 may be transported across the detection surface of the lactate sensor via a chemical gradient. The speed of migration may be lowered as the sweat droplets 112 are being transported through the lactate sensor by employing a "lower power" chemical gradient than that employed by the fluid transport assembly upstream (and in some cases downstream) of the lactate sensor. This may be achieved by providing a smaller local hydrophilic-hydrophobic change per unit of length in the direction of the migration coinciding with the lactate sensor.

In a second example, the sweat droplets 112 may be transported across the detection surface of the lactate sensor by use of an electrowetting arrangement 144. Fig. 29 shows part of an exemplary electrowetting arrangement 144. Electrowetting driven sweat droplet 112 migration may be effected by charging and discharging a series of electrowetting tiles 124, as previously described. In the example shown in Fig. 29, an electrowetting wave is created over the electrowetting tiles 124 numbered 1 to 8. The connection scheme shown in the upper pane of Fig. 29 may result in a new electrowetting wave being created every eight tiles.

The following connection scheme may thus be used in order to transport the sweat droplet 112 at a constant velocity across the series of electrowetting tiles 124 labelled 1 to 32. As shown in the upper pane of Fig. 29 (connection scheme A), tile 1 is connected to tiles 9, 17, 25; tile 2 is connected to tiles 10, 18, 26; tile 3 is connected to tiles 11, 19, 27; tile 4 is connected to tiles 12, 20, 28; tile 5 is connected to tiles 13, 21, 29; tile 6 is connected to tile 14, 22, 30; tile 7 is connected to tile 15, 23, 31; and tile 8 is connected to tile 16, 24, 32.

An electrowetting wave may be created by, for example, the electric generator implementing the following sequence: charging tile 1 (and all connected tiles); waiting 0.1 seconds; discharging tile 1 (and all connected tiles) and simultaneously charging tile 2 (and all connected tiles); waiting 0.1 seconds; discharging tile 2 (and all connected tiles) and simultaneously charging tile 3 (and all connected tiles); waiting 0.1 seconds; discharging tile 3 (and all connected tiles) and simultaneously charging tile 4 (and all connected tiles); waiting 0.1 seconds; discharging tile 4 (and all connected tiles) and simultaneously charging tile 5 (and all connected tiles); waiting 0.1 seconds; discharging tile 5 (and all connected tiles) and simultaneously charging tile 6 (and all connected tiles); waiting 0.1 seconds; discharging tile 6 (and all connected tiles) and simultaneously charging tile 7 (and all connected tiles); waiting 0.1 seconds; discharging tile 7 (and all connected tiles) and simultaneously charging tile 8 (and all connected tiles); waiting 0.1 seconds; discharging tile 8 (and all connected tiles); waiting for 1 second and repeating the cycle.

With this connection scheme, a new electrowetting wave is created every eight tiles. Moreover, the electrowetting waves all have the same velocity of one tile per 0.1 of a second. The electrowetting tiles 124 may, for example, each have a length in the transport direction of 60 $\mu$m, and each pair of adjacent electrowetting tiles 124 may be separated from each other in the transport direction by 10 $\mu$m. Accordingly, each sweat droplet 112 may travel 70 $\mu$m every 0.1 seconds, which corresponds to a sweat droplet 112 speed of 700 $\mu$m per second. Since a 1 second time period separates consecutive cycles, the frequency of occurrence of the electrowetting waves is, in this specific example, 1 Hz.

In the case of the connection scheme shown in the upper pane of Fig. 29, the respective electrowetting waves created every eight tiles effectively combine to form one electrowetting wave over the entire length of the series of electrowetting tiles 124. For the sake of clarity, the connections are drawn in two dimensions, but in practice VIAs may be used in three dimensions in order to create the connections. The latter also applies to the electrowetting arrangement 144 shown in Fig. 11.

A different connection scheme (connection scheme B) is shown in the lower pane of Fig. 29 which is designed to provide a slower sweat droplet 112 migration speed through the lactate sensor compared to upstream and downstream portions of the electrowetting arrangement 144. This is to accommodate the relatively slow response of the lactate sensor, e.g. electrochemical lactate sensor, explained above.

The connection scheme B shown in the lower pane of Fig. 29 is similar to that shown in the upper pane, but tile 1 is also connected to the tiles labelled A, tile 4 is also connected to the tiles labelled B, and tile 8 is also connected to the tiles labelled C. Tiles A, B and C are local to the lactate sensor. Note that the black dot 274 denotes an electrical connection, but an intersection 276 without a black dot means that there is no electrical connection.

Tile 1 is charged (together with the connected tiles, including tile A), there is a 0.1 second delay, and tile 1 is discharged (together with the connected tile) and tile 2 is simultaneously charged, and so on. Due to the local connection scheme, tile B is charged 0.4 seconds after tile A, and tile C is charged 0.4 seconds after tile B. Consequently, the migration velocity in the locality of the lactate sensor is decreased by a factor of four with respect to the connection scheme A shown in the upper pane of Fig. 29.

The local velocity through the lactate sensor is thus one tile per 0.4 seconds, rather than one tile per 0.1 seconds. Consequently, the local average velocity of the sweat droplets 112 through the lactate sensor in this example is 175 µm per second. The frequency at which the electrowetting waves are applied may still be 1 Hz, such that the risk of uncontrolled collision of sweat droplets 112 in the region of the lactate sensor, i.e. due to sweat droplets 112 catching up with one another, may be minimized. It should be noted that sweat droplets 112 which are less than 0.4 seconds apart will coalesce on tile A, but this may not pose difficulties since a one second resolution may be generally sufficient. In addition, if the detection surface of the lactate sensor spans the same area as tiles A-C, for instance by the detection surface of the lactate sensor being opposite the electrowetting tiles A-C, the contact time with the lactate sensor may be increased.

As noted above, when the migration speed is 700 µm per second, the time taken for each sweat droplet 112 to traverse the lactate sensor may be about 0.19 to 0.29 seconds when the sweat droplets 112 are hemispherical, and 0.15 to 0.57 seconds when the sweat droplets 112 are beam-shaped sweat droplets 112. However, when an electrowetting wave is used which transports the sweat droplets 112 four times slower (e.g. connection scheme B), the shortest time for a sweat droplet 112 to pass through the lactate sensor may be prolonged to 0.60 seconds. Moreover, in the scenario in which the detection surface spans three electrowetting tiles 124, the total contact time of a sweat droplet 112 with the sensor may be 1.80 seconds. This is longer that the shortest response times of conventional lactate sensors.

It is noted that the connection scheme may be such that a step duration in the locality of the sensor 166 is not equal to or more than one second, because this may risk that sweat droplets 112 in the locality are caught up by sweat droplets 112 transported by an electrowetting wave with a cycle time of 1 second, and thereby cause uncontrolled sweat droplet 112 collisions to occur.

Repetition of the three local tiles (A, B and C) may further prolong the time taken for the sweat droplet 112 to pass through the sensor 166. For example, four consecutive sets of these three tiles (A B C A B C A B C A B C) in the connection configuration B may increase the time taken for the sweat droplet 112 to pass through the sensor 166 to 2.40 seconds. If at the same time, the area of the detection surface is increased to span these 12 tiles, the contact time may be increased to 9.60 seconds. Note that after this local slow down through the sensor 166, the velocity may be increased again downstream of the sensor 166 by applying the first connection scheme A. Of course, with further repetition of the three local tiles (A, B and C), the contact time with the sensor 166 can be further increased. For example, with 10 repeats and ensuring that the detection surface of the sensor 166 spans these tiles, a contact time of about 60 seconds may be achieved. Whilst increasing the cycle time of the electrowetting wave from 1 to 2 seconds, might at first glance appear to provide a means for increasing the contact time with the sensor 166, this would also cause growth of the sweat droplets 112, necessitating the use of larger tiles, which may negate the increase in the contact time.

Having established that the contact time of each sweat droplet 112 with the lactate sensor may be matched to the response time of the lactate sensor, the system may be correspondingly employed to measure the lactate concentration per sweat droplet 112. Depending on the sweat rate, typically 5 to 30 sweat droplets per sweat burst of a sweat gland 108 may be transported to the lactate sensor. Thus, the lactate concentration as a function of time may be determined.

As briefly described above, on the time scale of a sweat burst, there may be a virtually constant contribution of the lactate originating from the blood, and there may be a changing contribution of the lactate produced by the sweat gland cells. For example, lactate originating from blood may remain virtually unchanged during a period of 3 minutes, whereas lactate produced by the sweat glands may change according to the 3 minute cycle time of the sweat glands.

The apparatus, systems and method of the present disclosure may enable the variation in the lactate concentration in sweat as a function of time to be closely monitored. In other words, the present disclosure may enable the dynamics of lactate production in sweat glands to be observed with a relatively high degree of detail/resolution. The dynamics of lactate production in sweat glands during a sweat burst is likely to be different from the virtually constant lactate concentration in sweat solely derived from the lactate in blood.

Consequently, by using, for example, suitable filtering techniques, the respective time scales may be determined, and the lactate concentration in sweat derived from the lactate in blood may be determined. In this way, a reliable correlation between lactate blood values and lactate values in sweat may be established. It may prove unnecessary to find an exact correlation, but increasing or decreasing trends in lactate concentration over time should correlate between blood and sweat. At the very least, the present disclosure may enable interrogation of lactate dynamics, which is a prerequisite for verifying that time-scale-based differentiation of sweat gland-derived and blood-derived changes in lactate concentration is possible.

The upper pane of Fig. 30 provides a plot of the sweat rate sensor signal as a function of time during a 30 second sweat burst. In this example there is one sweat gland excreting at an average sweat rate of 0.4 nl/min/gland. The lower left and lower right panes of Fig. 30 provide two plausible models for varying lactate concentration as a function of sweat gland metabolism. A base level lactate concentration 278 in sweat originating from blood is indicated in both the lower left and lower right panes. During a period of 30 seconds, this base level is virtually constant, but may increase when, for example, there is an impending infection.

The model shown in the lower left pane of Fig. 30 shows that, during a sweat gland burst, the sweat glands cells produce an increasingly larger lactate concentration up to a certain maximum, and subsequently this concentration decreases again.

The model shown in the lower right pane of Fig. 30 shows that at each sweat burst the lactate concentration increases, and during the subsequent rest period the lactate concentration only slowly decreases due to back diffusion into the tissue. At each subsequent sweat burst, the lactate concentration increases further, and finally after a number of sweat bursts a final maximum is reached and the sweat gland becomes inactive for a longer time, in spite of further nerve stimulation.

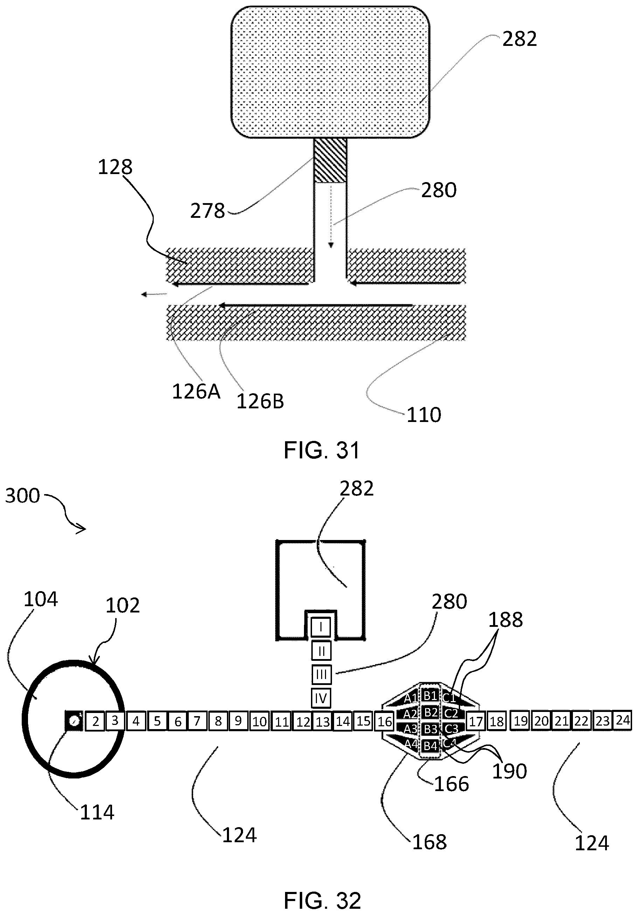

It is noteworthy that the base level lactate concentration changes only slowly over hours and this base level lactate concentration may be regarded as virtually constant within 10 sweat bursts (equivalent to a period of about 30 minutes). Hence random deviations between the sweat bursts may be attributed to a change in lactate sensor response rather than a genuine concentration change. Such an observation may thus be used to indicate when the lactate sensor should be calibrated, for example triggering an on-line calibration of the lactate sensor, as will be described further herein below with reference to Fig. 31.

More generally, the sensor 166 may comprise a biomarker sensor for determining the concentration of a biomarker present in sweat. The biomarker sensor may be supplied with sweat droplets 112 by the apparatus 100, as previously described. In this respect, the biomarker sensor may be provided either as an alternative to a capacitance, impedance, conductivity, and/or optical sensor whose purpose is to act as a sweat rate sensor, or in addition to such a sweat rate sensor. When the biomarker sensor is provided in addition to such a sweat rate sensor, the biomarker sensor may either be in series with the sweat rate sensor or in a parallel independent microfluidic circuit.

It is reiterated that the biomarker sensor may itself serve to sense each sweat droplet 112, and to measure the time taken for the sweat droplet to traverse the detection surface of the biomarker sensor. This is due to the relatively high sensitivity of biomarker sensors, since such biomarker sensors tend to be required to sense relatively low, e.g. sub-millimolar, concentrations of biomarkers, such as glucose. Accordingly, biomarker sensors may be sufficiently sensitive to be used to count sweat droplets 112, and measure the contact time of each sweat droplet 112 with the sensor 166. Accordingly, the system may be implemented in some examples with a biomarker sensor only, as previously described. Omitting an additional sweat flow rate sensor may advantageously reduce the complexity of the system, and may also conserve energy thus extending the operating lifetime of a sweat patch in which the system, or at least part of the system, is included.

The biomarker sensor should respond to changes in biomarker concentration sufficiently rapidly to measure the biomarker concentration in a continuous manner during passage of a discrete sweat droplet 112 through the biomarker sensor. Typically, electrochemical sensors as used for semi-continuous monitoring are based on an enzyme conversion step which may involve more than one hundred conversions per second per enzyme. Response times of one second have been reported (see, for example, the lactate sensing example described above). Therefore, electrochemical sensors may respond sufficiently quickly to be applied in the present system.

The biomarker sensor may, however, require frequent calibration and/or priming. There are several reasons for this, including: gradual chemical degradation of the biomarker sensor, drift relating to electronic components, variation in environmental conditions, such as higher or lower temperature and humidity, changes in atmospheric pressure, exposure to relatively high concentrations of the target analyte of interest, harsh storage and operating conditions, such as when the biomarker sensor is dropped or bumped onto a hard surface or submerged in liquid, and variation in fabrication from one sensor to another.

Off-line calibration of the biomarker sensor may have a negative workflow impact when the system is being used for monitoring a subject. Accordingly, the system may be configured to permit on-line calibration, as will now be described.

In an example, the system comprises a reservoir for storing calibration fluid for the biomarker sensor, and a dosing arrangement for supplying the calibration fluid dropwise to the biomarker sensor.

Various methods may be contemplated for effecting dropwise supply of the calibration fluid to the biomarker sensor, e.g. an electrochemical biomarker sensor. The calibration fluid contains the dissolved calibration component, required for calibrating the biomarker sensor, at a known concentration. The reservoir may, for instance, be primed prior to first use by irreversibly opening a valve, thereby fluidly connecting the reservoir to the rest of the system. Such a "breaker" is commonly used in transfusion technology as a means of irreversibly opening such sealed containers of fluids.

As well as containing the calibration component, the calibration fluid may further comprise, for example, additional components for stabilizing the resulting biomarker sensor reading. These additional components may, for instance, include proteins that are also present in sweat. Whilst in sweat such proteins may be present in varying concentrations, and thus influence the sensor measurement to varying degrees, in the calibration fluid, these proteins may be present at a constant and relatively high concentration. This may cause the additional components to saturate the absorption and interaction with the biomarker sensor, thereby creating a more stable sensor output which is substantially or solely governed by the concentration of the biomarker(s) of interest.

As shown in Fig. 31, the dosing arrangement 278 may be configured to inject a calibration fluid droplet into a conduit 280, which conduit 280 passes the calibration fluid droplet to the biomarker sensor (not visible). Chemical and/or topological, i.e. passive, gradients of the type discussed above in relation to the fluid transport assembly may, for example, be employed to transport the calibration fluid droplet to the biomarker sensor.

The dosing arrangement 278 may, for example, comprise a valve for controlling the injection of the calibration fluid droplet from the reservoir 282 into the conduit 280. The valve may control the injection of the calibration fluid droplet by switching from a closed state to an open state and back every time a calibration fluid droplet is to be supplied to the biomarker sensor. The calibration fluid droplet may then be transported via the conduit 280 to the biomarker sensor.

As shown in Fig. 31, the conduit 280 meets a passage 284 which transports sweat droplets 112 to the sensor 166. The passage 284 may, for example, be provided between the plate 110 and the further plate 128, as previously described. In this respect, the conduit may be regarded as being part of the fluid transport assembly. Similar to the sweat droplets 112, the calibration fluid droplets may be transported to the biomarker sensor down the chemical and/or topological gradients represented by the arrows 126A and 126B.

Alternatively, the calibration fluid droplet may be transported to the biomarker sensor via the electrowetting tiles 124 of an electrowetting arrangement 144. In such an example, the dosing arrangement 278 includes a valve for injecting a calibration fluid droplet from the reservoir 282 into the conduit 280. However, in an alternative example, the dosing arrangement also comprises electrowetting tiles 124, and an electrowetting wave may cause the calibration fluid droplet to migrate via the electrowetting tiles 124 from the reservoir 282 towards the biomarker sensor.

The electrowetting tiles 124 in the conduit 280 may meet the electrowetting tiles 124 of the fluid transport assembly. The electric field generator may, for example, provide an electrowetting wave to the electrowetting tiles 124 of the conduit 280 between the electrowetting waves used to transport the sweat droplets 112. In this manner, the calibration fluid droplet may arrive at the electrowetting tile 124 common to both the conduit 280 and the passage 284 of the fluid transport assembly, before being transported to the biomarker sensor by a further electrowetting wave provided along the series of electrowetting tiles 124 of the passage 284.

More generally, the system is configured to control the timing of the transportation of the calibration fluid droplet to the biomarker sensor such that the calibration fluid droplet does not coincide with a migrating sweat droplet 112. The system thereby distinguishes between the calibration fluid droplet and the sweat droplets 112 by virtue of the timing of dosing of the calibration fluid droplet with respect to the transportation of the sweat droplets 112.

In another example, the calibration fluid droplet may be transported to the biomarker sensor by a pressure gradient. The pressure gradient may be provided by storing the calibration fluid in the reservoir 282 at a pressure above atmospheric pressure, for example at about 3-4 bar. The pressure at the sensor 166 side of the valve of the dosing arrangement 278 may thus be lower than, e.g. approximately atmospheric, the pressure in the reservoir 282. Such pressurization may be achieved, for example, using pressurized air.

When the valve is opened the calibration droplet may be forced by the pressure into the passage 284 (via the conduit 280) leading to the biomarker sensor.

Fig. 32 schematically depicts a non-limiting example of a sweat sensing system 300. Sweat is collected by various chambers 102, although only a single chamber 102 is shown for clarity. In this example, a sweat enters the chamber 102 via the inlet 104, and a hemispherical sweat droplet 112 is formed and protrudes from the outlet 114. The sweat droplet 112 may, upon growing to a certain size/volume, contact the further plate 128 which opposes the outlet 114, and be detached onto the further plate 128. The further plate 128 is provided in this example with a series of electrowetting tiles 124 for transporting the sweat droplet 112 to the sensor 166, as previously described with reference to Fig. 6.

The fluid transport assembly in this case comprises the branched structure described above in relation to Fig. 12, but Fig. 32 only shows the pathway fluidly connecting the chamber 102 to the sensor 166 for the sake of clarity. It is also noted that only a limited number of electrowetting tiles 124 are shown for the electrowetting arrangement 144 employed in the system of Fig. 32, for simplicity of representation.

The fluid transport assembly of the system 300 shown in Fig. 32 includes the further plate 128 and side walls which at least partially define an enclosed passage for minimizing evaporation of the sweat droplets 112 during their migration to the sensor 166.

The distance 130 between the outlet 114 and the lower surface of the further plate 128 is typically 150 $\mu$m in this example. This distance 130 may define the size/volume of the sweat droplets 112, as previously described. Following initiation of the electrowetting wave at tile 1, the sweat droplet 112 is transported in the direction of the sensor 166.

The sensor 166 includes a channel 168 which is dimensioned such that each of the sweat droplets 112 forms a meniscus at the head and tail of the sweat droplet 112 spanning the cross-section of the channel 168, as previously described. In this respect, the height of the channel (about 30 $\mu$m) is reduced relative to the height of the passage (about 150 $\mu$m) in this example.

A plurality of electrowetting paths 188, respectively comprising electrowetting tiles labelled as A1, B1, C1; A2, B2, C2; A3, B3, C3; and A4, B4, C4 are provided in the channel 168. The sensor 166 comprises a plurality of sensor modules 190; each of the sensor modules 190 being arranged to sense sweat being transported by a respective electrowetting path or paths 188.

Each of the respective sensor modules 190 may, for example, include a sweat rate sensor and/or a biomarker sensor. To this end, the channel 168 may be provided with electrodes and/or a biomarker sensing surface, e.g. mounted on one or more surfaces of the channel 168. The sweat rate sensors enable the number of sweat droplets 112 passing through the channel 168 to be counted, as well as sensing the time taken for each sweat droplet 112 to pass through the sensor 166. The sweat rate sensor may, for example, include a capacitance sensor, an impedance sensor, a conductivity sensor, and/or an optical sensor. The biomarker sensor(s) determine the biomarker concentration per sweat droplet 112, although the biomarker sensor may also itself enable the number of sweat droplets 112 to be counted and/or the time taken for each sweat droplet 112 to pass through the sensor 166, as previously described.

Alternatively, the system 300 may include a further sensor 166 (not visible in Fig. 31) downstream of the sensor 166 shown in Fig. 31. In this respect, the sweat rate and biomarker sensors may, for instance, be provided at different locations to each other along the migratory path of the sweat droplets 112. The electrowetting tiles 124 labelled 17 to 24 may transport the sweat droplets 112 to such an additional downstream sensor and/or to a waste container.

In the area defined by tiles A1-4, B1-4 and C1-4, the migration velocity of the sweat droplets may be slowed down via the electrical connection scheme described above in relation to Fig. 28.

At regular intervals, the electrowetting tiles 124 labelled I to IV are activated, in between electrowetting waves passing along the electrowetting tiles labelled 1 to 24, thereby causing a droplet of a calibration fluid to be transported to the electrowetting tile 124 labelled 13. The calibration fluid droplet may be subsequently migrated to the sensor 166, e.g. to a biomarker sensor, via the subsequent electrowetting wave passing along the electrowetting tiles labelled 1 to 24. The known biomarker concentration in the calibration fluid droplet may then be measured, and, if required, a correction to the measured biomarker concentrations in the sweat droplets 112 may be correspondingly applied.

While the apparatus 100 shown in Fig. 12 may assist to prevent collisions of fully developed sweat droplets 112 with non-fully-developed sweat droplets 112, an alternative apparatus 100 is proposed which may increase the density of collection chambers 102 accessing the skin 106.

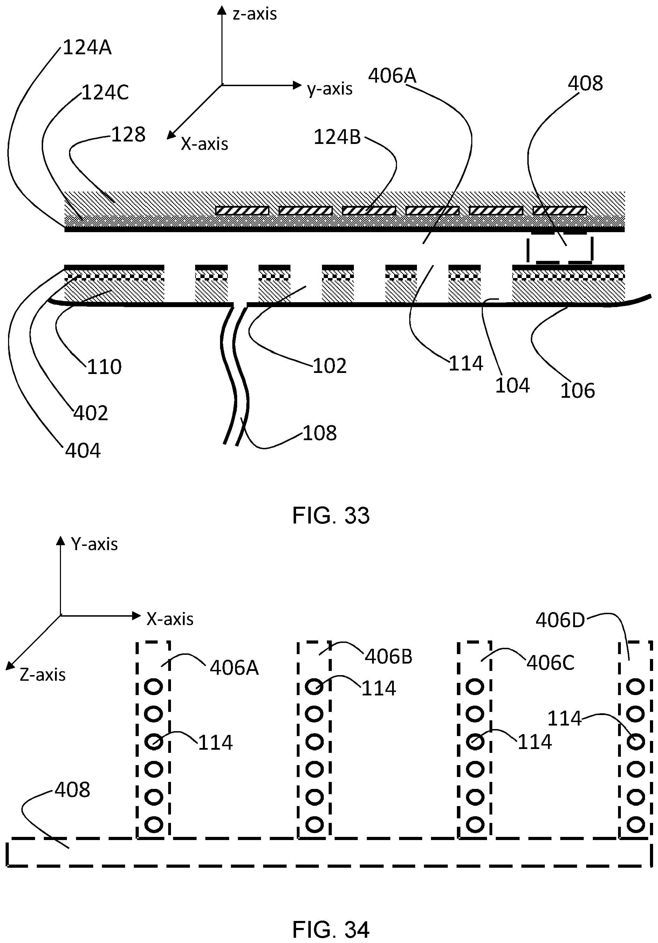

Filling a chamber 102 having, for example, a height of 25 $\mu$m may take hours for a person in a sedentary state. But providing an apparatus 100 with more chambers 102 of lower volume may assist to reduce the time required for the chambers 102 to fill. To this end, Fig. 33 shows part of an apparatus 100 having increased chamber 102 density.

The apparatus 100 comprises at least one first track; one track 406A of the at least one first track being visible in Fig. 33. Each of the at least one first track comprises a plurality of chambers 102 which receive sweat from the surface of the skin 106. Each of the at least one first track is fluidly coupled to a second track 408.

The chambers 102 in this example may be cylindrical, since this may make for increased chamber 102 density.

In the example shown in Fig. 33, the chambers 102 of the first track 406A are each delimited by the plate 110. Following filling of one or more of the chambers 102 with sweat, one or more sweat droplets 112 may protrude from one or more respective outlets 114, as previously described.

At least some, e.g. each, of the first tracks may extend perpendicularly to the second track 408. In the example shown in Fig. 33, the first tracks each extend parallel with the y-axis, and the second track 408 extends parallel to the x-axis.

In the example shown in Fig. 33, the sweat droplet 112 is transported along the first track 406A and the second track 408 in the direction of the sensor via an electrowetting assembly comprising electrodes 124B, as will be explained in greater detail herein below. Alternative designs for the fluid transport assembly may also be contemplated, e.g. using the above-described carrier fluid.

In the non-limiting example shown in Fig. 33, each first track 406A comprises six cylindrical chambers 102, although alternative chamber 102 numbers (and shapes) may be contemplated, such as two, three, four, five, seven, eight or more.

As shown in Fig. 33, each of the chambers 102 is oriented parallel with the z-axis, and is delimited by the plate 110.

The plate 110 is positioned against the skin 106 and the inlets 104 of the cylindrical chambers 102 are disposed on the skin 106 sampling site. The outlet 114 of each cylindrical chamber 102 exits onto the first track 406A, as shown.

The first 406A and second 408 tracks may each comprise two physical boundaries: (i) the plate 110 delimiting the outlets 114, and (ii) the further plate 128. The first and second tracks do not necessarily have sidewalls. Spacers may, for example, define the distance between the collection plate 110 and the further plate 128.

The apparatus 100 may comprise a conductive layer 402, for example an indium tin oxide layer, disposed beneath a hydrophobic layer 404 which is in direct contact with the first track 406A. The conductive layer 402 may serve as a ground electrode.

The further plate 128 may comprise a hydrophobic layer 124A in direct contact with the first track 406A. Adjacent this hydrophobic layer 124A is at least one dielectric layer 124C, e.g. one, two or more dielectric layer(s) 124C. The electrodes 124B of the electrowetting assembly are positioned adjacent the at least one dielectric layer 124C. Thus, the at least one dielectric layer 124C is disposed between the electrodes 124B and the hydrophobic layer 124A.

In the first track 406A, each outlet 114 is aligned with an electrode 124B of the electrowetting assembly, except the outlet 114 which is furthest away from the second track 408 that has no aligned electrode.

In the second track 408 there may be no chambers 102, and hence no outlets 114. Thus, the second track 408 may, in such an example, be solely employed for transporting sweat droplets 112 to at least one sensor (not visible in Fig. 33).

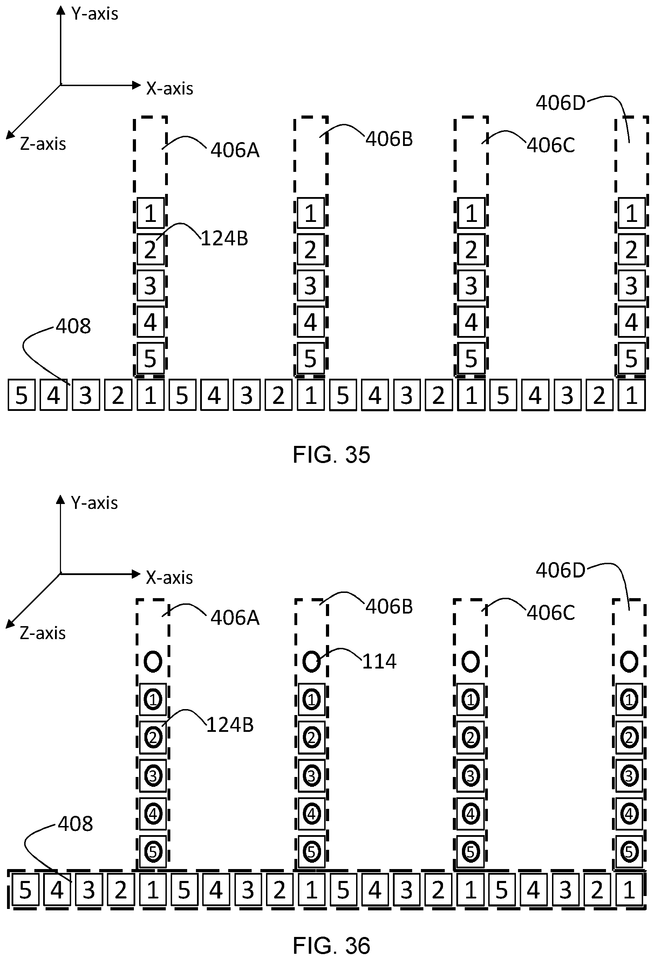

Fig. 34 shows a plan view of part of the collection plate 110. Fig. 35 shows a part of the further plate 128, in particular showing the electrodes 124B of the electrowetting assembly.

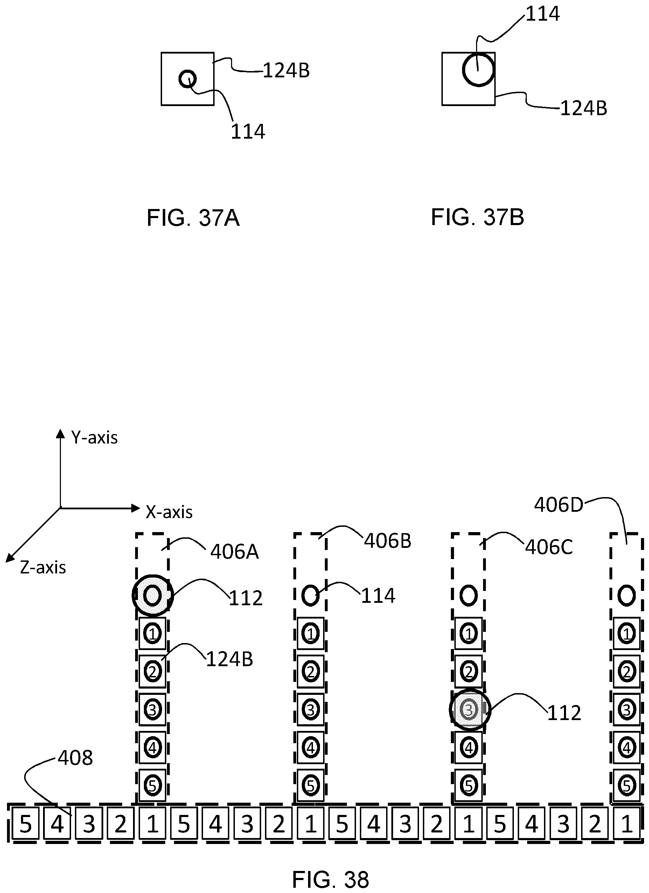

Fig. 36 shows an overlay of the plan views provided in Figs. 34 and 35. Fig. 38 shows the overlay of Fig. 36, but with two sweat droplets 112 being transported along the first tracks 406A, and 406C.

Fig 37A shows a plan view of an alternative outlet 114 of a chamber to the outlet 114 design shown in Figs. 34-36. In this example, the radius of the outlet 114 is reduced in order to minimize the length of the contact line between sweat droplet 112 and the outlet 114. The risk of pinning of fully developed sweat droplets 112 in the outlets 114 of other collection chambers 102 may accordingly be reduced.

This may be alternatively or additionally be achieved by utilizing outlets 114 that are positioned off-center with respect to the respective opposing electrode 124B, as shown in Fig. 37B.

More generally, for the apparatus 100 of this example, collision of fully developed sweat droplets 112 with non-fully-developed sweat droplets 112, which would otherwise give ill-defined sweat droplet 112 volumes, may be largely prevented.

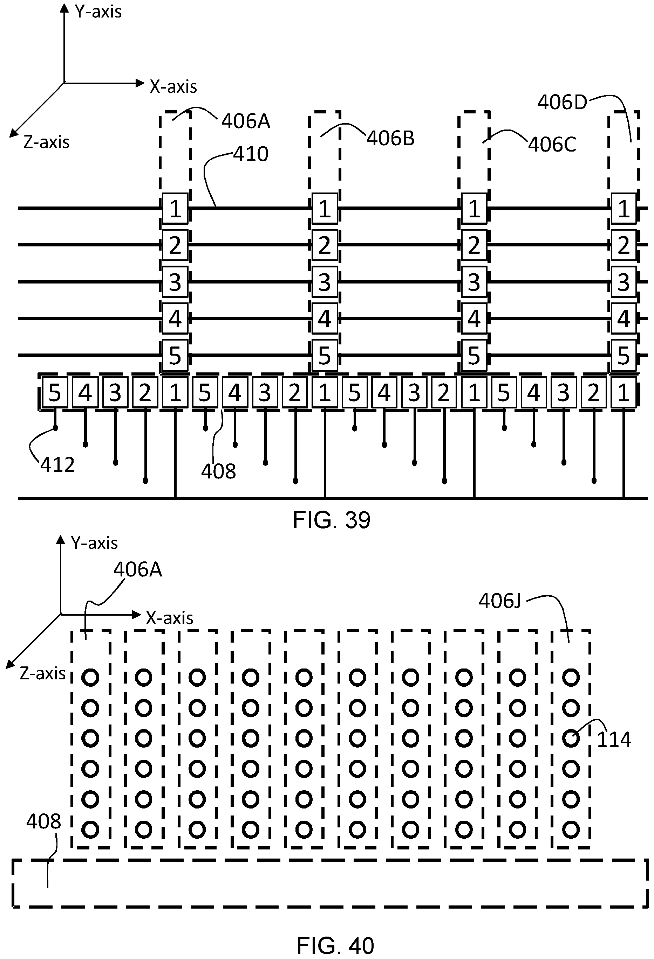

The electrodes 124B may be utilized to create an electrowetting wave by sequentially charging/discharging the electrodes 124B, as previously described. Electrodes 124B may be electrically coupled to each other, as shown in Fig. 39, and consequently only five electrical connections 410 may, in this example, be required to charge/discharge the electrodes 124B such as to move an electrowetting wave continuously over the first and second tracks 406A-D; 408. Thus, the complexity of the electrowetting assembly may be reduced, e.g. relative to an electrowetting assembly in which every electrode 124B is individually charged/discharged.

A plurality of VIAs 412, e.g. conductive through-holes, may be employed in order to provide the relatively simple assembly shown in Fig. 39, in which five electrical connections 410 are made to an interface connector. Alternatively, to reduce the number of VIAs 412, each electrode 124B may, for example, be fitted with a passive electrical bandwidth filter; the bandwidth being the same for all electrodes 124B numbered with the same number ("1", "2", "3", "4", or "5" in the example shown in Fig. 39). In the latter case, five different AC frequencies can be used over one power line to sequentially activate the electrodes 124B to create the electrowetting wave.

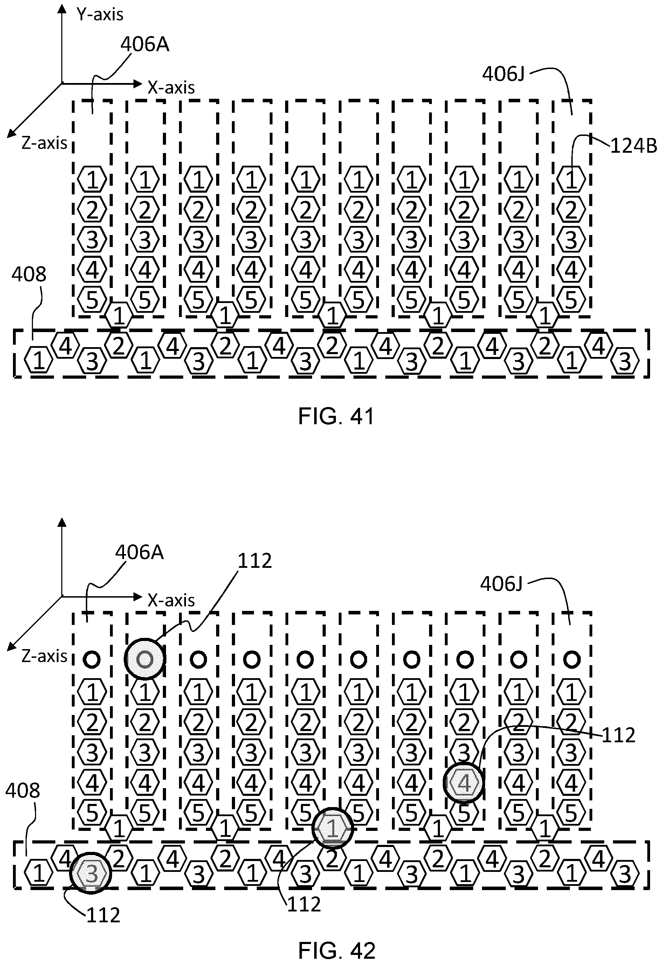

Whilst square electrodes 124B are evident in the example shown in Figs. 35, 36, 38, and 39, this is not intended to be limiting. Figs. 40 and 41 show plan views of an alternative example in which hexagonally shaped electrodes 124B are employed. In this manner, the density of chambers 102/outlets 114 may be increased.

In the example shown in Figs. 40 and 41, an electrowetting wave is induced by charging/discharging the electrodes 124B numbered 1 to 5 in the first tracks 406A-J. The second electrowetting wave in the second track 408 is induced by sequentially charging/discharging the electrodes 124B numbered 1 to 4.

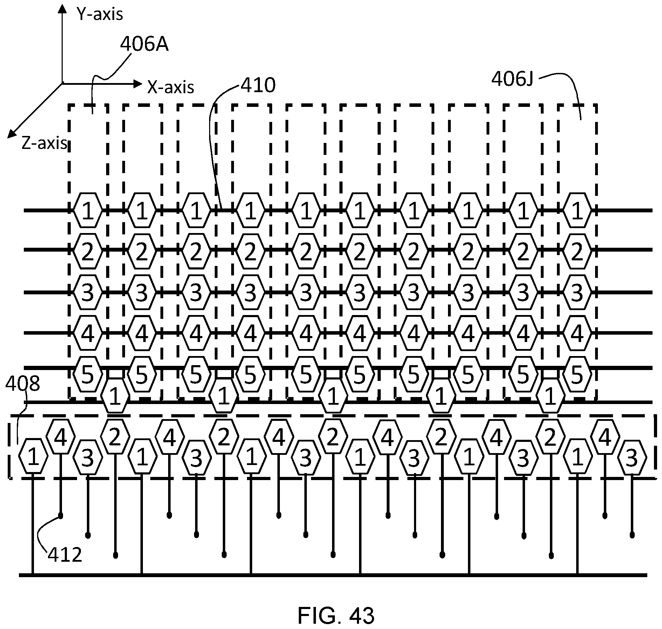

Fig. 42 shows sweat droplets 112 being transported using the apparatus 100 shown in Figs. 40 and 41. Fig. 43 shows electrical connections of the electrodes 124B shown in Figs. 41 and 42. Similarly to the example described above with reference to Fig. 39, the electrodes 124B of the example shown in Fig. 43, e.g. the majority of the electrodes 124B, may be connected using VIAs 412.

The part of the apparatus 100 shown in Fig. 43 may be termed a "collection unit". The collection unit in this example comprises ten first tracks 406A-J, with a total of sixty outlets 114. Twenty electrodes 124B, and fifteen VIAs 412 (three of the depicted electrodes 124B and two of the depicted VIAs 412 are from a subsequent collection unit) are in the first track 408.

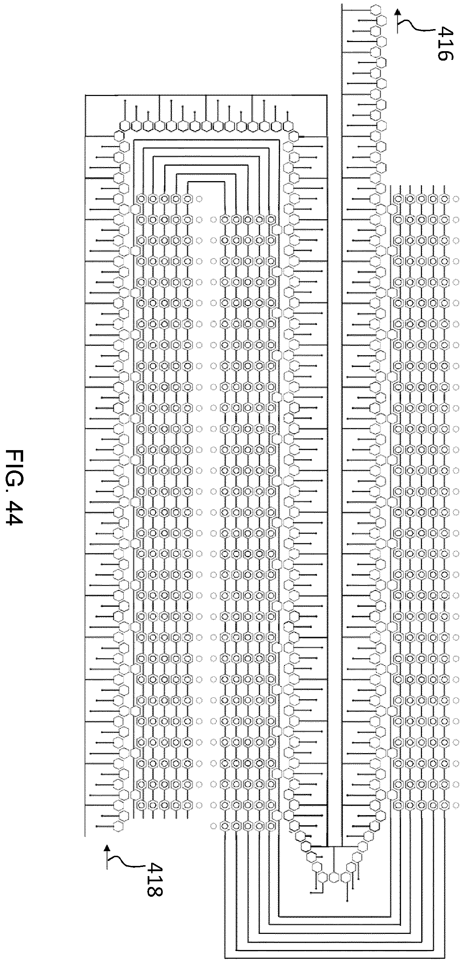

Fig. 44 depicts an exemplary apparatus 100, which may be regarded as a "snake highway and collection unit", which includes three collection units, e.g. three of the collection units shown in Fig. 43. The arrow 416 points towards the sensor(s) (not visible in Fig. 44), and the arrow 418 points to the start of the second track 408.

Various alternative "snake highway and collection unit" assemblies may be envisaged, for instance by combining five collection units in a row and four collection units in a column. The numbers and arrangement of the collection units may be freely selected.

It is noted that the tiles used for the stepwise electrowetting may be well-suited for creating the "snake highway and collection unit" making this exemplary apparatus 100 straightforward to implement in practice, e.g. using a relatively small number of VIAs 412, as previously described.

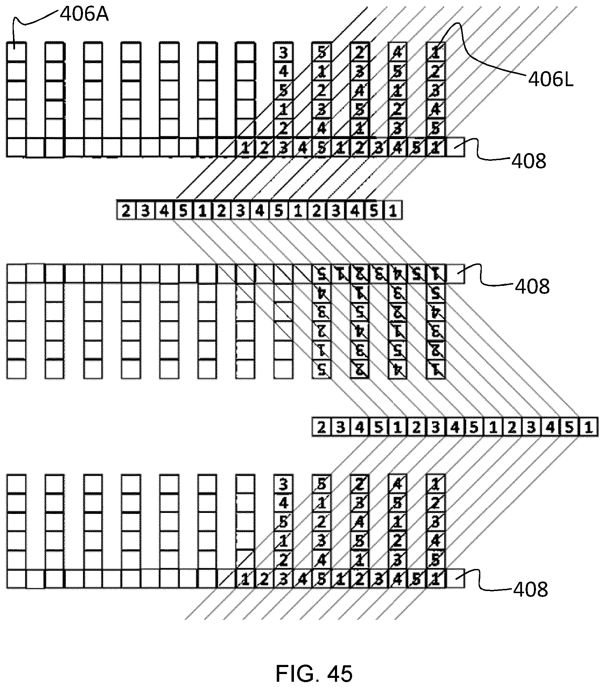

Fig. 45 shows an apparatus 100 having an alternative electrical connections design. By connecting the electrodes 124B with electrically conducting paths in diagonal fashion with respect to the tracks 406A-L, 408, the number of VIAs can be substantially reduced. For instance, connecting several units in a top to bottom zig-zag fashion (by switching the diagonal orientation 90 degrees) means that only a few VIAs may be required on the top and bottom of the apparatus 100. In this example, the first and second tracks 406, 408 can be electrically connected, whereas in the examples shown in Figs. 39, 43, and 44, the first and second tracks 406, 408 employ different electrical connections.

Noteworthy is the time it takes for a sweat droplet 112, originating from the outlet 114 that is the furthest located from the sensor, to the sensor. For this calculation, it may be assumed that the sensor is placed at the end of the snake shown in Fig. 44, amounting to about two hundred and forty electrodes 124B. With an electrowetting wave stepping from an electrode to a subsequent electrode in 0.5 seconds, the travel time may be about 2 minutes. When a stepping time of 0.1 seconds is utilized the travel time may be 24 seconds.

In a non-limiting example, each first track contains six outlets 114, and each outlet 114 may correspond to a sweat gland 108 excreting sweat into the chamber 102. So there may be a sweat gland 108 associated with a given outlet 114 or not. Assuming a cylindrical chamber 102 which is 30 $\mu$m in diameter, and assuming a sweat gland 108 exits onto the skin 106 with a diameter of 40 $\mu$m, which can be exactly aligned with the chamber 102 or can just barely touch the chamber 102, the chamber 102 may access a skin surface approximating a circle with a diameter of 110 $\mu$m. This constitutes a skin surface area of about $9.5 \times 10^3$ $\mu$m$^2$. For a first track, with six chambers 102, this amounts to a total accessible skin surface for collecting sweat of about $5.7 \times 10^4$ $\mu$m$^2$. In the case of a person, e.g. patient, in a sedentary state there may be about 7 to 10 active glands per cm$^2$ of skin 106. This may be regarded as the minimum number of active sweat glands 108 to be monitored to get a sufficient reliable average sweat rate per gland. Hence, the minimum skin surface to be assessed for sweat monitoring may be set to 1 cm$^2$. However, a person in a high active state or a patient trying to cool its body can have up to typically one hundred active glands per cm$^2$. The more active glands 108, the more droplet collisions between fully developed and non-fully-developed droplets can occur. Therefore, for determining the number of collisions between a fully developed sweat droplet 112 and a non-fully-developed sweat droplet 112, the one hundred active glands per cm$^2$ is used for further explanation.

As such, 1754 first tracks may be required to access 1 cm$^2$ of skin surface for sweat collection. The number of active glands may vary between body locations and can even vary from person to person. These numbers will be used herein below for further explanation, however a person skilled in the art can understand that other numbers can be employed as well, e.g. depending on the body location, and thus the apparatus 100 and methods may be adapted accordingly.

Considering the one hundred active glands per square cm$^2$ and the accessible skin surface for collecting sweat by the six chambers 102 per first track, $5.7 \times 10^4$ $\mu$m$^2$, on average there may be 0.057 active glands present in the denoted accessible skin surface area. With the Poisson distribution equation:

$$PX = [\ <x>x\ /\ x!\ ] * \exp(-<x>)$$

where PX is the probability that x active sweat glands are present in the skin surface area accessible for the first track, <x> is the average number of active sweat glands in the particular skin surface area, and the x! factor is the factorial value of x, the following probabilities can be determined:

$$P0 = 0.9446$$

$$P1 = 0.0538$$

$$P2 = 0.0015$$

These are the chances of the occurrence of respectively no active sweat gland, one active sweat gland and two active glands ejecting sweat onto the first track. These numbers are rounded, but eight decimals after the decimal point are used in subsequent calculations. There may be two or more active sweat glands contributing to sweat droplet formation in the first track, and there may be a substantial chance that a fully developed sweat droplet will hit a non-fully-developed sweat droplet. For further calculation, it may be assumed as a worst case that the probability of occurrence of two or more active sweat glands is equal to that of collision between a fully developed sweat droplet and a non-fully-developed sweat droplet.

To calculate the factor between the chance of non-colliding droplets on the first track and the chance of colliding fully developed sweat droplets with a non-fully-developed sweat droplet the following chances are evaluated:

$$P>1 = 1 - (P0+P1) \qquad = 0.00156$$

$$P>2 = 1 - (P0+P1+P2) \quad = 0.00003$$

These are the chances of the occurrence of respectively more than one sweat gland and more than two active sweat glands per first track. Again, these numbers are rounded, but eight decimals after the decimal point are used in subsequent calculations.

The ratio between the chance of occurrence of one active sweat gland and the chance of occurrence of more than one active sweat gland is:

$$P1/(1-(P0+P1) = 34.4$$

Thus, in about 1 out of 34 measurements the sweat droplet may have an undefined size ranging from 1 to 2 fully developed sweat droplets. This is equivalent with 2.9% of the measurements giving an undefined droplet size. With the above-described subtraction algorithm, a residue of 2.9% may be acceptable. In addition, a larger sweat droplet (a merged fully developed sweat droplet plus a non-fully-developed sweat droplet) may still be identified as one fully developed sweat droplet plus some unidentified sweat droplet, hence reducing the already acceptable residue.

In a first consideration, a given sweat droplet having the size of a fully developed droplet may be attributed to: two non-fully-developed sweat droplets merged with a size of a fully developed sweat droplet; or three non-fully-developed sweat droplets merged with a size of a fully developed sweat droplet. This, however, may be impossible because non-fully-developed droplets may not be transported by the (electrowetting assembly of the) first track.

In a further consideration, a given sweat droplet with the size of two fully developed sweat droplets may be attributed to: two merged fully developed sweat droplets; or a fully developed sweat droplet merged with two droplets with a total size of a fully developed sweat droplet. The latter configuration requires three active sweat glands ejecting sweat onto one first track. The ratio between the chance of occurrence of one active sweat gland and the chance of occurrence of more than two active sweat glands is:

$$P1/(1-(P0+P1+P2) = 1820$$

This may constitute an even smaller residue.

Note that there may be some variation in size between fully developed sweat droplets because a droplet can reach the fully developed status just before a passing electrowetting wave or just after the passing electrowetting wave. Since the sweat rate sensor may not only count the number of sweat droplets but also the passing time (with a relationship with the sweat droplet size) still the precise sweat volume per sweat droplet may be determinable. From the above analysis, 1 out of 35 measurements the sweat droplet size may be wrongly identified. Nevertheless, the majority of the signals may express the correct size of the sweat droplets and using the above-described algorithm, an acceptable residue can be reached.

It is further noted that a fully developed sweat droplet can collide with another fully developed sweat droplet on the second track. Fortunately, that may present little difficulty. For instance, when two fully developed sweat droplets collide, a coalescent droplet forms that has a two times larger volume, which may be easily recognized by the sweat rate sensor, as previously described. This may be accomplished, for instance, by looking at the pulse width, which may be *a priori* well-defined for a sweat droplet which is twice the size. Indeed, since most sweat droplets may be single/non-merged sweat droplets, the baseline size of such a sweat droplet may be established.

Calculation of the chamber surface area

A typical surface area may comprise about 0.1 to 1 active glands. As such may assist the apparatus to be used for determination of the sweat rate per sweat gland, as previously described.

There are between 50 to 600 glands per $cm^2$ skin area, depending on the body location.

In sedentary state about 10% of these sweat glands constitute active sweat glands. For a person engaged in intense exercise and/or exposed to relatively high temperature conditions, the active sweat glands may be close to 100%. Consequently, the number of active glands vary in the order of 5 to 600 glands per $cm^2$ skin area. This is equal to 0.05 to 6 active glands per $mm^2$ skin area.

The following provides a calculation of the desired surface area.

Table 2. Skin surface area

| Active glands per $mm^2$ | 0.05 | 0.1 | 6 |
|---|---|---|---|
|  |  |  |  |

(continued)

| Number of glands per surface area | | | |
|---|---|---|---|
| 0.1 | 2 mm$^2$ | 1 mm$^2$ | 0.0167 mm$^2$ |
| 1 | 20 mm$^2$ | 10 mm$^2$ | 0.167 mm$^2$ |

These surface areas may in some examples be attributed to one chamber.

The filling time of a chamber may, for example, additionally have a maximum in the order of a minute but not above 30 to 60 minutes. In the latter case, the time between two determinations may be so high that clinical relevance can be disputed depending on the application. Preferably, to cover all applications, the filling time of the chamber may be in the order of seconds to minutes.

The sweat rate in sedentary state is in the order of 0.2 nl/min for one gland, which equals $3.3 \times 10^3$ $\mu m^3$ per second. For a person doing exercise this can reach 5 nl/min for one gland. In exceptional cases, the sweat rate may be even higher, such as 5 nl/min, which equals $8.25 \times 10^4$ $\mu m^3$ per second.

In order to calculate the filling time of a chamber, we introduce an exemplary chamber height of 10 $\mu m$. The filling time equals the chamber volume divided by sweat rate.

Table 3. Chamber filling time

| Sweat rate | Chamber height in $\mu m$ | Chamber surface area in $\mu m^2$ | Volume chamber in $\mu m^3$ | Filling time |
|---|---|---|---|---|
| 3.3 *10$^3$ $\mu m^3$ per second | 10 | 0.0167 x 10$^6$ | 0.0167 x 10$^7$ | 50.6 seconds |
| | | 0.167 x 10$^6$ | 0.167 x 10$^7$ | 8.4 minutes |
| | | 1 x 10$^6$ | 1 x 10$^7$ | 50.5 minutes |
| | | 2 x 10$^6$ | 2 x 10$^7$ | 1.7 hours |
| | | 10 x 10$^6$ | 10 x 10$^7$ | 8.4 hours |
| | | 20 x 10$^6$ | 20 x 10$^7$ | 16.8 hours |
| | | | | |
| 8.25 * 10$^4$ $\mu m^3$ per second | 10 | 0.0167 x 10$^6$ | 0.0167 x 10$^7$ | 2.0 seconds |
| | | 0.167 x 10$^6$ | 0.167 x 10$^7$ | 20.2 seconds |
| | | 1 x 10$^6$ | 1 x 10$^7$ | 2.0 minutes |
| | | 2 x 10$^6$ | 2 x 10$^7$ | 4.1 minutes |
| | | 10 x 10$^6$ | 10 x 10$^7$ | 20.2 minutes |
| | | 20 x 10$^6$ | 20 x 10$^7$ | 40.3 minutes |

Clearly, the chamber filling time for persons in sedentary state may not fulfil the maximum filling time requirement for all sweat gland surface area densities. Therefore, the chamber volume should be smaller, in the table below a range is determined.

Table 4. Filling time of small chambers

| Sweat rate | Chamber height in $\mu m$ | Chamber diameter in $\mu m$ | Chamber surface area in $\mu m^2$ | Volume chamber in $\mu m^3$ | Filling time |
|---|---|---|---|---|---|
| 3.3 *10$^3$ $\mu m^3$ per second | 10 | 100 | 7.852 x 10$^3$ | 7.852 x 10$^4$ | 23.8 seconds |
| | 20 | 100 | 7.852 x 10$^3$ | 1.570 x 10$^5$ | 47.6 seconds |
| | | | | | |

(continued)

| Sweat rate | Chamber height in $\mu$m | Chamber diameter in $\mu$m | Chamber surface area in $\mu$m$^2$ | Volume chamber in $\mu$m$^3$ | Filling time |
|---|---|---|---|---|---|
| $8.25 * 10^4 \mu$m$^3$ per second | 10 | 100 | $7.852 \times 10^3$ | $7.852 \times 10^4$ | 0.81 seconds |
| | 20 | 100 | $7.852 \times 10^3$ | $1.570 \times 10^5$ | 1.90 seconds |

In this case, the maximum filling time is in the order of one minute maximum.

For analysis reasons, at least 5 to 10 active glands may be measured, because there can be some variation in sweat rate per gland.

For example, taking into account (i) an active gland surface area density of 0.1 active glands per mm$^2$ and (ii) in average 10 active glands to be measured one requires a skin surface area to be sampled of 100 mm$^2$ (1 cm$^2$). In the table below, the calculations are shown for the various sweat gland densities.

Table 5. Skin surface area to be sampled

| Active glands per mm$^2$ | 0.05 | 0.1 | 6 |
|---|---|---|---|
| | | | |
| Affiliated total skin area for in average 5 active glands | 1 cm$^2$ | 0.5 cm$^2$ | 0.00835 cm$^2$ |
| Affiliated total skin area for in average 10 active glands | 2 cm$^2$ | 1 cm$^2$ | 0.0167 cm$^2$ |

For a patient in a sedentary state, with low number of active glands, a skin area of at least 1 cm$^2$ may be required.

A suitable surface area of a relatively small chamber may be in the order 0.0078 mm$^2$, constituting a diameter of 100 $\mu$m. A suitable range in diameter is between 50 and 200 $\mu$m.

The number of such small chambers may be in the order of 12800 (100 mm$^2$/0.0078 mm$^2$). A suitable range is between 3200 and 51200.

In the design of the apparatus, one can have individual small chambers all entering the electrowetting pathway, e.g. as in the examples shown in Figs. 33 to 45.

Alternatively, in the design of the apparatus, one can have a multitude of individual small chambers, and the sweat droplets converge towards a single path, e.g. as in the example shown in Fig. 12. For instance, 100 small chambers all exit via one pathway. As such conceptually, one may regard the 100 small chambers as a single collection vessel.

Finally, in another alternative, the chambers may have surface areas in the range of 0.0167 to 20 mm$^2$, e.g. each constituting one chamber in the design shown in Fig. 12. In this case only the two smallest chambers, see Table 3, may fulfil the criterion of max filling time, leading to a surface area between 0.0167 and 0.167 mm$^2$.

The chambers with a surface area between 1 and 10 mm$^2$ may fulfil this criterion for persons doing exercise only at least 5 nl/min/gland. The largest chamber of 20 mm$^2$ may be outside the filling criterion, however may be suitable for persons sweating > 5 nl/min/gland.

The apparatus, systems and methods of the present disclosure may be applied for non-invasive, semi-continuous and prolonged monitoring of biomarkers that indicate health and well-being, for example for monitoring dehydration, stress, sleep, children's health and in perioperative monitoring. As well as being applicable for subject monitoring in general, the present apparatus, systems and methods may be specifically applied to provide an early warning for sudden deterioration of patients in the General Ward and Intensive Care Unit, or for investigation of sleep disorders. Currently, measurements may only be made in a spot-check fashion when a patient is visiting a doctor, although it is noted that the present disclosure may also be usefully applied in performing such spot-check measurements.

Other variations to the disclosed embodiments can be understood and effected by those skilled in the art in practicing the claimed invention, which is defined by the appended claims. In the claims, the word "comprising" does not exclude other elements or steps, and the indefinite article "a" or "an" does not exclude a plurality. Measures recited in mutually different dependent claims can advantageously be combined. Any reference signs in the claims should not be construed as limiting the scope.

**Claims**

1. A system (300) comprising:

   a sensor (166) for sensing sweat droplets;
   an apparatus (100) for receiving sweat from one or more sweat glands and transporting said sweat as discrete sweat droplets to the sensor; and
   a processor configured to:

   record the sweat droplets sensed by the sensor during a time period;
   determine time intervals between consecutive sensed sweat droplets during the time period; **characterized in that** the processor is further configured to
   identify, using the time intervals, at least one active period of each of the one or more sweat glands during which the respective sweat gland is excreting sweat, and at least one rest period of each of the one or more sweat glands during which the respective sweat gland is not excreting sweat, the active and rest periods being assigned to said one or more sweat glands.

2. The system (300) according to claim 1, wherein the processor is further configured to determine the number of sweat glands to which the active and rest periods are assigned.

3. The system (300) according to claim 2, wherein the processor is further configured to:

   receive a measure of the volume of each of the recorded sweat droplets; and
   determine the sweat rate per gland from the number of recorded sweat droplets, the measure of the volume of each of the recorded sweat droplets, and the determined number of sweat glands; optionally wherein the processor is configured to identify said at least one active period and said at least one rest period based on said measure of the volume of each of the recorded sweat droplets and said time intervals.

4. The system (300) according to claim 3, wherein the sensor (166) is configured to sense an indicator of the volume of the sweat droplets, and the processor is configured to receive said sensed indicator.

5. The system (300) according to claim 3 or claim 4, wherein the processor is configured to fit data received from the sensor to a first template model, thereby to identify the active and rest periods of each of the one or more sweat glands, said data comprising at least the time intervals, and the measure of the volume of each of the recorded sweat droplets.

6. The system (300) according to claim 5, wherein said fitting to the first template model additionally uses: a number of sweat droplets in the at least one active period, a duration of the at least one active period, and/or a duration of the at least one rest period.

7. The system (300) according to claim 5 or claim 6, wherein the processor is configured to assess a goodness of fit of said data to the first template model, and optionally, based on the goodness of fit, fit at least a portion of the data to a further first template model.

8. The system (300) according to any one of claims 5 to 7, wherein the processor is configured to, following fitting said data to the first template model, fit at least a portion of the data to a second template model, wherein the first template model is based on at least some of said sweat droplets deriving from a sweat sample constituted by sweat excreted from a single sweat gland, and the second template model is based on at least some of said sweat droplets deriving from a further sweat sample constituted by sweat excreted from two or more sweat glands.

9. The system (300) according to any one of the preceding claims, wherein the apparatus (100) is arranged to transport sweat droplets having a predetermined volume to the sensor (166).

10. The system (300) according to any one of the preceding claims, wherein the sensor (166) comprises a sensing device for detecting a parameter relating to the concentration of an analyte whose concentration varies as a function of sweat rate, wherein the processor is configured to use said parameter in assigning the active and rest periods to the one or more sweat glands.

**11.** The system (300) according to claim 10, wherein the sensing device is a conductivity sensor and the parameter is conductivity.

**12.** The system (300) according to any one of the preceding claims, wherein the sensor (166) comprises a biomarker sensor; optionally wherein the processor is configured to receive a plurality of biomarker concentrations from the biomarker sensor during said at least one active period of a respective sweat gland and determine a variation of the biomarker concentration in time within said at least one active period.

**13.** The system (300) according to any one of the preceding claims, wherein the apparatus (100) comprises a plurality of chambers (102), each of the chambers having an inlet (104) for receiving sweat from the skin, and an outlet (114) arranged such that a sweat droplet forms and protrudes therefrom following filling of the chamber with sweat; and a fluid transport assembly arranged to release each sweat droplet protruding from the outlets and transport each said released sweat droplet to the sensor, the respective outlet being thereby made available for a subsequent sweat droplet to form and protrude therefrom upon further filling of the respective chamber, wherein the fluid transport assembly is arranged to transport the released sweat droplet at least as fast as the subsequent sweat droplet protrudes from the respective outlet such that the sweat droplets from the same chamber do not contact each other.

**14.** The system (300) according to claim 13, wherein the apparatus (100) comprises:

at least one first track (406A, 406B, 406C, 406D) in which said chambers are defined; and
a second track (408), each of the at least one first track being fluidly coupled to the second track, wherein the second track is arranged to transport sweat droplets received from the at least one first track towards the sensor, and wherein the fluid transport assembly comprises:

a series of tiles (124), wherein said tiles (124) are provided along said at least one first track (406A, 406B, 406C, 406D) and along said second track (408); and
an electric field generator for charging and discharging each of the tiles of the series in sequence, such as to release each said sweat droplet from the respective outlet and to transport each said sweat droplet towards the sensor; optionally wherein the tiles are provided in pairs, each said pair comprising a first tile provided along the at least one first track and a second tile provided along the second track, an electrical connection connecting the first tile to the second tile.

**15.** A method (224) comprising:

receiving (226) sweat from one or more sweat glands;
transporting (228) said sweat as discrete sweat droplets to a sensor;
sensing (230) the sweat droplets using the sensor during a time period;
recording (232) the sweat droplets sensed during the time period;
determining (234) time intervals between consecutive sensed sweat droplets during the time period; the method being **characterized by**
using a processor to identify (238), using the time intervals, at least one active period of each of the one or more sweat glands during which the respective sweat gland is excreting sweat, and at least one rest period of each of the one or more sweat glands during which the respective sweat gland is not excreting sweat, the active and rest periods being assigned to said one or more sweat glands.

**16.** The method according to claim 15, further comprising:
determining (240) the number of sweat glands to which the active and rest periods are assigned.

**17.** The method (224) according to claim 16, further comprising:

receiving (236) a measure of the volume of each of the recorded sweat droplets; and
determining (242) the sweat rate per gland from the number of recorded sweat droplets, the measure of the volume of each of the recorded sweat droplets, and the determined number of sweat glands; optionally wherein said using the processor to identify (238) said at least one active period and said at least one rest period is based on said measure of the volume of each of the recorded sweat droplets and said time intervals.

**Patentansprüche**

1. Ein System (300), umfassend:

   Einen Sensor (166) zum Fühlen von Schweißtröpfchen;
   eine Vorrichtung (100) zum Aufnehmen von Schweiß von einer oder mehreren Schweißdrüsen und zum Transportieren des Schweißes als diskrete Schweißtröpfchen zum Sensor; und ein Prozessor, konfiguriert zum:

   Aufzeichnen der Schweißtröpfchen, welche der Sensor während einem Zeitraum fühlt;
   Bestimmen von Zeitintervallen zwischen aufeinanderfolgenden erfassten Schweißtröpfchen während des Zeitraums; **dadurch gekennzeichnet, dass** der Prozessor ferner dazu konfiguriert ist, unter Verwendung der Zeitintervalle mindestens eine aktive Periode jeder der einen oder mehreren Schweißdrüsen zu identifizieren, während der die jeweilige Schweißdrüse Schweiß ausscheidet, und mindestens eine Ruheperiode jeder der einen oder mehreren Schweißdrüsen zu identifizieren, während der die jeweilige Schweißdrüse keinen Schweiß ausscheidet, wobei die aktive und die Ruheperiode dieser einen oder mehreren Schweißdrüsen zugeordnet sind.

2. System (300) nach Anspruch 1, wobei der Prozessor ferner so konfiguriert ist, dass er die Anzahl von Schweißdrüsen bestimmt, denen die aktiven und Ruheperioden zugeordnet sind.

3. System (300) nach Anspruch 2, wobei der Prozessor ferner konfiguriert ist zum: Empfangen einer Messung des Volumens jedes der aufgezeichneten Schweißtröpfchen; und Bestimmen der Schweißrate pro Drüse aus der Anzahl der aufgezeichneten Schweißtröpfchen, dem Maß des Volumens jedes der aufgezeichneten Schweißtröpfchen und der ermittelten Anzahl der Schweißdrüsen; wobei optional der Prozessor so konfiguriert ist, dass er die mindestens die besagte eine aktive Periode und die besagte mindestens eine Ruheperiode basierend auf dem besagten Maß des Volumens jedes der aufgezeichneten Schweißtröpfchen und der besagten Zeitintervalle identifiziert.

4. System (300) nach Anspruch 3, wobei der Sensor (166) so konfiguriert ist, dass er einen Indikator des Volumens der Schweißtröpfchen erfasst, und der Prozessor so konfiguriert ist, dass er den besagten erfassten Indikator empfängt.

5. System (300) nach Anspruch 3 oder Anspruch 4, wobei der Prozessor so konfiguriert ist, dass er vom Sensor empfangene Daten an ein erstes Vorlagenmodell anpasst, um dadurch die aktiven und Ruheperioden jeder der einen oder mehreren Schweißdrüsen zu identifizieren. Die besagten Daten umfassen mindestens die Zeitintervalle und das Maß des Volumens jedes der aufgezeichneten Schweißtröpfchen.

6. System (300) nach Anspruch 5, wobei das Anpassen an das erste Schablonenmodell zusätzlich verwendet: eine Anzahl von Schweißtropfen in der mindestens einen aktiven Periode, eine Dauer der mindestens einen aktiven Periode und/oder eine Dauer der mindestens einen Ruhezeit.

7. System (300) nach Anspruch 5 oder Anspruch 6, wobei der Prozessor so konfiguriert ist, dass er eine Anpassungsgüte der besagten Daten an das erste Vorlagenmodell bewertet und optional, basierend auf der Anpassungsgüte, zumindest einen Teil der Daten an ein weiteres erstes Vorlagenmodell anpasst.

8. System (300) nach einem der Ansprüche 5 bis 7, wobei der Prozessor so konfiguriert ist, dass er nach dem Anpassen der besagten Daten an das erste Vorlagenmodell mindestens einen Teil der Daten an ein zweites Vorlagenmodell anpasst, wobei das erste Vorlagenmodell darauf basiert, dass zumindest einige der besagten Schweißtröpfchen von einer Schweißprobe stammen, die aus Schweiß besteht, der von einer einzelnen Schweißdrüse ausgeschieden wird, und das zweite Vorlagenmodell basiert darauf, dass zumindest einige der besagten Schweißtröpfchen von einer weiteren Schweißprobe stammen, die aus Schweiß besteht, der aus zwei oder mehr Schweißdrüsen ausgeschieden wird.

9. System (300) nach einem der vorhergehenden Ansprüche, wobei die Vorrichtung (100) so angeordnet ist, dass sie Schweißtröpfchen mit einem vorbestimmten Volumen zu dem Sensor (166) transportiert.

10. System (300) nach einem der vorhergehenden Ansprüche, wobei der Sensor (166) eine Erfassungsvorrichtung zum Erfassen eines Parameters umfasst, der sich auf die Konzentration eines Analyten bezieht, dessen Konzentration als Funktion der Schweißrate variiert, wobei der Prozessor konfiguriert ist, den besagten Parameter beim Zuordnen der aktiven und Ruheperioden zu der einen oder den mehreren Schweißdrüsen zu verwenden.

**11.** System (300) nach Anspruch 10, wobei die Erfassungsvorrichtung ein Leitfähigkeitssensor und der Parameter die Leitfähigkeit ist.

**12.** System (300) nach einem der vorhergehenden Ansprüche, wobei der Sensor (166) einen Sensor für einen Biomarker umfasst; wobei optional der Prozessor zum Empfangen einer Vielzahl von Biomarker-Konzentrationen von dem Biomarker-Sensor während der besagten mindestens einen aktiven Periode einer jeweiligen Schweißdrüse und zum Bestimmen einer Variation der Biomarker-Konzentration im Laufe der Zeit innerhalb des besagten mindestens einen aktiven Zeitraums konfiguriert ist.

**13.** System (300) nach einem der vorhergehenden Ansprüche, wobei die Vorrichtung (100) eine Vielzahl von Kammern (102) umfasst, wobei jede der Kammern einen Einlass (104) zum Aufnehmen von Schweiß von der Haut und einen Auslass (114) aufweist, so angeordnet, dass sich nach dem Füllen der Kammer mit Schweiß ein Schweißtropfen bildet und daraus herausragt; und
eine Anordnung zum Transport von Flüssigkeit, die so angeordnet ist, dass sie jedes aus den Auslässen herausragende Schweißtröpfchen freisetzt und jedes besagte freigesetzte Schweißtröpfchen zum Sensor transportiert, wobei der jeweilige Auslass dadurch für ein nachfolgendes Schweißtröpfchen verfügbar gemacht wird, welches sich bildet und daraus hervortritt, wenn die jeweilige Kammer weiter gefüllt wird, wobei die Anordnung zum Flüssigkeitstransport angeordnet ist, um das freigesetzte Schweißtröpfchen mindestens so schnell zu transportieren, wie das nachfolgende Schweißtröpfchen aus dem jeweiligen Auslass herausragt, so dass die Schweißtröpfchen aus derselben Kammer einander nicht berühren.

**14.** System (300) nach Anspruch 13, wobei die Vorrichtung (100) umfasst: Mindestens eine erste Spur (406A, 406B, 406C, 406D), in der die besagten Kammern definiert sind; und
eine zweite Spur (408), wobei jede der mindestens einen ersten Spur mit der zweiten Spur flüssig gekoppelt ist, wobei die zweite Spur so angeordnet ist, dass sie von der zumindest einen ersten Spur empfangene Schweißtröpfchen zum Sensor transportiert, und wobei die Baugruppe zum Transport von Flüssigkeit umfasst:

eine Reihe von Kacheln (124), wobei die Kacheln (124) entlang der mindestens einen ersten Spur (406A, 406B, 406C, 406D) und entlang der zweiten Spur (408) vorgesehen sind; und
einen elektrischen Feldgenerator zum aufeinanderfolgenden Laden und Entladen jeder Kachel der Reihe, um jedes Schweißtröpfchen von dem jeweiligen Auslass freizusetzen und jedes besagte Schweißtröpfchen zum Sensor zu transportieren; wobei optional die Kacheln paarweise vorgesehen sind, wobei jedes besagte Paar eine entlang der mindestens einen ersten Spur bereitgestellte erste Kachel und eine entlang der zweiten Spur bereitgestellte zweite Kachel umfasst, wobei eine elektrische Verbindung die erste Kachel mit der zweiten Kachel verbindet.

**15.** Ein Verfahren (224), umfassend:

Empfangen (226) von Schweiß aus einer oder mehreren Schweißdrüsen;
Transportieren (228) des besagten Schweißes als diskrete Schweißtröpfchen zu einem Sensor;
Erfassen (230) der Schweißtröpfchen unter Verwendung des Sensors während einer Zeitspanne;
Aufzeichnen (232) der während des Zeitraums erfassten Schweißtröpfchen;
Bestimmen (234) von Zeitintervallen zwischen aufeinanderfolgenden erfassten Schweißtröpfchen während des Zeitraums; wobei das Verfahren **gekennzeichnet ist durch** Verwenden eines Prozessors zum Identifizieren (238) unter Verwendung der Zeitintervalle, mindestens einer aktiven Periode jeder der einen oder mehreren Schweißdrüsen, während der die entsprechende Schweißdrüse Schweiß ausscheidet, und mindestens einer Ruheperiode jeder der einen oder mehrere Schweißdrüsen, während denen die jeweilige Schweißdrüse keinen Schweiß absondert, wobei die aktiven und Ruhephasen diesen besagten einen oder mehreren Schweißdrüsen zugeordnet sind.

**16.** Verfahren gemäß Anspruch 15, welches weiterhin umfasst: Bestimmen (240) der Anzahl der Schweißdrüsen, denen die aktiven und Ruheperioden zugeordnet sind.

**17.** Verfahren (224) gemäß Anspruch 16, welches weiterhin umfasst:

Empfangen (236) eines Maßes des Volumens jedes der aufgezeichneten Schweißtröpfchen; und Bestimmen

47

(242) der Schweißrate pro Drüse aus der Anzahl der erfassten Schweißtröpfchen, des Maßes für das Volumen jedes der aufgezeichneten Schweißtröpfchen und der ermittelten Anzahl von Schweißdrüsen;

wobei optional der Prozessor zum Identifizieren (238) der besagten mindestens einen aktiven Periode und der besagten mindestens einen Ruheperiode auf dem besagten Maß des Volumens jedes der aufgezeichneten Schweißtröpfchen und den besagten Zeitintervallen basiert.

**Revendications**

1. Un système (300) comprend:

   Un capteur (166) pour détecter les gouttelettes de sueur;
   un appareil (100) pour recevoir la sudation depuis une ou plusieurs glandes sudoripares et transporter ladite sueur en tant que gouttelettes de sueurs discrètes vers le capteur; et
   un processeur configuré pour:

   enregistrer les gouttelettes captées par le capteur lors d'une période déterminée;
   déterminer les intervalles entre les gouttelettes de sueur captés lors d'une période prédéterminée; **caractérisée par le fait que** le processeur est également configuré pour
   identifier, en utilisant les intervalles, au moins une période active de chacune d'une ou plusieurs glandes sudoripares au cours de laquelle les glandes sudoripares respectives excrètent de la sueur, et au moins une période de repos de chacune d'une ou de plusieurs glandes sudoripares au cours de laquelle la glande sudoripare respective n'excrète pas de sueur, les périodes actives et de repos étant assignées à une ou plusieurs glandes sudoripares.

2. Le système (300) selon la revendication 1, où le processeur est également configuré pour déterminer le nombre de glandes sudoripares auxquels les périodes actives et de repos sont assignées.

3. Le système (300) selon la revendication 2, où le processeur est également configuré pour:

   recevoir une mesure du volume de chacune des gouttelettes de sueur enregistrées; et
   déterminer le taux de sudation par glandes à partir du nombre de gouttelettes de sueurs enregistrées, la mesure du volume de chacune des gouttelettes de sudation enregistrées et le nombre déterminé de glandes sudoripares; éventuellement où le processeur est configuré pour identifier au moins ladite période active et au moins ladite période de repos fondée sur ladite mesure du volume de chacune des gouttelettes de sueur enregistrées et desdits intervalles.

4. Le système (300) selon la revendication 3, où le capteur (166) est configuré pour détecter un indicateur du volume des gouttelettes de sueur, et le processeur est configuré pour recevoir ledit indicateur détecté.

5. Le système (300) selon la revendication 3 ou 4 où le processeur est configuré pour adapter les données reçues par le capteur au premier modèle, permettant ainsi d'identifier les périodes actives et de repos de chacune des glandes sudoripares, lesdites données comprennent au moins des intervalles, et la mesure du volume de chacune des gouttelettes de sueurs enregistrées.

6. Le système (300) selon la revendication 5, où ledit ajustement au premier modèle utilise en plus: un nombre de gouttelettes de sueurs lors d'au moins une période active, une durée d'au moins une période active, et une durée d'au moins une période de repos.

7. Le système (300) selon la revendication 5 ou 6, où le processeur est configuré pour évaluer un bon ajustement desdites données au premier modèle, et s'il est éventuellement fondé sur le bon ajustement, l'ajustement d'au moins une partie des données à un autre premier modèle.

8. Le système (300) selon l'une quelconque des revendications 5 à 7, où le processeur est configuré pour, après l'adaptation desdites données au premier modèle, adapter au moins une partie des données à un second modèle, où le premier modèle est fondé sur aux moins certaines desdites gouttelettes de sueur en provenance de l'échantillon de sueur constituée de sueur excrétée depuis une seule glande sudoripare, et le second modèle qui est fondé sur au moins certaines desdites gouttelettes de sueur provenant d'un autre échantillon de sueur constitué de sueur excrétée par deux ou plusieurs glandes sudoripares.

9. Le système (300) selon l'une quelconque des revendications précédentes, où l'appareil (100) est disposé pour transporter des gouttelettes de sueur ayant un volume prédéterminé par rapport au capteur (166).

10. Le système (300) selon l'une des revendications précédentes, où le capteur (166) comprend un dispositif de détection pour détecter un paramètre relatif à la concentration d'un analyte dont la concentration varie en tant que fonction du taux de transpiration, où le processeur est configuré pour utiliser ledit paramètre dans l'attribution de période active et de repos à une ou plusieurs glandes sudoripares.

11. Le système (300) selon la revendication 10, où le dispositif de détection est un capteur de conductivité et le paramètre est la conductivité.

12. Le système (300) selon l'une quelconque des revendications précédentes, où le capteur (166) comprend un capteur de biomarqueurs ;
éventuellement le processeur est configuré pour recevoir une pluralité de concentrations de biomarqueurs du capteur de biomarqueurs lors d'au moins une période active d'une glande sudoripare et déterminer une variation d'une concentration de biomarqueurs dans au moins une période active.

13. Le système (300) selon l'une quelconque des revendications précédentes, où l'appareil (100) comprend une pluralité de chambres (102), chacune des chambres possédant une entrée (104) pour recevoir de la sueur provenant de la peau, et une sortie (114) disposée de telle sorte que les gouttelettes de sueur se forment et fassent saillie à partir de celles-ci après le remplissage de la chambre avec la sueur ; et un ensemble de transport de fluide disposé pour libérer chaque goutte de sueur dépassant des sorties et transporter chacune desdites gouttelettes de sueur libérées vers le capteur, la sortie respective étant ainsi rendu disponible pour une goutte de sueur ultérieure pour former et faire saillir de celle-ci après un nouveau remplissage de la chambre respective, où l'ensemble de transport de liquide est disposé de manière à transporter la gouttelette de sueur libérée aussi rapidement que possible alors que la goutte de sueur suivante dépasse de la sortie respective de telle sorte que les gouttelettes de sueur de la même chambre ne sont pas en contact les unes avec les autres.

14. Le système (300) selon la revendication 13, où l'appareil (100) comprend :

au moins une première piste (406A, 406B, 406C, 406D) dans lequel lesdites chambres sont définies ; et
une deuxième piste (408), chacune d'au moins une première piste étant couplée de manière fluide à la seconde piste, où la seconde piste est disposée pour transporter les gouttelettes de sueur reçues à partir d'au moins une première piste vers le capteur, et où l'ensemble de transport du liquide comprend :

une série de carreaux (124), où lesdits carreaux (124) sont fournis le long dudit axe de la première piste (406A, 406B, 406C, 406D) et le long de ladite seconde piste (408) ; et
un générateur de champ électrique pour charger et décharger chacun des carreaux de la série en séquence, afin de libérer chacune desdites gouttelettes de sueur et pour transporter chacune desdites gouttelettes de sueur vers le capteur ; éventuellement où les carreaux sont fournis en paires, chacune desdites paires comprenant un premier carreau fourni le long d'au moins une première piste et un second carreau fourni le long d'une seconde piste, une connexion électrique connectant le premier carreau au second carreau.

15. Une méthode (224) comprend :

la réception (226) de la sueur d'une ou plusieurs glandes sudoripares ;
le transport (228) de ladite sueur en tant que gouttelettes de sueur discrètes vers un capteur ;
la détection (230) de gouttelettes de sueur utilisant le capteur lors d'une période déterminée ;
l'enregistrement (232) de gouttelettes de sueur captées lors d'une période déterminée ;
déterminer (234) des intervalles entre des gouttelettes de sueur captées consécutives lors d'une période déterminée ; la méthode étant **caractérisée par** l'utilisation d'un processeur pour identifier (238), en utilisant les intervalles, au moins une période active de chacune des glandes sudoripares, au cours desquels la glande sudoripare respective excrète de la sueur, et au moins une période de repos d'une ou plusieurs glandes sudoripares au cours de laquelle la glande sudoripare respective n'excrète pas de sueur, les périodes actives et de repos étant assignées à une ou plusieurs glandes sudoripares.

**16.** La méthode de la revendication 15 comprend également:
la détermination (240) du nombre de glandes sudoripares auxquelles les périodes actives et de repos sont assignées.

**17.** La méthode (224) selon la revendication 16 comprend également:

la réception (236) d'une mesure du volume de chacune des gouttelettes de sueurs enregistrées; et
la détermination (242) du taux de transpiration par glandes à partir du nombre de gouttelettes de sueurs enregistrées, la mesure du volume de chacune des gouttelettes de sueurs enregistrées, et le nombre déterminé de glandes sudoripares; éventuellement où ladite utilisation du processeur pour identifier (238) au moins une période active et une période de repos sont fondées sur ladite mesure du volume de chacune des gouttelettes de sueurs enregistrées et desdits intervalles.

FIG. 1

FIG. 2

FIG. 3

FIG. 4

FIG. 5

FIG. 6

100

112    114

126A                                          128

110                                           126B

104    108    106

118

FIG. 7

100

134    136

112    114

132    132

110

104

102    108    106

118

FIG. 8

FIG. 9

FIG. 10

FIG. 11a

Fig 11b

FIG. 12

FIG. 13

FIG. 14

FIG. 15

FIG. 16

FIG. 17

FIG. 18

FIG. 19

FIG. 20

FIG. 21

FIG. 22

EP 4 044 923 B1

224

226

228

230

232

234

236

238

240

242

FIG. 23

243

244

246

248

250

252

254

256

FIG. 24

63

FIG. 25

FIG. 26

FIG. 27

FIG. 28

FIG. 29

FIG. 30

FIG. 31

FIG. 32

FIG. 33

FIG. 34

FIG. 35

FIG. 36

FIG. 37A          FIG. 37B

FIG. 38

FIG. 39

FIG. 40

FIG. 41

FIG. 42

FIG. 43

FIG. 44

FIG. 45

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018125695 A1
- US 20150112165 A1
- WO 2019060689 A1
- US 2018042585 A1
- US 2010179403 A1
- WO 2018125695 A

### Non-patent literature cited in the description

- **MENA-BRAVO ; DE CASTRO.** Sweat: A sample with limited present applications and promising future in metabolomics. *J. Pharm. Biomed. Anal.,* 2014, vol. 90, 139-147
- **GAO et al.** Fully integrated wearable sensor arrays for multiplexed in situ perspiration analysis. *Nature,* 2016, vol. 529, 509-514
- **TAYLOR.** Regional variations in transepidermal water loss, eccrine sweat gland density, sweat secretion rates and electrolyte composition in resting and exercising humans. *Extrem Physiol Med,* 2013, vol. 2, 4
- **SIMMERS.** Prolonged and localised sweat stimulation by iontophoretic delivery of the slowly-metabolised cholinergic agent carbachol. *Journal of Dermatological Science,* 2018, vol. 89, 40-51
- **HEIKENFELD et al.** Digital nanoliter to milliliter flow rate sensor with in vivo demonstration for continuous sweat rate measurement. *Lab Chip,* 2019, vol. 19, 178
- **YANG et al.** Wearable microfluidics: fabric-based digital droplet flowmetry for perspiration analysis. *Lab on a Chip,* 04 January 2017
- **NISHIYAMA et al.** Irregular activation of individual sweat glands in human sole observed by a videomicroscopy. *Autonomic Neuroscience: Basic and Clinical,* 01 April 2001, vol. 88 (1-2), 117-126
- **MORGENTHALER et al.** *Langmuir,* 2003, vol. 19 (25), 10459-10462
- **KS TEH.** Additive direct-write microfabrication for MEMS: A review. *Front. Mech. Eng.,* 2017, vol. 12 (4), 490-509
- **CHEN et al.** In vivo single human sweat gland activity monitoring using coherent anti-Stokes Raman scattering and two-photon excited autofluorescence microscopy. *British Journal of Dermatology,* 2016, vol. 174, 803-812